# EUROPEAN PATENT SPECIFICATION

(11) **EP 1 989 300 B1**
(45) Date of publication and mention of the grant of the patent: **29.08.2012**
(21) Application number: 07705998.8
(22) Date of filing: 09.02.2007
(51) Int. Cl.: C12N 1/14, C12R 1/645, C12N 9/24, C12N 9/42, C12P 7/06, C12P 19/02, C12P 19/04, C12P 19/12, C12P 5/02, A23K 1/165

(54) **TALAROMYCES EMERSONII STRAIN AND USES THEREOF**
TALAROMYCES EMERSONII-STAMM UND DESSEN VERWENDUNG
SOUCHE DE TALAROMYCES EMERSONII ET UTILISATION DE CELLE-CI

(30) Priority: 10.02.2006 IE 20060090
(43) Date of publication of application: 12.11.2008
(60) Divisional application: 11157751.6
(73) Proprietor: National University of Ireland Galway, Galway (IE)
(72) Inventor: TUOHY, Maria, Gerardine, Co. Galway (IE); MURRAY, Patrick, Gerard, Galway City (IE); GILLERAN, Caroline, Teresa, Roscommon Town, Roscommon (IE); COLLINS, Catherine, Majella, County Cork (IE); REEN, Francis, Jeremiah, County Kerry (IE); MCLOUGHLIN, Lassarina, Patrick, Co. Leitrim (IE); LYDON, Anne, Geraldine, Stephanie, Galway City (IE); MALONEY, Alan, Patrick, Galway City (IE); HENEGHAN, Mary, Noelle, County Mayo (IE); O'DONOGHUE, Anthony, John, County Cork (IE); MAHON, Cathal, Sean, County Laois (IE)
(74) Representative: Gates, Marie Christina Esther
(86) International application number: PCT/IE2007/000016
(87) International publication number: WO 2007/091231

(56) References cited:
- WO-A-01/42433
- WO-A-01/70998
- WO-A-02/24926
- WO-A-2005/100582
- GB-A- 2 150 933
- US-A- 3 880 742
- MCCARTHY T C ET AL: "Comparison of wild-type and UV-mutant beta-glucanase-producing strains of Talaromyces emersonii with potential in brewing applications" JOURNAL OF INDUSTRIAL MICROBIOLOGY & BIOTECHNOLOGY, vol. 32, no. 4, April 2005 (2005-04), pages 125-134, XP002398221 ISSN: 1367-5435
- MAHESHWARI R ET AL: "Thermophilic fungi: Their physiology and enzymes" MICROBIOLOGY AND MOLECULAR BIOLOGY REVIEWS, vol. 64, no. 3, September 2000 (2000-09), pages 461-488, XP002398222 ISSN: 1092-2172
- MCCARTHY T ET AL: "Evaluation of three thermostable fungal endo-beta-glucanases from Talaromyces emersonii for brewing and food applications" PROCESS BIOCHEMISTRY, vol. 40, no. 5, April 2005 (2005-04), pages 1741-1748, XP004755247 ISSN: 1359-5113
- MCCARTHY T ET AL: "Catalytic properties and mode of action of three endo-beta-glucanases from Talaromyces emersonii on soluble beta-1,4- and beta-1,3;1,4-linked glucans" INTERNATIONAL JOURNAL OF BIOLOGICAL MACROMOLECULES, vol. 33, no. 1-3, November 2003 (2003-11), pages 141-148, XP002330766 ISSN: 0141-8130
- TUOHY M G ET AL: "Kinetic parameters and mode of action of the cellobiohydrolases produced by Talaromyces emersonii" BIOCHIMICA ET BIOPHYSICA ACTA. PROTEIN STRUCTURE AND MOLECULAR ENZYMOLOGY, vol. 1596, no. 2, 29 April 2002 (2002-04-29), pages 366-380, XP004353624 ISSN: 0167-4838 cited in the application
- MURRAY P G ET AL: "Isolation and characterization of a thermostable endo-beta-glucanase active on 1,3-1,4-beta-D-glucans from the aerobic fungus Talaromyces emersonii CBS 814.70" ENZYME AND MICROBIAL TECHNOLOGY, vol. 29, no. 1, 5 July 2001 (2001-07-05), pages 90-98, XP001015796 ISSN: 0141-0229 cited in the application
- TUOHY M G ET AL: "Characterization of the individual components of the xylanolytic enzymes system of Talaromyces emersonii" BIORESOURCE TECHNOLOGY, vol. 50, no. 1, 1994, pages 37-42, XP000876865 ISSN: 0960-8524
- TUOHY M G ET AL: "The xylan-degrading enzyme system of Talaromyces emersonii: novel enzymes with activity against aryl beta-D-xylosides and unsubstituted xylans." THE BIOCHEMICAL JOURNAL. 1 MAR 1993, vol. 290 ( Pt 2), 1 March 1993 (1993-03-01), pages 515-523, XP000876862 ISSN: 0264-6021
- TUOHY M G ET AL: "Production of thermostable xylan-degrading enzymes by Talaromyces emersonii" BIORESOURCE TECHNOLOGY, vol. 39, no. 2, 1992, pages 131-138, XP002330765 ISSN: 0960-8524
- MORRISON J ET AL: "Cellulase production by Talaromyces emersonii CBS 814.70 and a mutant UV7 during growth on cellulose, lactose and glucose containing media." ENZYME AND MICROBIAL TECHNOLOGY, vol. 9, no. 7, 1987, page 422, XP008068681
- MOLONEY A P ET AL: "Isolation and characterization of the 1,4-beta-D-glucan glucanohydrolases of Talaromyces emersonii" BIOCHEMICAL JOURNAL, vol. 225, no. 2, 1985, pages 365-374, XP000929303 ISSN: 0264-6021
- MOLONEY A P ET AL: "Isolation of mutants of Talaromyces emersonii CBS 814.70 with enhanced cellulase activity" ENZYME AND MICROBIAL TECHNOLOGY, vol. 5, no. 4, 1983, pages 260-264, XP002398223 ISSN: 0141-0229 cited in the application
- MARKOV A V ET AL: "New effective method for analysis of the component composition of enzyme complexes from Trichoderma reesei" BIOCHEMISTRY (MOSCOW), vol. 70, no. 6, June 2005 (2005-06), pages 657-663, XP008068738 ISSN: 0006-2979
- BHAT M K: "Cellulases and related enzymes in biotechnology" BIOTECHNOLOGY ADVANCES, vol. 18, no. 5, August 2000 (2000-08), pages 355-383, XP004211815 ISSN: 0734-9750
- ZALDIVAR J ET AL: "Fuel ethanol production from lignocellulose: A challenge for metabolic engineering and process integration" APPLIED MICROBIOLOGY AND BIOTECHNOLOGY, vol. 56, no. 1-2, July 2001 (2001-07), pages 17-34, XP002201309 ISSN: 0175-7598
- SUN Y ET AL: "Hydrolysis of lingnocellulosic materials for ethanol production: a review" BIORESOURCE TECHNOLOGY, vol. 83, 2002, pages 1-11, XP002988039 ISSN: 0960-8524
- MURRAY P G ET AL: "Molecular cloning, transcriptional, and expression analysis of the first cellulase gene (cbh2), encoding cellobiohydrolase II, from the moderately thermophilic fungus Talaromyces emersonii and structure prediction of the gene product." BIOCHEMICAL AND BIOPHYSICAL RESEARCH COMMUNICATIONS, vol. 301, no. 2, 7 February 2003 (2003-02-07), pages 280-286, XP002398226 ISSN: 0006-291X cited in the application

## Description

### Introduction

The present invention relates to a strain of Talaromyces emersonii and enzymes and enzyme systems isolable therefrom for use in environmental and waste management, chemical and biochemical production and processing, biotechnological processes, in test or diagnostic kits, prebiotics and synbiotics, healthcare products, functional and novel foodstuffs and beverages, surfactant production, agri and horticultural applications.

Currently Europe faces a crisis in waste management with 2,000 million tonnes of waste being produced each year. Much of this waste is organic, derived from plant materials (biomass), is rich in carbohydrates (sugars) and therefore, represents a valuable resource when broken down to its component sugars. Other types of waste such as fruit waste putrefies and poses an environmental hazard. This waste is generally combined with pig feed to extract some value from it, however it is considered a low cost waste.

Biomass represents a highly varied and variable feedstock, yet is the most renewable energy feedstock on Earth. If harnessed, it could provide a sustainable alternative to the ever-depleting stocks of fossil fuels. There is a global interest in converting the energy reserve in biomass to usable energy forms. While a major focus has been the production of biofuels, such as bioethanol, for transport purposes, markets for valuable co-products generated during biofuel production (e.g. CO₂, lignin-rich residues, chemical feedstocks) have also been identified. Currently, in the US, approximately 3 billion gallons of ethanol are produced from corn per annum, mainly for use in the transport fuel sector. This only represents approx. 1% of the total motor fuel consumption, and it is predicted that by 2010, bioethanol production will have increased more than 7-fold, and will include other biomass substrates such as woody-residues. If the woody residues are derived from rapidly renewable `waste' sources generally regarded as 'scrub' or brushwood, considerable value can be derived both in terms of process costs, meeting production targets and local environmental issues. The advantages of bioethanol as a clean and environmentally friendly alternative to petroleum and other fossil fuels are clear. Global adoption of bioethanol as a main motor fuel would offset many of the air pollution problems that are a feature of densely populated urban areas. Modem cars can be easily adapted to run on ethanol/gasoline mixtures (`gasohol') and new engines are available that can utilise pure ethanol as the sole fuel source.

Plant biomass, including softwood species, are rich in complex carbohydrates (polysaccharides) that can be broken down by enzymatic or chemical means to simple, fermentable sugars. For example, softwoods such as Sitka spruce and pine contain (% dry weight) approximately 41-43% cellulose (a polymer of β-1,4-linked glucose units), 20-30% hemicellulose (a mixture of mannose, galactose xylose and arabinose containing polysaccharides) and 25-30% lignin, a non-carbohydrate polyphenolic polymer of high calorific value. So about 65-70% of the dry weight of woody residues is complex carbohydrate that can be used to provide sugar-rich feedstocks for fermentation to bioethanol.

Fungi represent one of the most important microbial life forms that break down such materials. Talaromyces emersonii is a thermophilic aerobic fungus found naturally in compost heaps and other eco-systems degrading biomass-rich materials. Thermal stability is a characteristic feature of many of the Talaromyces emersonii enzymes systems isolated to-date. Considered to be a 'soft-rot' species, which can target all parts of plant material, this euasomycete produces comprehensive carbohydrate-modifying enzyme systems, including cellulolytic, hemicellulolytic, pectinolytic, and amylolytic enzymes, as well as an array of oxidase/oxidoreductase and proteolytic activities. Thus Talaromyces emersonii can access complex growth substrates encountered in its natural habitat.

McCarthy et al. (2005) J. Ind. Microbiol. Biotechnol. 32:125-134 discloses a comparison of wild-type and UV-mutant beta-glucanase-producing strains of *Talaromyces emersonii* with potential in brewing applications. Moloney et al. (1983) Enzyme Microb. Technol. 5:260-264 discloses isolation of mutants of *Talaromyces emersonii* CBS 814.70 with enhanced cellulase activity. Maheswari et al. (2000) Microbiol. Mol. Biol. Rev. 64:461-488 discloses that the optimal and maximum growth temperature for *Talaromyces emersonii* is respectively 40-45 and 55°C.

PCT Publication Nos. WO 01/70998 and WO 02/24926 disclose the isolation of cellulases from Talaromyces emersonii and in particular cellulases having β-glucanase and xylanase activity. The disadvantage of these enzymes is that they can only target one type (or a limited number) of a constituent(s) of a substrate. Thus in order to metabolise a certain substrate it is necessary to identify the components of that substrate, produce polypeptides having suitable enzyme activity to metabolise these components, produce a required amount of the polypeptide by routine procedures such as recombinant techniques, modify the polypeptides if necessary to alter the thermostability or pH optimum for example of the enzymes, express the polypeptide and use the resultant enzyme to target that constituent of that substrate. These are very time consuming and expensive procedures.

Furthermore, these procedures do not take into account the fact that some enzymes can exhibit more than one activity or, when used in combination with another enzyme, could have modified efficiency. Thus these methods could lead to the overproduction of enzymes which are not required thus also leading to over expenditure and time costs. There is therefore a need for enzymes and enzyme systems isolated from Talaromyces emersonii and a method of isolating such enzymes and enzyme systems which overcomes the above-mentioned disadvantages.

There is also a need for optimized enzyme compositions, particularly thermally stable enzyme compositions, to generate `syrups' rich in fermentable sugars, for use in biomass to bioethanol initiatives. As the target biomass substrates or feedstocks for bioethanol production can vary from waste streams (e.g. VFCWs and OFMSWs (Vegetable Fraction of Collected Wastes and Organic Fraction of Municipal Solid Wastes) to agricultural crops (e.g. corn, sugar beet, grasses, etc.) to woody biomass, getting the right enzyme preparation, at a low process cost, to obtain maximum yields of fermentable sugars is a significant challenge. For many years, the cost of enzymes used in conversion of biomass prior to production of bioethanol by microbial fermentation has been a major negative factor. It is thus an object to produce low-cost enzyme preparations for these purposes.

The enzyme preparations are derived from thermophilic, generally regarded as safe (GRAS) fungal sources, whereas fungal enzymes in use to-date in commercial and research bioethanol applications are mainly from mesophilic sources (e.g. Trichoderma sp./Gliocladium sp., Aspergillus sp. and Penicillium sp.).

There is also a need for enzyme preparations which work at higher redactions temperatures, i.e..thermostable enzymes allow shorter reaction times/enzymatic treatment steps, allow simultaneous pasteurization of the hydrolysate, result in significant overall hydrolysis/saccharification, have a potential for reducing enzyme loading, and/or a potential for recycling of the enzyme preparation, all of which may serve to reduce costs associated with the use of these enzymes. of these enzymes.

### Statement of the Invention

the present invention relates to a strain of Talaromyces emersonii which was deposited with the International Mycological Institute (CABI Bioscience UK) on November 2^{nd} 2005 under the number IMI 393751.

The advantage of using Talaromyces emersonii as the enzyme source is that it is a 'generally regarded as safe' (GRAS) microorganism and has a long history of use in the food, beverage, agri-feed and pharmaceutical sectors.

The advantage of this novel strain of Talaromyces emersonii is that the enzymes derived therefrom have optimum activity at temperatures between 54°C and 85°C, with some enzymes maintaining activity at temperatures of up to 95°C. These enzymes will thus retain activity even when high processing temperatures are desirable or required, e.g. production of sugar-rich feedstocks for biochemical, biopharma, chemical or bio-fuel production (ethanol and methane), where reaction temperatures of 65-90°C facilitate simultaneous higher reaction rates, faster substrate conversion and simultaneous pasteurisation of feedstocks, which enhances storage and transport potential. Additionally as higher temperatures can be used with these enzymes, each process has a shorter reaction time so there is both a time and cost saving. A further advantage is that if th temperature is sufficiently high, pasteurisation will occur killing any undesirable microorganisms which cannot withstand such high temperatures. Additionally the enzymes purified from this strain have been shown to have a longer shelf life.

The strain of the invention can provide an enzyme system comprising a cellobiohydrolase I or a cellobiohydrolase II or a mixture thereof, a β-glucosidase 1, a xylanase and an endo-β-(14-glucanase. The invention also provides a method of using this micro organism for the bioconversion of plant or plant-derived materials, such as virgin plant materials of terrestrial and marine origin, and waste streams thereof, including corree, tea, brewing and beverage residues, fruit and fruit peeling/skins, vegetable peelings, catering and food processing, leaves/horticultural wastes, florist wastes, cereals and cereal processing residues, other agri and garden wastes, bakery production and shop wastes, paper products such as glossy coloured magazines and coloured newsprint, black and white newsprint, white, coloured and recycled paper, brown paper, paper bags, card and cardboard, paper cups and plates, tissues, wipes, cellophane and Sellotape^{®}, biodegradable packing, cellulose-rich hospital wastes such as bandages, papers, wipes, bandages, masks, textiles such as pyjamas and towelling and for subsequent use in the production of biofuel (bioethanol and bioges).

The strain of the invention can produce an enzyme system comprising a cellobiohydrolase I or a cellobiohydrolase II or a mixture thereof, a β-glucosidase 1, a xylanase and an endo-β-(1,3)4-glucanase. The invention also provides a method of using of this strain in the production of monosaccharide-rich feedstocks from plant residues for generation of high value products, e.g. antibiotics, antibiotics and anti-virals, carotenoids, antioxidants, solvents and other chemicals and biochemicals, including food-grade ingredients, additives for cosmetics, oligosaccharides and glycopeptides for research and functional glycomics.

The invention provides A thermophilic strain of Talaromyces emersonii, which has a growth temperature range of 30 to 90°C, with an optimum range of 30 -55oC and which actively produces enzymes at temperatures above 55°C. The strain of Talaromyces emersonii was deposited with the deposition no. IMI 393751. The invention relates to a mutant thereof also encoding thermostable enzymes, An enzyme produced by the strain which retains activity at temperatures above 55°C. The enzyme may be selected from the group consisting of carbohydrate-modifying enzymes, proteolytic enzymes, oxidases andoxidoreductases.

The strain of the invention can produce an enzyme composition comprising a callobiohydrolase I or a cellobioliydrolase II or a mixture thereof, a β-glucosidase 1, a xylanase and an endo-β-(1,3)4-glucanase. The enzyme composition may comprises 0.5 to 90% cellobiohydrolase I or a cellobioliydrolase II or a mixture thereof, 0.1 to 33 % β-glucosidase 1, 0.6 to 89% xylanase and 0.4 to 68% endo-β-(1,3)4-glucanase.

The strain of the invention can also produce an enzyme system comprising CBH I (10-30%), CBHII (10-15%), β-(1,3)4-glucanase (20-45%), β-glucosidase (2-15%), and Xylanase (18-55%). The composition may further comprising one or more of the following; β-Xylosidase, a-Glucuronidase, exoxylanase, α-L-Arabinofuranosidase, pectinolytic enzymes, hemicellulases, starch modifying enzymes, oxidoreductaseloxidase and esterases; and proteases.

The invention also provides a method of using of this micro organism in processing and recycling of timbers, wood, and wood derived products. It is effective against highly lignified woody materials, and is resistant to potential inhibitor molecules present in woody residues and processed materials (e.g. extractives, resins, lignin breakdown products, furfural and hydroxyfurfural derivative).

The strain of the invention can also produce an enzyme system comprising CBH I (15-30%), CBH II (10-40%), β(1,3)4-glucanase (15-40%), β-glucosidase (2-15%), Xylanase (15-30%), and 1-8 %β-Xylosidaso. The composition may further comprising one or more of the following; Exoxylanase; α-Glucuronidase; α-L-Arabinofuranosidase; pectinolytil enzymes, including galactosidases, rhamnogalacturonase, polygalacturonase, exogolacturonase and galactanase; starch modifying activity; other hemicellulases, including galactosidases; oxidoreductase oxidase and esterases; and The invention also method of using of this protease. The invention also provides a method of using of this strain in textile processing and recycling. The strain of the invention can also produce an enzyme system comprising CBH I (5-55%), CBH II (8-50%), β(1,3)4-glucanase (10-30%), β-glucosidase (0.5-30%), Xylanase (5-30%), and β-Xylosidase (0.1-10%). The composition may further comprise one or more of the following; α-L-Arabinofuranosidase;α-glucuronidase; Other hydrolases, including selected Pectinolytic enzymes, esterases; Protease; and oxidases. The invention also provides a method of using this strain system in the saccharification of paper wastes.

The strain of the invention can also produce an enzyme system comprising CBH I (2-10%), CBH II (2-10%), β(1,3)4-glucanase (10-45%), β-glucosidase (5-10%), and Xylanase (1-30%). The composition may further comprise one or more of the following; N-Acetylglucosaminidase; chitinase; β(1,3)6-glucanase; β-Xylosidase, α-Glucurondase; α-L-Arabinofuranosidase; pectinolytic enzymes, including galactosidases, rhamnogalacturonase, polygalacturonase, exogalacturonase and galactanase; starch modifying activity; other hemicollulases, including galactosidases; oxidoreductase/ oxidase and esterases; and protease. The invention also provides a method of using this strain in antifungal, biocontrol and slime control strategies in environmental, medical and construction sectors (e.g. control of dry-rot), and in the pulp and paper industry (e.g. slime control).

The strain of the invention can also produce an enzyme system comprising CBHI (1-20%), CBH II (1-28%), β(1,3)4-glucanase (15-40%), β-glucosidase (2-15%), Xylanase (18-55%), β-Xylosidese (0.1-10%) and α-L-Arabinofuranosidase (0.5-5.0%). The composition may further comprise one or more of the following; α-Glucumnidase; starch modifying activity; other hemicellulases, including galactosidases; oxidoredumase/ oxidase and esterases; protease; exoxylanase; Other hydrclases, including Pectinolytic enzymes, Phenolic acid and acetyl(xylan)esterases; Protease; and Lignin-modifying oxidase activities. The invention also provides method. of using this strain in horticultural applications, e.g. production of novel, bioactive compounds for growth promotion and disease resistance. For example this strain can be used for the release of bioactive flavonoid glycosides, production of oligogalacturonides from pectin-rich materials,

xyloglucooligosaccharides from xyloglucans, galacotoongosaccharides from galactans, substituted xylooligosaccharides from plant xylans, or 1,3-glucooligosaccharides (laminarialigosaccharides) from fungal cell wall ß-glucan, or algal laminaran, for promotion of growth in plants, activation of plant defense response mechanisms against plant pathogens and increasing the resistance to disease, as some of these oligosaccharides (e.g. laminarioligosaccharides) can initiate and mediate bioactive properties that are anti-fungal, anti-bacterial and anti-nematode.

The strain of the invention can also produce an enzyme system comprising CBH I (5-30%), CBHII (1-15%), β(1,3)4-glucanase (10-40%), β-glucosidase (2-15%), Xylanase (18-48%), 0.1-20% β-Xylosidase, 1-10%, α-Glucuronidase and 0.1-5.0 % α-L-Arabinofuranosidase. The composition may further comprise one or more of the following; Exoxylanase; pectinolytic enzymes, including galactosidases, rhamnogalacturonase, polygalacturonase, exogalacturonase and galactanase; starch modifying activity; other hemicellulases, including galactosidases; oxidoreductase oxidase and esterases and protease. The invention also provides a method of using this strain in animal feed production to enhance the digestibility of cereal-based feedstuffs. This strain degrades fibre components of cereal-based feedstuffs to oligosaccharides and monosaccharides (simple sugars), some of which are absorbed in gut and metabolised. The strain also produces `prebiotic' oligosaccharides (e.g. mixed-linkage glucooligosaccharides from non-cellulosic cereal β-glucans) that boost the growth of prebiotic bacteria, and can release antioxidants, e.g. ferulic acid, and produce oligosaccharides (substituted glucurono-xylooligosaccharides from cereal xylans) that have an antibacterial effect on species of the gut microflora known to produce carcinogenic molecules (e.g. phenols, amines, etc.).

The strain of the invention can also produce an enzyme system comprising CBH I (0.5-10%), CBH II (0.5-10%), β(1,3)4-glucanase (15-43%), β-glucosidase (2-10%), Xylanase (30-88%), 0.1-2.0 %β-Xylosidase, 0.1-3.0 % α-Glucuronidase, 0.1-4.0 % α-L-arabinofuranosidase. The composition may further comprise one or more of the following; pectinolytic enzymes; starch modifying activity; oxidoreductaseloxidase and esterases; and protease. The invention also provides a method of using this strain in the production of low pentose-containing cereal-based feedstuffs for monogastric animals with improved digestibility and low non-cellulosic β-glucan contents. Non-cellulosic β-Glucans and arabinoxylans from cereals have high water-binding capacity and generate highly viscous solutions. Monogastric animals (e.g. pigs and poultry) are unable to degrade these carbohydrates, which impair the uptake and bioavailibility of important nutrients, increase the diffusion of digestive enzymes, impair adequate mixing of gut contents and act as a physical barrier to the degradation of protein and starch present in these feedstuffs. Overall, these polysaccharides can lead to poor growth performance characteristics. The strain in this invention can catalyse the degradation of cereal arabinoxylans and non-cellulosic β-glucans to produce oligosaccharides (DP3-10 mainly), some of which have potential probiotic properties, without the release of pentose sugars (arabinose and xylose), which are poorly metabolized by monogastric animals and can have anti-nutritional effects. There is provided a method of using alternative cereals as feedstuffs, e.g sorghum and maize.

The strain of the invention can produce an enzyme system comprising CBH I(3-15%), CBH-II (3-15%), (1,3)4-glucanase (25-45%), α-glucosidase (2-15%), Xylanase (18-55%), 0.5-7.0%β-Xylosidase, 0.5-10%, α-Glucuronidase, and 0.1-5.0 % α-L-Arabinofuranosidase. The composition may further comprise one or more of the following; Exoxylanase; pectinolytic enzymes, including galactosidases, rhamnogalacturonase, polygalacturonase, exogalacturonase and galactanase; starch modifying activity; other hemicellulases, including galactosidase; oxidoreductase/ oxidase and esterases; and protease. The invention also provides a method of using this strain in the production of functional feedstuffs with bioactive potential for use in veterinary and human healthcare. This strain can produce bioactive oligosaccharides from raw materials with GRAS status for use in animal healthcare (companion and large animals), including immunostimulatory β-glucooligo-saccharides from terrestrial and marine plants and fungi, xylooligosaccharides with prebiotic and anti-microbial properties from terrestrial and marine plants, chitooligosaccharides with growth promoting, antimicrobial and antiviral potential from crustacean and fungal cell walls, and phenolic compounds with antioxidant potential.

The strain of the invention can produce an enzyme system comprising CBHI (1-15%), CBHII (1-15%), β(1,3)4-glucanase (10-45%), a-glucosidase (2-10%), Xylanase (1-55%), 0.5-12%β-Xylosidase. The composition may further comprise one or more of the following; α-Glucuronidase, α-L-Arabinofuranosidase; β(1,3)6-glucanase; N-Acetylglucos-aminidase; chitinase pectinolytic enzymes, including galactosidases, rhamnogalacturonase, polygalacturonase, exogalacturonase and galactanase; starch modifying activity; other hemicellulases, including galactosidases; oxidoreductase/ oxidase and esterases; protease. The invention also provides a method of using this strain in the production of specialised dairy or dietary products, e.g. foodstuffs and beverage formulations for geriatric and infant healthcare. For example, this strain can be used for production of prebiotic-rich, easily digested foodstuffs for geriatric and infant nutrition, and also for the production of lactose-free products for individuals with galactosaemia or those who are lactose intolerant.

The strain of the invention can produce an enzyme system comprising CBHI (1-10%), CBH II (5-15%), β(1,3)4-glucanase (15-40%), a-glucosidase (2-30%), Xylanase (15-55%), 1-12%β-Xylosidase, 1-8%, a-Glucuronidase and 0.5-5.0 % a-L-Arabinofuranosidase. The composition may further comprise one or more of the following; pectinolytic enzymes, including galactosidases, rhamnogalacturonase, polygalactuonase, exogalacturonase and galactanase; starch modifying activity; other hemicellulases, including galactosidases; oxidoreductase/ oxidase and esterases; protease. The invention also provides a method of using this strain in the bakery and confectionary sectors, and in the formulation of novel healthtfood bakery products. The strain is suitable for the utilization of novel cereals/raw materials such as rye, maize and sorghum. The strain can increase loaf volume and enhance shelf-life by selectively modifying the arabinoxylan components of cereal flours, generate prebiotic and immunostimulatory oligosaccharides and effect the release of antioxidant molecules (e.g. ferulic and coumaric acids), and modify the texture, aroma and sensory properties of bakery and confectionary products.

The strain can also produce an enzyme system comprising CBH I (1-20%), CBH II (140%), (1-40%), β(1,3)4-glucanase (15-45%), α-glucosidase (2-30%), Xylanase (10-55%), 0.5-10 %β-Xylosidase, 0.1-5 % α-L-Arabinofuranosidese. The composition may further comprise one or more of the following; β(1,3)6-glucanase; N-Acetylglucosaminidase; chitinase α-glucuronidase; pectinolytic enzymes, including galactosidases, rhamnogalacturonase, polygalacturonase, exogalacturonase and galactanase; starch modifying activity; oxidoreductase/ oxidase and esterases; protease. There is provided a method of using this strain in the generation of flavour, aroma and sensory precursor compounds in the food industry.by releasing monosaccharide and disaccharides that can be fermented to a variety of products including citric acid, vanillin, etc., releasing flavour glycoconjugates (aroma precursors) from fruits/fruit pulps (e.g. glucosylated aromatic alcohols found in several fruits, including melon, as well as geraniol, limonene, etc.), peptides with savoury, bitter and sweet tastes, amino acids for transformation to sweeteners (e.g. phenylalanine) and phenolic molecules (cinnamic acids, flavonoid glycosides such as quereetin-3-O-rhamnoside), that have flavour, aroma or sensory properties or are precursors of such products, e.g. rhamnose, which can be biotransformed to furaneol, a molecule with a strawberry flavour. In addition, some of these molecules, such as the flavonoid glycosides have antioxidant and anti-microbial (anti-protozoal) activity.

The strain can also produce an enzyme system comprising CBH I (1-15%), CBH II (1-15%), β(1,3)4-glucanase (10-45%), β-glucosidase (2-30%), Xylanase (1-55%), 0.5-12%β-Xylosidase. The composition may further comprise one or more of the following;a-L-Arabinofuranosidase; β(1,3)6-glucanase; N-Acetylglucosaminidase; chitinase a-Glucuronidase; pectinolytic enzymes, including galactosidases, rhamnogalact-uronase, polygalacturonase, exoplacturonase and galactanase; starch modifying activity; other hemicallulases, including galactosidases; oxidoreductasc/ oxidase and esterases; and protease. The invention also provides a method of using this strain for the generation of functional foods, specifically, the modification of plant carbohydrates (terrestrial and some marine) to generate foodstuffs with enhanced health-promoting properties, e.g. foodstuffs enriched in immunostimulatory glucooligosaccharides, xylooligosaccharides, soybean oligosaccharides, (arabine)gatactooligosaccharides, (galacto) and/or (gluco)mannooligosaccharides, gentiooligosaccharides, isomaltooligosaccharides and palatinose oligosaccharides, and promote the release of antioxidant molecules such as carotenoids and phenolic substances (cinnamic acids, catechins and flavonoid glycosides).

The strain can produce an enzyme sysoem comprisiag CBH I (1-15%), CBH II (1-15%), β(1,3,)4-glucanase (10-45%), β-glucosidase (1-15%), Xylanase (1-30%), 0.5-20 %β-Xylosidase. The composition may further comprise one or more of the following; α-L-Arabinofuranosidase; β(1,3)6-glucanase; N-Acetylglucosaminidase; chitinase; α-Glucuronidase; pectinolytic enzymes, including galactosidases, rhamnogalacturonase, polygalacturonase, exogalacturonase and galactanase; starch modifying activity; oxidoreductase oxidase and esterases; protease. The invention also provides a method of using this strain for production of novel designer non-alcoholic and alcoholic beverages, fruit juices and health drinks. This system can modify ß-(1,3)4-glucans, pectic substances, arabinans, xylans, lactose, proteins and phenolic substances to generate low-calorie non-alcoholic and alcoholic beers/lagers, fruit juices and health drinks with improved sensory, anti-oxidant, immune-boosting, anti-bacterial, antiviral potential For example, a 'light' beer with low residual β-glucan content to prevent haze formation (but sufficient β-glucan to provide mouthfeel characteristics) that contains bioactive mixed-linkage glucooligosaccharides and cinnamic acids (antioxidants), or a fruit juice rich in pectin fragments (oligosaccharides), that have a prebiotic effect, and cinnamic acids and selected flavonoid glycosides that provide antioxidant potential.

The strain can produce an enzyme composition CBH I (1-25%), CBH II (1-28%), β(1,3)4-glucanase (18-40%), β-glucosidase (2-30%), Xylanase (15-55%), and β-Xylosidase (0.7-20%). The composition may further comprise one or more of the following; α-Glucuronidase (1-10%), α-L-Arabinofuranosidase (0.1-5.0%), 1-15% Exoxylanase, 5-25%: pectinolytic enzymes, 2-12% starch modifying activity, 2-11% hemicellulases, 1-15% oxidoreductases/oxidase and esterases and 2-15% protease. The composition may be used in the production of monosaccharide-rich feedstocks from plant residues.

The strain can produce an enzyme composition comprising CBH I (12-55%), CBH II (15-30%), β(1,3)4-glucanase (12-26%), β-glucosidase (5-12%), Xylanase (5-30%), β-Xylosidase (0.1-10%) and α-L-Arabinofuranosidase (0.5-3.0%). The composition may further comprise one or more of the following; α-Glucuronidase, other hydrolases including pectinolytic enzymes, phenolic acid and acetyl(xylan)esterases, protease and lignin-modifying oxidase activities, proteases and oxidases. The composition may be used in processing and recycling of wood, paper products and paper.

The strain can produce an enzyme composition comprising CBH I (3-15%), CBH II (3-15%), β(1,3)4-glucanase (15-45%), β-glucosidase (1-15%), Xylanase (16-55%), and β-Xylosidase (0.5-7%). The composition may further comprise one or more of the following; α-Glucuronidase, α-L-Arabinofuranosidase, exoxylanase, pectinolytic enzymes, enzymes with starch modifying activity, hemicellulases, oxidoreductases/oxidase and esterases and proteases. The composition may be used in the production of biopharmaceuticals, such as bioactive oligosaccharides (including mixed linkage 1,3(4) and 1,3(6)-glucooligo- saccharides, galactooligosaccharides xyloglucooligosaccharides, pectic oligosaccharides, branched and linear xylooligosaccharides, (galacto)glucomannooligosaccharides), glycopeptides and flavonoid glycosides from terrestrial and marine plants, plant residues, fungi and waste streams or by-products rich in simple sugars.

The strain can produce an enzyme composition comprising CBH I (1-20%), CBH II (1-40%), β(1,2)4-glucanase (15-45%), β-glucosidase (2-12%), Xylanase (1-35%), and β-Xylosidase (1-5%). The enzyme composition may further comprise one or more of the following; α-Glucuronidase, α-L-Arabinofuranosidase, exoxylanase, pectinolytic enzymes, starch modifying activity, hemicellulases oxidoreductases/oxidase and esterases and proteases. The composition may be used to increase the bioavailability of biomolecules with natural anti-bacterial and anti-viral activity, including flavonoid and cyanogenic glycosides, saponins, oligosaccharides and phenolics (including ferulic, and p-coumaric acids, epicatechin, catechin, pyrogallic acid and the like).

The strain can produce an enzyme composition comprising CBH I (3-15%), CBH II (3-15%), β(1,3)4-glucanase (25-45%), β-glucosidase (2-15%), Xylanase (10-30%), and β-Xylosidase (0.5-8%). The enzyme composition may further comprise one or more of the following; α-Glucuronidase, α-L-Arabinofuranosidase, exoxylanase, pectinolytic enzymes, starch modifying activity, hemicellulases, oxidoreductases/oxidase and esterases and protease. The composition may be used to increase the bioavailability of natural antioxidant biomolecules, e.g. carotenoids, lycopenes, xanthophylls, anthocyanins, phenolics and glycosides from all plants materials, residues, wastes, inluding various fruit and berries.

The strain can produce to an enzyme composition comprising CBH I (1-25%), CBH II (1-40%), β(1,3)4-glucanase (15-40%), β-glucosidase (2-15%), Xylanase (18-35%), and β-Xylosidase (0.5-12%). The enzyme composition may further comprise one or more of the following; α-Glucuronidase, α-L-Arabinofuranosidase, pectinolytic enzymes, starch modifying activity, hemicellulases, oxidoreductases/ oxidase and esterases and protease. The composition may be used for the generation of feedstocks from raw plant materials, plant residues and wastes for use in microbial production of antibiotics by fungi and bacteria, including *Penicillium sp.* and *Streptomyces* sp.

The strain can produce an enzyme composition comprising CBH I (1-30%), CBH II (140%), β(1,3)4-glucanase (15-40%), β-glucosidase (2-15%), Xylanase (18-35%), and β-Xylosidaso (0.5-8%). The enzyme composition may further comprise one or more of the following; α-Glucuronidase, α-L-Arabinofuranosidase, pectinolytic enzymes, starch modifying activity, hemicellulases, oxidoreductases/ oxidase and esterases; exoxylanase and proteases. The composition may be used in the generation of feedstocks from raw plant materials, plant residues and wastes for use in microbial production of citric acid.

The strain can produce an enzyme composition comprising CBH I (2-15%), CBH II (2-15%), β(1,3)4-glucanase (20-45%), β-glucosidase (2-25%), Xylanase (1-30%), and β-Xylosidase (0.5-8 %). The enzyme composition may further comprise one or more of the following; α-Glucuronidase, α-L-Arabinofuranosidase, pectinolytic enzymes, starch modifying activity, hemicellulases, oxidoreductases/ oxidase and esterases, exoxylanase and proteases. The composition may be used in the production of oligosaccharides from algal polysaccharides (e.g. laminaran and fucoidan) and additives derived from plant extracts, by generally regarded as safe processes, in the formulation of cosmetics.

The strain can produce an enzyme composition comprising CBH I (3-30%), CBH II (1-10%), β(1,3)4-glucanase (10-45%), β-glucosidase (2-12%), Xylanase (1-48%), and β-Xylosidase (0.1-8%), The enzyme composition may further comprise one or more of the following; α-Glucuronidase, α-L-Arabinofuranosidase, pectinolytic enzymes, starch modifying activity, hemicellulases, oxidoreductases/ oxidase and esterases, exoxylanase and proteases. The composition may be used in the production of oligosaccharides and glycopeptides for use as research reagents, in biosensor production and as tools in functional glycomics to probe receptor-ligand interactions and in the production of substrate libraries to profile enzyme-substrate specificity.

The strain can produce an enzyme composition comprising CBH I (5-15%), CBH II (5-30%), β(1,3)4-glucanase (20-45%), β-glucosidase (1-12%), Xylanase (10-30%), and β-Xylosidase (0.5-8%). The enzyme composition may further comprise one or more of the following; α-Glucuronidase, α-L-Arabinofurenosidase, pectinolytic enzymes, enzymes with starch modifying activity, hemicellulases, oxidoreductases/ oxidase and esterases, and proteases. The composition may be used for the production of modified cellulose and β-glucans, cellooligosaccharides, modified starches and maltooligosaccharides, lactulose and polyols (e.g. mannitol, glucitol or dulcitol, xylitol, arabitol).

A substrate produced by any of the above methods may be used as a feedstock in the production of biofuel, and bio-ethanol or bio-gas such as methane or carbon dioxide, whenever produced by that process. The advantage of using this strain to produce bioethanol or bio-gas, is that this is a cost effective method of producing a valuable product which can be used in many industries, from a waste product of little value. At the same time as produging a valuable product, there is a reduction in waste which in turn has a positive environmental impact.

All of the methods described above could be carried out with the enzyme compositions as defined herein, or with the microbial strains described herein.

The invention also provides use of the strain or mutants thereof further comprising use of enzymes derived from other fungal species including Chaetomium thermophile and Thermoascus aurantiacus.

The microorganism strain is Talaromyces emersonii IMI 393751 or a mutant thereof which also is capable of producing enzymes capable of activity at temperatures at or above 55°C.

There is further described a method for obtaining an enzyme system suitable for converting a target substrate the method comprising: obtaining a sample of the target substrate; allowing an inoculum of a microorganism strain to grow on the target substrate and secrete enzymes; recovering the enzymes secreted during growth on the target substrate; determining enzyme activities and enzyme properties; constructing a gene expression profile; identifying enzyme proteins and constructing a protein expression profile; comparing the gene expression with the protein expression profile; purifying the enzymes. The enzymes may then be stored. The method may further comprise analysing the enzymes; and/or designing an enzyme system.

### Detailed Description of the Invention

The invention will be more clearly understood from the following description , given by way of example only with reference to the accompanying drawings, in which:
Fig. 1: Process outline for a method for designing an enzyme system suitable for converting a target substrate.

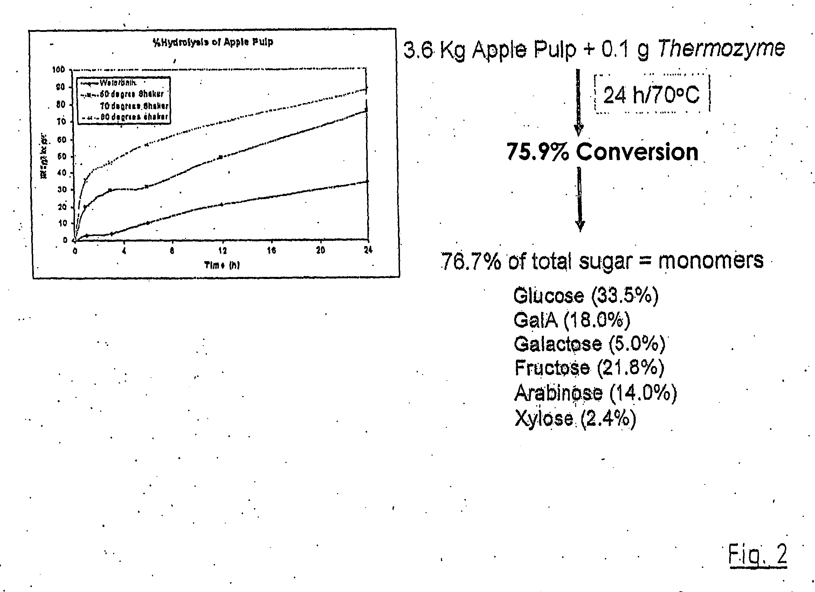

Fig. 2: Generation of a sugar-rich feedstock for biofuel production by thermozyme treatment of apple pulp/pomace.
Fig. 3: Thin layer-chromatogram of the products generated during paper cup hydrolysis.
Fig. 4: Electron microscopy of paper cups before (0h) and after (24h) treatment with the Talaromyces emersonii paper cup induced enzyme cocktail demonstrating extensive hydrolysis of the target substrate.

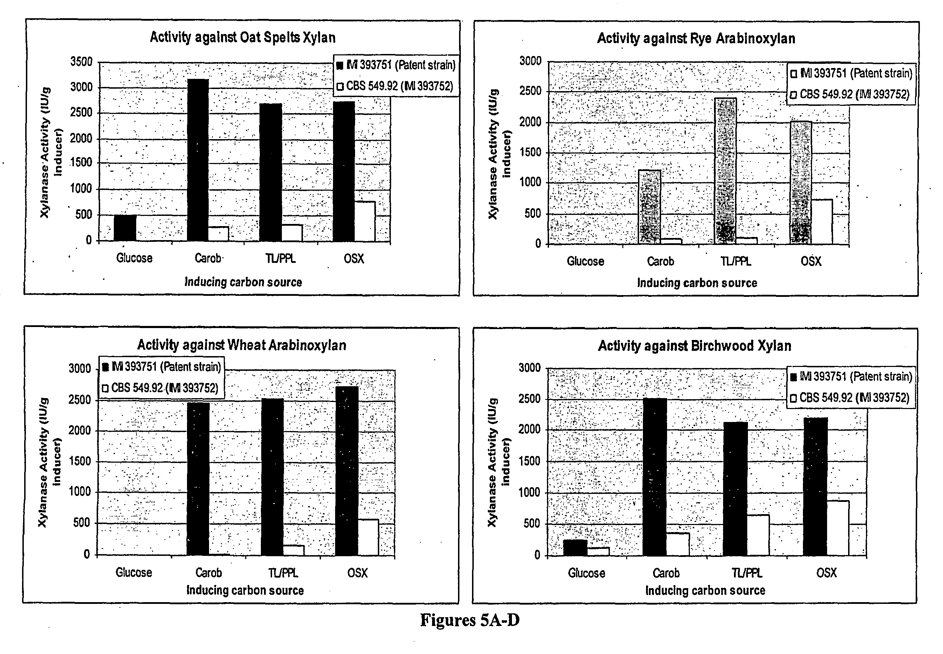

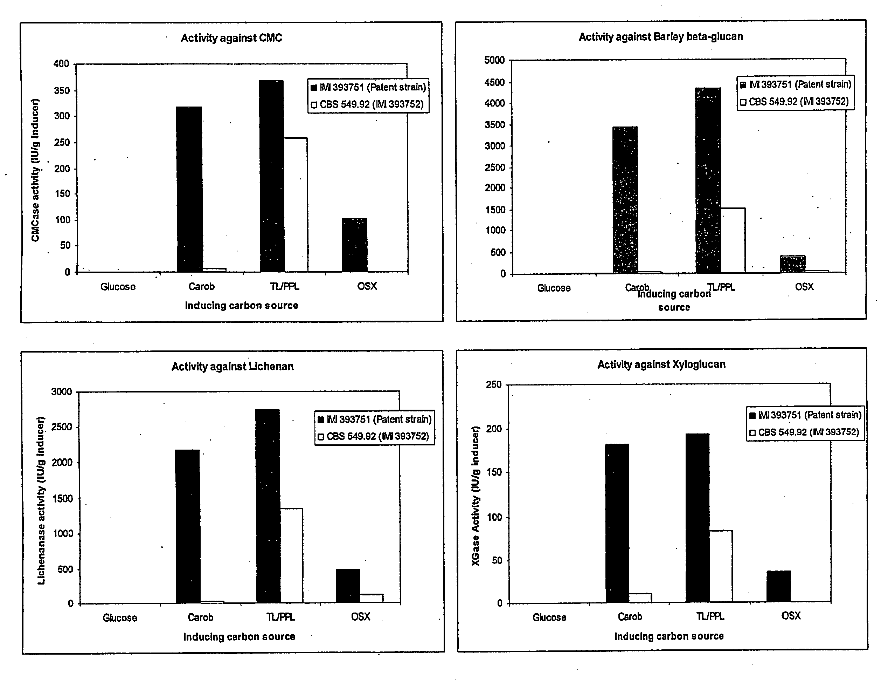

Fig. 5A-D: Comparison of xylanase production by the 393751 strain and the wild type CBS 549.92 (formerly CBS 814.70) strain.
Fig. 6A-E: Comparison of glucanase and (galacto)mannanase production by the 393751 strain and the wild type CBS 549.92 strain.
Fig. 7: Volume reduction of sterilized cellulose-rich clinical waste catalyzed by the 10 enzyme cocktails at 50°C after 24 h.
Fig. 8: Volume reduction of STG sterilized cellulose-rich clinical waste catalyzed by the 10 enzyme cocktails at 70°C after 24 h.
Fig. 9A-B: Untreated cellulose-rich waste (A), and enzymatically-treated waste (B)
Fig. 10A-B: Ethanol production by *S. cerevisiae* on hydrolysates obtained by treatment of sterilized cellulose-rich clinical waste at 70°C for 24 h (60% moisture) with Cocktail 5 (A) and Cocktail 8 (B).
Fig. 11A: The effect of pH on cellulase activity in the *T. emersonii* cocktails.
Fig. 11B: The effect of pH on xylanase activity in the *T*. *emersonii* cocktails.
Fig. 12: The effect of temperature on cellulase activity in the *T*. *emersonii* cocktails.
Fig. 13: The effect of temperature on xylanase activity in the *T. emersonii* cocktails.
Fig. 14: Activity of the purified novel xylanase against different xylans. OSX, Oat Spelts Xylan,
WSX, Wheat straw xylan, LWX, larchwood xylan, BWX, birchwood xylan, RM, Rhodymenan (red algal 1,3;1,4-β-D-xylan). Activity is expressed as a % relative to Oat spelts xylan (100%).
Fig. 15A-B: Activity of purified (A) Xyn IV and (B) Xyn VI against different xylans. Activity is expressed as a % relative to Oat spelts xylan (100%).
Fig. 15C-D: Activity of purified (C) Xyn VII and (D) Xyn VIII against different xylans. Activity is expressed as a % relative to Oat spelts xylan (100%).
Fig. 15E-F: Activity of purified (E) Xyn IX and (F) Xyn X against different xylans. Activity is expressed as a % relative to Oat spelts xylan (100%).
Fig. 15G: Activity of purified Xyn XI, against different xylans. Activity is expressed as a % relative to Oat spelts xylan (100%).
Fig. 16: %Relative Activity of purified Xyn XII against a variety of purified polysaccharides. OSX, Oat spelts xylan; BBG, Barley β-glucan; LIC, Lichenan; CMC, Carboxymethylcellulose; LAM, Laminarin; D1, Dextran; D2, Dextrin; INU, Inulin; ARA, Arabinan; GAL L, Galactan (Lupin); GAL P, Galactan (Potato); RG, Rhamnogalacturonan; LWAG, Larchwood arabino- galactan.
Fig. 17: Specific Activity of purified Xyn XII against aryl β-xylosides and aryl β-glucosides.
Fig. 18: Comparison of the specific activities of selected xylanases expressed by IMI393751 and CBS549.92 against OSX as assay substrate.

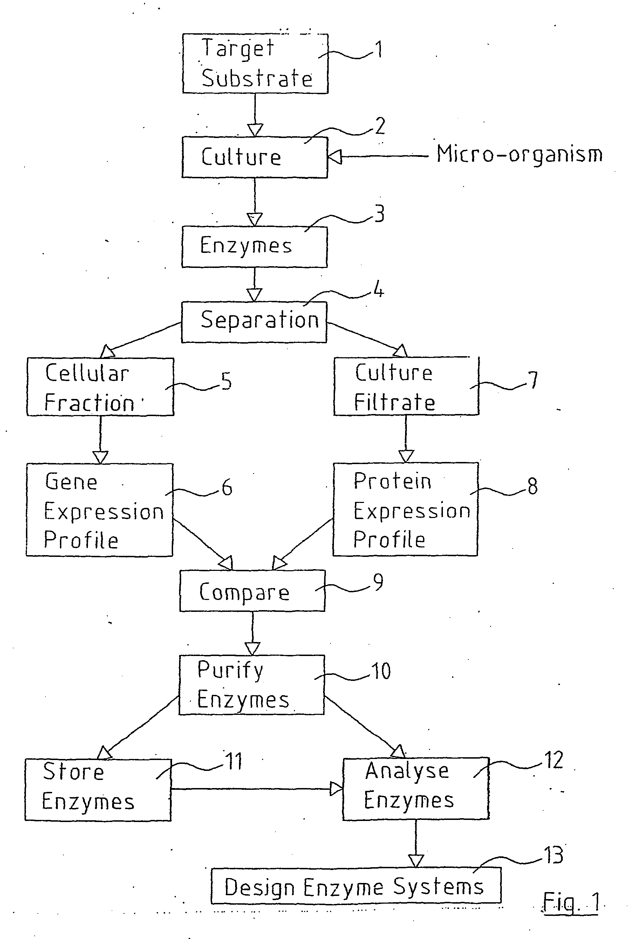

Referring to Fig. 1, there is provided a process outline for designing an enzyme system for converting a target substrate. In step 1 a target substrate is obtained. In step 2 the target substrate is inoculated with the microorganism which is cultivated on the target substrate to provide a culture. The microorganism secretes enzymes and these enzymes are recovered in step 3, by obtaining samples of the culture. The culture samples are separated into cellular fraction and culture filtrate in step 4. The cellular fraction (mRNA) is analysed in step 5, to determine the enzyme activities and properties. In step 6, a gene expression profile is constructed based on the analysis of step 5. In step 7, the culture filtrate is screened for protein activity and a protein expression profile is constructed in step 8. In step 9 the gene and protein expression profiles are compared. In step 10 the enzymes are purified. The enzymes may be stored in step 11 and in step 12 the enzymes may be further analysed and an enzyme system is designed in step 13.

It has been found that when fungus is used as the microorganism, better results are obtained when the substrate is inoculated with the mycelia of the fungus. This cultivation is preferably carried out in a fermenter and the reaction conditions will vary depending on the type of microorganism and substrate used. Cultivation may be either in the form of liquid or solid-state fermentation. For Talaromyces emersonii a cultivation temperature in the region of 45° to 65° is preferable, the enzymes being optimally active up to 85-90°C.

The enzymes are recovered from the target substrate by separation of cellular (fungal) biomass from extracellular culture filtrate using centrifugation in the case of enzymes produced by liquid fermentation, or with the aid of a cell separation system for larger cultures. The enzymes are then recovered from the cellular biomass fraction by homogenisation of a known weight of the biomass in two volumes of buffer. Suitable buffers include 50mM ammonium acetate, pH 4.5-6.0 or 50mM sodium phosphate, pH 7.0-8.0. For the extraction of enzymes for solid state cultures, the cultures were mixed with 10 volumes of 100mM citrate phosphate buffer, pH 5.0 containing 0.01% (v/v) Tween 80, homogenised and extracted by shaking for 2 hours at 140 rpm at room temperature. An enzyme-rich extract is then recovered by centrifugation.

It is essential that a comparison of the enzyme system at both genome and proteome levels is carried out i.e. the genes, expression products (mRNA) and proteins identified are compared. This is due to the fact that there may be genetic information present which is expressed at mRNA level, but not translated to functional protein at the protein level. Transcriptomic, genomic and proteomic analyses are important for determining the relative abundance of certain enzymes.

An isolate of the filamentous fungus strain, from which some of the enzymes have been isolated has been deposited with the International Mycological Institute (IMI) (CABI Bioscience UK), Bakeham Lane, Englefield Green, Egham, Surrey TW20 9TY, United Kingdom for the purposes of patent procedure on Nov 22^{nd} 2005- Deposition No. IMI 393751.After purification of the enzymes they can be optionally stored or directly analysed to obtain (a) detailed information on the individual thermostability/thermal activity in general catalytic and functional properties, (b) detailed information on their mode of action and catalytic potential, individually and in combination, (c) information on synergistic interactions, (d) partial sequence information that would assist in cloning of the genes, and (e) in some cases to obtain sufficient protein to facilitate collection of the 3-D structural data. The analysis of these enzymes is then exploited to identify key enzyme systems and optimum harvesting times for these systems. The systems can be optimised with respect to levels of key activities and key enzyme blends, performance characteristics and conditions (at laboratory scale) for target applications.

### Example 1 T. emersonii IMI393751, isolation

Freshly harvested (∼200 g) clean grass (lawn) cuttings and other mixed plant biomass were placed in a closed container to simulate a composting environment, and incubated in a constant temperature chamber, at 65°C, for approximately 2 h (combined pasteurisation and equilibration of the substrate). Humidity/moisture content was maintained at ∼65-70%. The container was fitted with a line providing a low pulse of moist, filtered air at intervals. After 2 h, a spore suspension (1 x 10⁸ spores in 2% sterile water) of *T. emersonii* (laboratory stocks of a 12 year old isolate, originally from CBS814.70) was used to inoculate the centre region of the biomass. The temperature was maintained at 65°C for 2 days, and increased in 2°C intervals every 24 h thereafter until an air temperature of 70°C was reached internally in the chamber. The culture was grown for a further 7 days before a sample of the inoculated 'hot-spot' or central region was removed aseptically and transferred to agar plates (Emerson's agar medium for thermophilic fungi), and subcultured, to ensure culture purity; purity was cross-checked by microscopic analysis. Liquid media containing basic nutrients (Tuohy *et al.,* 1992; Moloney *et al.,* 1983) and 2% (w/v) glucose was inoculated with 1 cm² pieces of mycelial mat from 36 h old agar plate cultures. Liquid cultures were grown at 55°C for 36 h in 250 mL Erlenmeyer flasks (containing 100 mL of growth medium), with shaking at 220 rpm. Aliquots (2.0 mL) of mycelial suspensions were removed after 36 h, washed aseptically with sterile water, transferred to sterile Petri dishes (10 mm diameter) and irradiated with UV light for timed intervals (10-60 s). Sterile agar media (noble agar containing 0.2% w/v of individual catabolite repressors, such as 2-deoxyglucose) were inoculated with samples of the irradiated fungal mycelium. Replicate cultures were incubated at 45 and 58°C. Single colonies were carefully selected (fluffy white appearance), aseptically transferred to Sabouraud dextrose agar plates and purified further via several transfers. Strain IMI 393751 represents an isolate taken from the plates incubated at 58°C. The mutant was evaluated in a comparative study with the parent organism, for enhanced thermal stability and enzyme production, which revealed clear differences between both strains in terms of culture appearance, ability to sporulate (strain IMI 393751 is non-sporulating), thermophilicity and stability, enzyme production patterns under identical growth conditions, differential expression and levels of individual enzyme activities.

### Physiological/Mycological differences between CBS 814.70 and IMI393751

**Appearance of the culture during growth on different agar media** - when cultivated on Emerson's agar CBS 814.70 has the typical features of this species (Stolk & Samson, 1972), i.e. pale, creamy/buff colour, with pale yellowish shades near the agar surface (around the inoculation zone), which turns a deeper dark buff/reddish brown colour as the culture matures, and consists of dense mycelial mat of many ascomata. In contrast, IMI393751 is much whither and the culture has a very 'fluffy' appearance.

Differences with respect to sporulation - CBS 814.70 is a sporulating strain of T. emersonii which produces conidiophores, asci and ascospores. The conidiophores appear as perpendicular branches on hyphae (pale yellow in colour and septate). The asci, or spore containing sacs are somewhat ellipsoidal in shape, and can often be found in chains; the ascospores are smooth and elliptical in shape (appear green in an electron micrograph). In contrast, IMI393751 mycelia produces very few classical conidiophores, asci and ascospores. In complete contrast to CBS814.70, IMI393751 only produces a paltry number of spores under extreme and quite specific conditions, whereas strain CBS 814.70 produces spores on several different media.

**Differences With Respect To Thermophilicity And Optimum Growth Temperature Range.** CBS 814.70 has a strict growth range from 35-554°C, with poor growth at lower or higher temperatures and an optimum growth range of 40-45°C. IMI393751, on the other hand, exhibits a wider growth temperature range of 30-65°C, with good growth at 80oC, 85oC and up to 90°C, with an optimum between 48-55°C. IMI393751 grows very well >55°C and continues to produce high levels of fungal biomass (mycelia) and to actively secrete significant quantities of various proteins.

**Differences with respect to growth at higher pH values** - CBS 814.70 does not grow well (in fact starts to die off) at pH values above pH 6-6.5 (previous data, Tuohy & Coughlan, 1992), whereas IMI393751 grows well and secretes substantial levels of enzymes at pH values up to pH 9.0.

**Growth under nutrient limiting/depleted conditions.** IMI393751 survives well in nutrient depleted cultures for up to 55 days, whereas CBS 814.70 undergoes autolysis from 7 days onwards and few surviving mycelia/cells remain after 15 days. Mycelia harvested from 55-day old nutrient depleted liquid cultures of IMI393751 could be resuscitated by transfer to sabouraud dextrose agar media, whereas mycelial remnants of the CBS814.70 strain (55-day old) could not be revived by the same approach.

**Example 2: an enzyme system from Talaromyces emersonii for converting hemicellulosic materials.** The complete hydrolysis of xylan requires the synergistic action of xylanases, β-xylosidase, α-glucuronidase, α-L-arabinofuranosidase and esterases. Table 1. gives an example of selected target substrates (including wastes/residues) and the percentage inducing carbon source used in this example. The percentage induction refers to the weight of carbon source per volume of medium (g/100mls).

**Table 1: Growth substrates/inducing carbon sources for enzyme production by T. emersonii**

| **Carbon Source** | **Abbreviation** | | **% Inducing carbon source** | |
|---|---|---|---|---|
| Beechwood xylan | | BEX | | 1.0 |
| Birchwood xylan | | BWX | | 1.0 |
| Oat spelt xylan | | OSX | | 1.0 |
| Larchwood xylan | | LWX | | 0.3 |
| Wheat arabinoxylan | | WAX | | 1.0 |
| Spruce shavings | | SS | | 2.0-6.0 |
| Packing material | PK | | 0.5-6.0 | |
| Cereal straws | | CS | | 1.0-2.0 |
| Paper Cups | | PC | | 2.0-6.0 |
| White Office paper | | WOP | | 2.0-6.0 |
| Tea Leaves | | TL | | 2.0-4.0 |
| Methyl xylose | | MEX | | 0.2 |
| Xylose | | Xyl | | 1 |
| Glucuronic acid | GlcA | | 1 | |
| Solka floc | | SF | | 2^ |
| Glucose | | Glc | | 2^ |

| | | | | |
|---|---|---|---|---|
| ^Included for comparative purposes. | | | | |

Based on the results obtained, an enzyme system was designed using enzymes purified from Talaromyces emersonii for the degradation of a hemicellulose (xylan) or xylan-rich wood-derived product, residue or waste. The relative amounts of the key enzymes present in the enzyme system are tabulated in table2.

**Table 2: A system for degradation of a hemicellulose or xylan rich wood product.**

| **ENZYME^** | **% REQUIRED** |
|---|---|
| Xylanase | 10-50% |
| Exoxylanase | 4.0-25.0% |
| β-Xylosidase | 0.1-5.0% |
| α-Glucuronidase | 0.2-4.0% |
| α-L-Arabinofuranosidase | 0.1-5.0% |
| Accessory biopolymer-modifying activities | 25-50% |
| (e.g. cellulase, non-cellulolytic β-glucanase, pectinolytic activities, mannanolytic activities, acetyl(xylan) esterase, oxidoreductase, protease) | |

The relative amounts of the core activities, the profile and relative amounts of the accessory activities will vary depending on the composition of the target substrate. Table 3 outlines enzyme systems from Talaromyces emersonii which have been designed for specific applications.

**Table 3: Specific enzyme systems suitable for the degradation of oligosaccharides from wood xylans, newsprint and woody residues.**

| **Enzyme system & composition** | **Selected Target applications/ substrate** | **Enzyme dosage**** | **%Hydrolysis of carbohydrate** |
|---|---|---|---|
| TE Woodcell (LWX-induced)* | Oligosaccharides from Wood xylans | 15-30 nkat/g xylan | 91-100% (1-3 h) |
| 15-30% Xylanase^ | | | |
| 0.2-1.0% β-Xylosidase 0.1-3.0 % α-Glucuronidase 0.1-3.0 % α-L-Arabinofuranosidase | Bioconversion of Newsprint | 11.5-46.0 nkat/g newsprint^{$} | 70-85% of carbohydrate present^{%} |
| | | 46.0-150 nkat/g | |
| 7-10% Cellulase 20-35% non-cellulosic glucanase, pectinolytic enzymes, esterases) | Bioconversion of woody residues (e.g. sawdust, shavings, virgin wood, bark, etc.) | woody residues | 34-50% (70°C/24 h)^^ 35-55% (80°C/24 h^^ |

| | | | |
|---|---|---|---|
| ^Xylanase levels were measured with different model xylans as substrates *LWX, Larchwood glucuronoarabinoxylan **Xylanase levels: 45.2 IU/mL (753.5 nkat/mL), 40.9 IU/mL (681.8 nkatmL) and 27.5 IU/mL (458.4 nkat/mL) with Larchwood xylan, Birchwood xylan and Oat Spelts xylan as assay substrates, respectively; at the time of harvest, - where nkat = nanokatals (16.67IU/mL) ^{$}Depending on the grade of newsprint, i.e. coloured versus blackand white, polished versus roughly finished/recycled, etc. %Cellulose, 40-55% and Hemicellulose, 25-40% as the main types of carbohydrates present ^^% Conversion of Softwood substrate (unmilled and roughly milled fractions, untreated and pretreated with dilute acid). Total %Conversion can be increased (up to 74%) using this enzyme system in a blend with other T. emersonii or Chaetomium thermophile enzyme systems, or amplication of key enzyme activities by addition of recombinant enzyme. | | | |

### Example 3: System from Talaromyces emersonii for converting cereals, beet pulp, and other materials (including wastes) rich in arabinoxylans and acetylated hemicelluloses.

Table 4 gives relative amounts of different enzyme activities in designed enzyme systems from Talaromyces emersonii IMI 393751and two previously identified mutant strainsdesigned for conversion of cereals, beet pulp, and other materials (including wastes) rich in arabinoxylans and acetylated hemicelluloses.

**Table 4: Enzyme activities of systems for conversion of materials rich in arabinooxylans and acetylated hemicelluloses**

| **Enzyme Preparation/composition** | **T. emersonii (IMI 393751; 1:1 WB/BP as inducer)** | **T. emersonii (IMI 393751; Carob powder as inducer)** | **T. emersonii (mutant TC2; WB as inducer)** | **T. emersonii (mutant TC5; Tea leaves as inducer)** |
|---|---|---|---|---|
| | Enzyme activity profile (%) | Enzyme activity profile (%) | Enzyme activity profile (%) | Enzyme activity profile (%) |
| Xylanase | 20-30% | 15-25% | 35-50% | 12-20% |
| Exoxylanase | 5-10% | 2-5% | 10-15% | 2-8% |
| β-Xylosidase | 0.5-2.0% | 0.2-1.5% | 0.5-1.5% | 0.1-1.0% |
| α-Glucuronidase | 0.5-2.0% | 0.2-1.5% | 0.2-1.5% | 0.2-1.5% |
| α-L-Arabinofuranosidase | 0.5-2.0% | 0.2-2.0% | 0.2-1.2% | 0.1-1.5% |
| Biopolymer modifying enzymes including acetyl esterase and acetyl xylan esterase | 30-45% | 32-50% | 20-35% | 25-60% |

**Example 4: System from Talaromyces emersonii for converting non-cellulosic materials, such as tea leaves and carob powder.** Characterisation of three novel endoglucanases (EG)), which are important for both selective modification and degradation of a variety of cereal residues, including potential candidates for brewing and animal feedstuff, such as sorghum, maize and rye. These three enzymes have been purified from Talaromyces emersonii IMI393751.

The total carbohydrate content of purified enzyme preparations was determined by the phenolsulfuric acid method (Dubois et al.) by reference to glucose or mannose standard curves (20-100 µg.mL⁻¹).

**Protein Separation Techniques** Sequential gel filtration, ion-exchange, hydrophobic interaction and lectin affinity chromatographies and chromatofocusing (pseudo ion-exchange) was required to obtain highly purified preparations of EG V, EG VI and EG VII. Non-denaturing gel electrophoresis and isoelectric focussing followed by coomassie blue.staining was performed by standard methodology known to one in the field and give the relevant references (e.g. Murray *et al.,* 2001; Tuohy & Coughlan,. 1992, Tuohy *et al* 2002; Maloney *et al*., 2004). Initial experiments revealed that the pI values of EG V-VII were <pH 3.5. Therefore, chromatofocusing on PBE 94, pre-equilibrated with 0.025 M piperazine-HCl, pH 3.5, was used to determine accurate pI values for EG V-VII. the pH corresponding to the elution peak for β-glucanase was determined to be the pI. Differences of 0.01 pH units could be detected thus yielding accurate pI values for each enzyme.

**pH And Temperature Optima And Stabilities** Optimum pH values were determined by monitoring the enzyme activity at pH values between 2.5 and 9.0, at 50°C, in constant ionic strength citrate phosphate buffer. To evaluate the effects of pH on the activity of EG V-VII, purified samples of each enzyme were incubated at pH 4.0, 5.0 and 6.0 at 50°C. Aliquots were removed after 0, 1, 2, 3, 6, 9,12, 24, 36, 48 and up to 336 h (14 d), diluted appropriately in 100 mM NaOAc buffer, pH 5.0 (normal assay pH) and assayed for residual activity according to the standard assay method. The stability of each purified enzyme was investigated at 50°C, 60°C, 70°C and 80°C, in the absence of substrate. EG V-VII were not modular proteins in that none of the three enzymes adsorbed to cellulose (or other insoluble carbohydrates, i.e. they did not contain carbohydrate binding

Substrate specificity on various polysaccharides and synthetic glycosides was evaluated by measuringactivity against a wide variety of carbohydrates (BBG, lichenan, CMC, other polysaccharides and synthetic glycosides) using the normal β-glucanase assay procedure.

**Effects Of Metal Ions, Chemical Modification Reagents And Potential Inhibitors** A range of monovalent, divalent and heavy metal ions, at final assay concentrations of 1.0 mM, were investigated for their potential effects on the activities of EG V, EG VI and EG VII, by incubating the enzymes with the chemicals and then conducting the normal enzyme assay. Finally, the inhibitor effects of glycosides, such as disaccharides, lactones and flavanoid glycosides on the purified enzymes were examined. Specified concentrations of each glycoside were included in normal assay cocktails and residual activity determined by comparison with controls (no glycoside present).

A summary of the purification of EG V, EG VI and EG VII, and purification parameters such as yield (%) are given in Table 5.

**Table 5: Summary of the purification of endoglucanase enzymes from Talaromyces emersonii**

| **Purification Step** | **Total Activity (IU)** | **Total Protein (mg)** | **Specific Activity (IU.mg⁻¹)** | **Purification Factor (x-fold)** | **Yield (%)** |
|---|---|---|---|---|---|
| Crude Extract | 54,806.2 | 573.1 | 95.6 | 1.00 | 100.00 |
| Ultrafiltration (Amicon DC2) | 50,613.4 | 452.2 | 111.9 | 1.17 | 92.30 |
| Propan-2-ol pptn. | 50,016.9 | 364.8 | 137.4 | 1.44 | 91.30 |
| Anion-exchange(DE-52, pH5.5) Phenyl Sepharose CL-4B | 25,064.7 | 240.1 | 104.4 | 1.10 | 45.70 |
| Peak 1 | 3,862.6 | 24.9 | 154.9 | 1.62 | 7.10 |
| Peak 2 | 6,763.5 | 30.8 | 219.3 | 2.29 | 12.30 |
| Peak 3 | 14,411.1 | 42.1 | 342.3 | 3.58 | 26.30 |

| EG VII (Peak 1) | | | | | |
|---|---|---|---|---|---|
| Concanavalin A | 3,120.2 | 11.3 | 275.9 | 2.89 | 5.70 |
| Sephacryl S-100 HR | 670.1 | 3.2 | 212.5 | 2.22 | 1.20 |
| Chromatofocusing | 400.4 | 0.53 | 756.9 | 7.92 | 0.71 |

| EGV, EGVI (Peak 3) | | | | | |
|---|---|---|---|---|---|
| Anion-exchange (DE-52, pH4.5) | 4,662.3 | 10.40 | 447.4 | 4.68 | 8.50 |
| Anion-exchange (DE-52, pH7.0) | 3,339.2 | 5.77 | 579.1 | 6.06 | 6.10 |
| BioGel P-60 | 3,043.8 | 3.60 | 855.7 | 8.95 | 5.50 |

| Concanavalin A | | | | | |
|---|---|---|---|---|---|
| Peak 1 | 1,115.6 | 1.60 | 679.1 | 7.10 | 2.10 |
| Peak 2 | 863.50 | 1.20 | 738.7 | 7.73 | 1.60 |

| Chromatofocusing PBE 94, | | | | | |
|---|---|---|---|---|---|
| pH 3.5-2.0 | | | | | |
| EG V | 415.3 | 0.54 | 764.8 | 7.99 | 0.76 |
| EGVI | 168.7 | 0.18 | 947.6 | 9.09 | 0.31 |

Yields, as well as final specific activity values, were similar for EG V and EG VII, while EG VI was obtained at a lower yield and had a higher specific activity.

**Enzyme homogeneity Mᵣ and pI values** Positive staining with Schiff reagent (results not shown) indicated that all three enzymes were single subunit glycoproteins. The homogeneity of each enzyme preparation was verified by IEF. The pI values obtained were as follows: EG V, 2.45; EG VI, 3.00; EG VII, 2.85.

**Evidence for glycosylation and estimation of molar extinction coefficients** Total carbohydrate contents (w/w) were 22.6 ± 0.1 %, 14.7 ± 0.1 % and 65.9 ± 0.04% for EG V, EG VI and EG VII, respectively. Calculated molar extinction coefficient (ε₂₈₀) values (mol.1.cm⁻¹) were 1.04 x 10⁻⁵ for EG V, 8.80 x 10⁻⁶ for EG VI and 7.05 x 10⁻⁶ for EG VII.

**pH and temperature optima and stabilities** The three enzymes were active over relatively broad pH ranges but exhibited acidic pH optimum values of 5.7, 5.4 and 5.7 for EG V, EG VI and EG VII, respectively. A pH optimum of 4.5 was obtained for BBGase activity in theT. emersonii crude extract. Approximately 75% of the optimum activity was evident between pH 3.0 and 7.0 (EG V), pH 2.5 and 7.5 (EG VI) and pH 2.8 and 7.5 (EG VII).

**The release of reducing sugars from BBG,** at pH 5.0, over 10 min, at 30-90°C. Optimum temp values for activity were 78.0°C, 78.0°C and 76.0°C for EG V, EG VI and EG VII, respectively. Activation energies (Eₐ), estimated from Arrhenius plots, were 21.2 ± 0.05 kJ.mol⁻¹ for EG V, 23.5 ± 0.02 kJ.mol⁻¹ for EG VI, and 26.7 ± 0.05 kJ.mol⁻¹ for EG VII.

The effect of pH on enzyme stability was investigated at pH 5.0, pH 5.5 and pH 6.0 (at 50°C and 70°C). pH stability was greatest in the range pH 4.0-7.0, over a 1 h incubation period, with a marked decrease observed at pH < 4.0 and > 7.0 EG V lost no activity at pH 5.0 and 50°C over a period of 15 d, while EG VI and EG VII lost minimal activity-(13.0% and 11.5% respectively). However, at 70°C and the same pH, EG V, EG VI and EG VII lost 42%, 35% and 33% respectively, of the original activity in each sample over a 60 min period. At pH 5.5 (50°C), EG V, EG VI and EG VII lost 30%, 22% and 8% of their respective original activities over a 15 d period, but at 70°C, EG V was destabilised further (58% decrease in original activity after 60 min), while the activity of EG VI and EG VII after 60 min was very similar to that obtained following incubation at pH 5.5 and 70°C. EG V was considerably less stable at.pH 6.0 and 50°C losing 63% of its original activity in 15 d. EG VI and EG VII were considerably more stable at the latter pH with similar stabilities to those determined at pH 5.5 (30% loss of activity for both enzymes). By increasing the incubation temperature to 70°C, the activity of EG V decreased markedly (65%) over a 60 min incubation period (at pH 6.0), while EG VII remained remarkably stable losing only 22% of its original activity. Half-life (T½) values were in excess of 15 d at pH 5.0 and 50°C, while at 70°C values ranged from 67 min (EG V) to > 80 min (EG VI and EG VII.

**Evidence for modular structure** EG V, EG VI, and EG VII adsorbed weakly to Avicel (microcrystalline cellulose) at pH 5.0 and 4°C (8-15% adsorption); however, this extent of adsorption is not indicative of the presence of a carbohydrate binding module (CBM).

**Effect of metal ions, chemical modifiers and potential inhibitors on enzyme activity** The effects of 1 mM final concentrations of a range of mono, di and multivalent cations on the activity of EG V, EG VI and EG VII, were investigated. Activity was expressed as a % relative to a control (no metal ion present in incubation mixture). In general, heavy metal ions are thought to inactivate enzymes by forming covalent salts with cysteine, histidine or carboxyl groups. While a number of the metal ions, e.g. Mg²⁺, Zn²⁺ and Mo⁶⁺ enhanced the activity of all three enzymes and ions such as Ag⁺, Fe²⁺ and especially Hg²⁺ markedly decreased activity of EG V-VII, noticeable inter-enzyme differences were noted for the effects of other metal ions. Chloride salts of Na⁺ and K⁺ had either no effect or slightly stimulated the activity of each enzyme, e.g. K⁺ increased the activity of EG VII by 20%, while Na⁺ enhanced the activity of EG V by >39%. Ba²⁺ and Ca²⁺ decreased the activity of EG V by 16.5% and 21.5-24.6%, respectively, while both ions increased the activity of EG VI by 37.6% and 10%. Other divalent cations such as Cd²⁺, Co²⁺ and Cu²⁺ exerted a noticeably inhibitory effect on EG V (∼26.5-77.4% loss of activity). Almost total inhibition of the activity of all three enzymes by Hg²⁺ suggests the presence of an essential thiol group(s) involved in catalysis. Valency was noted to modulate activity, for example, Fe³⁺ exerted a more potent negative effect on the activity of all three enzymes (35.4-88.0% decrease in activity), in contrast to Fe²⁺, which actually enhances the activity of EG V by 20%, has minimum effects on EG VII and decreases the activity of EG VI by 38.6% (Fe³⁺ decreases the activity of EG VI by 53.6-59.3%). The nature of the counteranion, i.e. Cl⁻ versus SO₄²⁻ had a profound effect on activity where both salts of a particular anion were investigated. SO₄²⁻ salts of Ca²⁺ and Fe³⁺ selectively enhanced the activity of EG VII ∼2-fold and ∼4.5-fold, respectively, relative to the activity observed with the corresponding Cl⁻ salt. By contrast, the SO₄²⁻ salt of Mg²⁺ markedly decreased the activity of EG V (54.2%) and EG VI (50.1%) relative to the Cl⁻ salt of the same cation. A selection of reagents known to modify amino acid R-groups in proteins were tested for their effects on EG V, EG VI and EG VII, both in the absence and presence of substrate (substrate-protective effects could be observed in this manner) (see Table 6).

**Table 6: Chemical modification studies to identify amino acid groups essential for catalysis in EG V, EG VI and EG VII**

| **Chemical reagent** | **Enzyme + reagent^{a}** | | | **Substrate + reagent^{b}** | | | |
|---|---|---|---|---|---|---|---|
| | **EGV** | **EV VI** | **EG VII** | **EG V** | **EGVI** | **EG VII** | |
| Control | 100.0 | 100.0 | 100.0 | 100.0 | 100.0 | 100.0 | |
| DL-DTT | 218.9 | 514.3 | 575.0 | 264.3 | 915.5 | 348.6 | |
| Dithioerythritol | 270.6 | 657.2 | 773.8 | 271.9 | 917.90 | 360.9 | |
| Dimethylsuphoxide | 125.5 | 124.3 | 194.0 | 93.6 | 141.7 | 100.0 | |
| Sodium borohydride | 96.1 | 125.0 | 194.0 | 81.5 | 104.8 | 82.8 | |
| Sodium periodate | 181.0 | 357.1 | 300.0 | 283.3 | 1001.2 | 469.7 | |
| Woodward's Reagent K | 162.7 | 280.0 | 305.9 | 121.1 | 266.7 | 143.0 | |
| Thioglycolic acid | 211.1 | 187.1 | 188.1 | 148.1 | 269.0 | 150.0 | |
| o-Phthaldialdehyde | 106.1 | 100.0 | 155.0 | 94.6 | 139.0 | 98.8 | |
| N-Bromosuccinimide (NBS) | 0.0 | 0.0 | 0.0 | 0.0 | 0.0 | 0.0 | |
| p-Chloromercuribenzoate | 125.4 | 115.4 | 105.4 | 89.7 | 107.1 | 91.6 | |
| p-Hydroxymercuribenzoate | 92.8 | 98.3 | 99.5 | 261.7 | 125.0 | 94.8 | |
| 2,3,5-Triphenyltetrazolium Chloride | 97.5 | 97.6 | 98.5 | 89.1 | 97.4 | 100.0 | |
| Iodoacetamide | 103.6 | 99.9 | 101.6 | 87.4 | 122.6 | 83.7 | |
| Iodine | 143.4 | 180.6 | 200.0 | 97.2 | 147.6 | 104.8 | |
| Cysteine | 163.8 | 170.4 | 196.5 | 237.8 | 370.20 | 367.7 | |

| 1^{st} Incubation (30 min) Enzyme+ | | 2^{nd} Incubation | | EG V | EGVI | | EG VII (30min) + NBS |
|---|---|---|---|---|---|---|---|
| Control | | | X | 100.0 | 100.0 | | 100.0 |
| Cysteine | | | √ | 117.0 | 217.0 | | 167.0 |
| DDT | | | √ | 204.3 | 140.0 | | 94.6 |
| NBS | | | √ | 0.0 | 0.0 | | 0.0 |

| | | | | | | | |
|---|---|---|---|---|---|---|---|
| ^{a} Preincubation of enzyme with reagent for 30 min (50C) prior to addition of substrate ^{b} Preincubation of substrate with reagent for 30 min (50C) prior to addition of enzyme | | | | | | | |

The potent inhibitory effect of N-bromosuccinimide (NBS) suggests the involvement of tryptophan in binding and/or catalysis. However, o-phthaldialdehyde, another tryptophanmodifying reagent has no net effect on the activity of EG V, EG VI or EG VII, therefore NBS could be modifying another amino acid residue with which it is known to have side reactivity, e.g. cysteine. Furthermore, the failure of the substrate to protect against enzyme inactivation would rule out a direct role for tryptophan in binding or catalysis. As cysteine and dithiotreitol (DTT) both protected against inactivation, the effect of NBS may be due to side-reactions, e.g. oxidation of cysteine. The sulphydryl reagents iodoacetamide, p-hydroxymercuri-benzoate and N-ethylmaleimide cause little or no inhibition either in the presence or absence of substrate, which would seem to suggest that EG V, EG VI and EG VII do not have essential thiol groups. However, cysteine, DTT and dithioerythritol activate all three enzymes, especially EG VI and EG VII, which may suggest the reduction of a disulphide oxidized perhaps during extraction and/or enzyme purification, thus restoring the native conformation of the active site region of the enzyme, or the enzyme molecule as a whole. Sodium borohydride, a strong reducing agent, inhibits the three enzymes (especially EG VI) in the presence of substrate. By contrast, the oxidation of other reactive groups at the active site by the action of strong oxidizing agents such as sodium periodate, iodine and thioglycolic acid notably enhance the activity of all three enzymes with the effects being most pronounced with sodium periodate and EG VI, in the presence of substrate. Woodward's reagent K, a carboxylate-modifying reagent enhanced the activity of EG V, EG VI and EG VII, being most effective in the absence of substrate.

In general, phenolic substances, such as m-phenylphenol, (-)epicatechin, (+)catechin, o-coumaric acid, caffeic acid, ferulic acid, syringic acid, and tannic acid, to name but a few of the compounds tested, did not have a marked inhibitory effect on enzyme activity (in the absence of substrate) with the exception of tannic acid which is a potent inhibitor due to its protein precipitating function. In fact, some of the compounds, e.g. protocatechuic acid, syringic acid, cafeic acid and polyvinylalcohol markedly activated all three enzymes (results obtained during preincubation of enzyme with inhibitor in the absence of substrate). However, when co-incubated with enzyme and substrate, several of the phenolics were noted to effect a decrease in activity of ∼7-32% (substrate did not protect any of the enzymes against the potent effect of tannic acid).

In general, the majority of detergents tested did not result in loss of enzyme activity. However, taurocholic acid, taurodeoxycholic acid, Tween 20 and Tween 80 all decreased the activity of EG V, by 47.7%, 22.7%, 12.7% and 30.8% respectively, when pre-incubated with enzyme in advance of the addition of substrate. With the exception of Tween 80 (34.2% loss of activity), simultaneous incubation with substrate restored full activity. Enhanced activity was observed when substrate, enzyme and detergent (deoxycholic acid, CHAPS, taurodeoxycholic acid and chenodeoxycholic acid) were incubated simultaneously, e.g. the activity of EG VI was increased > 2 fold in the presence of substrate and deoxycholic acid.

Glycosides such as salicin, esculin and arbutin had no apparent effect on the activity of EG V-VII, similar to the disaccharides melibiose, maltose, sucrose and the alditol, sorbitol. However, concentrations of cellobiose from 50-75 mM markedly inhibited all three enzymes, especially EG V, which was also inhibited by lactose at concentrations > 75 mM. Lactose also effected ∼50% inhibition of EG VI and EG VII BBGase activity at concentrations of ∼120 mM. Glucono-δ-lactone and glucoheptono-1,4-lactone also inhibit EG V-VII but at much higher concentrations (500 to >750 mM for 50% inhibition).
**Substrate specificity** Crude extracts of T. emersonii, catalyse the hydrolysis of a variety of polysaccharides including cellulose, CMC, BBG, laminaran, lichenan, xylan, pectin, and a spectrum of synthetic glycoside derivatives. None of the three purified enzymes were active against 4-nitrophenyl derivatives of β-D-xylopyranoside, or α-D-galactopyranoside (Table 7).

**Table 7: Substrate specificities of purified EG V, EG VI and EG VII from Talaromyces emersonii**

| **Substrate** | | | **% Relative Activity^{a}** | | |
|---|---|---|---|---|---|
| | **Linkage** | **40.7kDa 'lichenase'** | **EG V** | **EG VI** | **EG VII** |
| Barley β-glucan | β-(1,3)(1,4) | 100.0 | 100.0 | 100.0 | 100.0 |
| Mixed DP BBG | β-(1,3)(1,4) | 159.0 | 165.0 | 230.0 | 107.6 |
| High DP BBG | β-(1,3)(1,4) | 118.0 | 249.9 | 342.8 | 144.1 |
| CMC | β-(1,4) | 0.5^{c} | 74.9 | 58.4 | 57.7 |
| Lichenan | β-(1,3)(1,4) | 118.9 | 204.3 | 177.7 | 166.9 |
| Laminaran | β-(1,3) | 0.0 | 0.0 | 21.3 | 18.1 |
| Xylan | β-(1,4) | 0.0 | 1.8 | 0.7 | 1.5 |
| Rhodymenan (xylan) | β-(1,3)(1,4) | 0.0 | 35.4 | 9.3 | 0.0 |
| Pectin | α-(1,4) | 0.0 | 0.0 | 0.0 | 0.0 |
| Mannan | β-(1,4) | 0.0 | 0.0 | 0.0 | 0.0 |
| Avicel | β-(1,4) | 0.0 | 0.0 | 0.0 | 0.0 |
| Filter Paper | β-(1,4) | 0.0 | 0.0 | 0.0 | 0.0 |
| 4NP-β-D- Glucopyranoside | β-(1,4) | 0.0 | 0.0 | 0.0 | 0.0 |
| 4NP-β-D- Galactopyranoside | β-(1,4) | 0.0 | 0.0 | 0.0 | 0.0 |
| 4NP-β-D- Xylopyranoside | β-(1,4) | 0.0 | 0.0 | 0.0 | 0.0 |

| | | | | | |
|---|---|---|---|---|---|
| ^{a} % Activity relative to activity against BBG, which is taken as 100.0%; ^{b}Purified Talaromyces emersonii lichenase [6]; ^{c} After 24h at 50°C | | | | | |

Furthermore, EG V, EG VI and EG VII did not display any activity against filter paper, Avicel, locust bean gum galactomannan, and did not catalyse the oxidation of cellobiose, even on extended incubation with substrate. The results are expressed in terms of % activity relative to the control (activity against BBG assigned a value of 100%). All three enzymes exhibit maximum activity against the mixed linkage β-glucans, BBG and lichenan, with markedly more activity on the latter substrate. Trace activity exhibited by all three enzymes with xylan on extended incubation periods may be explained by the fact that the oat spelts xylan preparation used contained minor, contaminating amounts of β-glucan.

After carrying out similar analysis on each of the enzymes expressed, an enzyme system was designed using enzymes purified from Talaromyces emersonii for the degradation of non-cellulosic material such as tealeaves, carob powder and other similar materials.

Table 8 gives an example of the relative amounts of different enzyme activities for this enzyme system.

**Table 8: System for conversion of non-cellulosic materials e.g. tea leaves, carob powder.**

| **Enzyme Composition** | **Enzyme activity profile (%)** |
|---|---|
| β-glucanase | 45.0-55.0 |
| Xylanase | 16.5-42.0 |
| β-glucosidase | 0.5-2.0 |
| β-xylosidase | 0.1-1.0 |
| Protease | 0.1-1.0 |
| Additional hemicellulase enzymes including β-galactosidase, esterases, α-glucuronidase etc. | 10.0-18.0 |

### Example 5: System from T. emersonii for converting cellulose, cellulose-rich wastes and cellooligosaccharides

Talaromyces emersonii IMI 393751 was grown on a variety of paper wastes and paper products as substrates. The enzymes excreted were extracted and enzyme expression was monitored and quantified by proteome and transcriptome analyses and by a thorough spectrum of functional assays. Several paper wastes proved to be excellent inducers of cellulases (and complementary activities, e.g. starch-hydrolysing enzymes, where coated/finished paper products were used). Differences were clearly evident with respect to the relative amounts/types of cellulase enzymes induced by different paper wastes/products. The data obtained confirmed that cellobiohydrolase I (CBH I) and cellobiohydrolase II (CBH II) isoenzymes were the most important cellulase activities and, where more complete saccharification/bioconversion of cellulose in the target substrate was desired (e.g. generation of monosaccharide-rich feedstocks for biofuel production), β-glucosidase I (BG I) was also very important.

Paper plates induced remarkable levels of filter paper (FP) degrading activity (1573 IU/g, where IU represents µmoles product formed/min reaction time/g inducing substrate), low endocellulase levels (27.5 IU/g) and low β-glucosidase levels (6.05 IU/g). At a transcriptome level, CBH I was the most abundant/highly expressed cellulase, an observation complemented at functional level with 242.0 IU/g CBH I type activity being detected. The enzymes produced during growth on paper cups were significantly more exo-acting, with 495.0 IU/g FP activity and 53.9 IU/g β-glucosidase being detected. In the latter example, gene expression and functional assays indicated that CBH II was the key cellulase (transcript and enzyme levels for CBH II were ∼2-fold the corresponding levels for CBH I enzymes). CBH II was again the key cellulase induced by brown paper, corrugated cardboard and white office paper. Individual enzyme systems, and combinations thereof (e.g. for the amplification of key exo-or side/accessory activities), were shown to be effective tools for the conversion of cellulose (and hemicelluloses/other carbohydrates) in a wide variety of cellulose-rich virgin, secondary and waste materials.

Enzymes were isolated and analysed by conventional procedures (Walsh, (1997) and *et al*., 2002)

CBH IA, containing traces of xylanase as the only contaminating activity, eluted at a NaCl concentration between 115 and 170 mM. Fractions 52-66 were pooled and dialyzed for 16 h against 4 changes of 100 mM ammonium acetate buffer, pH 5.0, and subjected to affinity chromatography on a column (1.4 x 11.3 cm) of CH Sepharose 4B substituted with p-aminobenzyl-1-thio-cellobioside. The residual contaminating xylanase activity did not bind and was eluted in the application and wash buffers. CBH IA was eluted using 0.1 M lactose in 100 mM ammonium acetate buffer, pH 5.0. Fractions 13-21 were pooled, dialyzed against distilled water to remove lactose and stored at 4°C until used.

CBH IB from the anion exchange step (DE-52 at pH 5.5) was dialysed versus 100 mM ammonium acetate buffer, pH 5.5 and applied to the affinity column as for CBH IA. The residual contaminating activities, mainly endoglucanase, did not bind to the affinity matrix and were eluted in the wash. CBH IB was specifically eluted using 0.1 M lactose in affinity buffer. Fractions 43-48 were pooled and dialysed against distilled water to remove lactose and stored at 4°C until further use.

Based on the above analysis, the following enzyme system was designed using enzymes purified from Talaromyces emersonii for the degradation of paper waste and paper products, and other waste containing cellooligosaccharides.

**Table 9: System for conversion of cellulose, cellulose-rich wastes and cellooligosaccharides**

| **Enzyme Composition** | **Enzyme activity Profile (%)** |
|---|---|
| Cellobiohydrolase1A and 1B | 5.0-80.0 |
| Cellobiohydrolase II | 6.0-45.0 |
| Endoglucanase (Cel 45, EGV, EGVI, EGVII) | 4.6-66.0 |
| β-xylosidase | 0.1-2.5 |
| Xylanase | 1.0-89.0 |
| Other hydrolases (including pectin modifying enzymes, arabinofuranosidase, β-galactosidase) | 1.2-20.5 |

### Example 6: System from thermophilic fungal species, Chaetomium thermophile and Thermoascus aurantiacus.

The strategy outlined for the design of enzyme systems/thermozyme compositions from T. emersonii IMI 393751 and previously known mutants was adapted for the production of carbohydrate-modifying compositions by over twenty-three mesophilic and thermophilic fungal species. Particular attention was given to the production/induction of potent hemicellulase (xylanase, mannanase) and pectinase-rich enzyme systems by these fungal species.

Chaetomium thermophile and Themoascus aurantiacus were individually cultivated in liquid fermentation, as described earlier, on the T. emersonii nutrient medium containing 1-6% inducing carbon source (enzyme production by solid fermentation was also investigated). A potent mannan-degrading enzyme system was obtained by cultivation of C. thermophile for 96-120 h on coffee waste. This composition of this system was characterised and shown to contain 45-60% mannan-hydolysing activities, 0.7-4.0% pectin-modifying enzymes, 35.2-52.0% xylan-modifying activities, with the remainder being attributed to cellulase activities (very low or trace CBH and β-glucosidase were noted).

Cultivation of the same fungus on soyabran yielded a potent xylanolytic enzyme system(70.2-86.5% of the total activities being attributed to xylan-modifying enzymes); ∼10.6-28.0% and ∼8.6-22.5% of the remaining carbohydrate-modifying activities were attributed to cellulase, pectinolytic and low levels of mannanolytic enzymes.

Similarly, a potent pectin-modifying enzyme system was induced during cultivation of Th. aurantiacus on wheat bran and beet pulp (1:1), with >56.5-80.0% of the total carbohydrate-modifying activity profile being represented by pectinolytic activities; this enzyme system also contained ∼22.1-40.1% xylan-modifying enzymes, with the remainder being mainly cellulase/β-glucan-modifying activities. In contrast, cultivation of Th. aurantiacus on soyabran induced a potent xylanolytic enzyme system (>62.1-85.8%), complemented by ∼3.5-11.0% pectin-modifying enzymes with the remaining activities being predominately β-glucan-modifying. In contrast to C. thermophile, Th. aurantiacus did not elaborate significant levels of mannan-degrading enzymes during cultivation on either substrate.

These systems, on their own and in combination with each other or other enzyme systems (e.g. T. emersonii ) have been shown to effect extensive saccharification (sugar release) of a wide range of different agricultural, food/vegetable, beverage, woody/paper and other carbohydrate-rich virgin and waste materials, and can be used for the generation of specialised oligosaccharide products or sugar-rich feedstocks for a wide range of biotechnological applications (e.g. biofuel production).

### Example 7: System from T. emersonii for the generation of sugar-rich feedstocks from food/beverage, paper and woody wastes to be used in biofuel production.

### (a) Bioconversion of a food/beverage waste: Apple pulp/pomace

Waste apples (pulped), apple pulp and pomace were obtained from local fruit suppliers, food processing and cider/beverage production outlets. T. emersonii was cultivated on 2-6% apple pomace/pulp (both by solid and liquid fermentation) and high levels of a range of carbohydrate-modifying enzymes were measured. This system was characterised by high levels of β-glucan hydrolases, mainly non-cellulosic β-glucanase (∼27.4% in 120 h liquid culture filtrates), substantial amounts of key exo-glycosidases with especially high levels of α-arabinofuranosidase (13.3% of the total carbohydrase activity) and β-galactosidase (22.6%). Additional esterase, pectin and xylan-modifying enzymes were also detected (>7.2-33.5%). Thus using the above analysis, it is possible to design an enzyme system suitable for degrading apple waste.

The initial studies used an enzyme loading which contained 2,344 nkat xylanase, 5,472 nkat mixed linked β-glucanase and 8,529 nkat lichenanase per 3.6 Kg substrate and a reaction temperature of 70°C was used. Complete pasteurisation of the hydrolysate was achieved at 70°C, and the hydrolysate was used to feed mesophilic and thermophilic upflow anaerobic reactors (UAHR). 100% utilization of the sugar feedstock has been observed, with concomitant production of methane (50-70% in the biogas stream). Subsequent optimization studies were conducted, which demonstrated that incubation at 80°C, with gentle agitation (~120 rpm) for a 24 h period with approx. 2/3 of the original enzyme dosage, achieved ∼87% saccharification of the carbohydrates present to simple, fermentable sugars.

The sugars produced by this enzyme system can be used as a monosaccharide-rich feedstock for biofuel production.

### (b) Bioconversion of paper waste: paper cups and paper products

T. emersonii was cultivated without supplementation on a variety of paper wastes in liquid fermentation (see Example 5). Paper cups proved to be a very efficient carbohydrase inducer, yielding a potent multi-component enzyme cocktail with high levels of xylanase and starch-degrading enzyme activities, and levels of cellulase activities higher than reported on conventional growth substrates. The potential of this enzyme system to efficiently release reducing sugars and effect degradation of paper wastes was clearly illustrated by biochemical tests and Scanning Electron Microscopy. The effectof thermozyme treatment on substrate integrity and morphology using scanning electron microscopy confirm the potential of these cocktails as potent biotechnological tools for paper waste conversion. SEM provided clear evidence for extensive cellulose fibre degradation (complete loss of fibre structure in certain samples) following treatment of the cellulose-rich substrate with the *T. emersonii* cocktails.

The enzyme cocktail produced by T. emersonii after 108 h growth on paper cups contains a battery of cellulose, hemicellulose and starch degrading enzymes and saccharification studies conducted with this multi-component cocktail demonstrates its ability to effectively release glucose and other reducing sugars from conventional cellulose and paper waste substrates. This enzyme cocktail was found to be active on all paper waste and conventional cellulose substrates analysed. While all substrates were increasingly degraded over time different biodegradation susceptibilities were exhibited in response to the different substrate compositions.

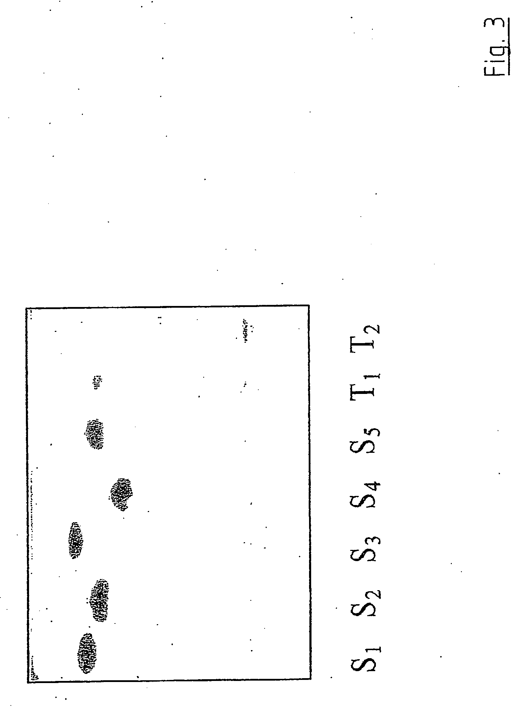

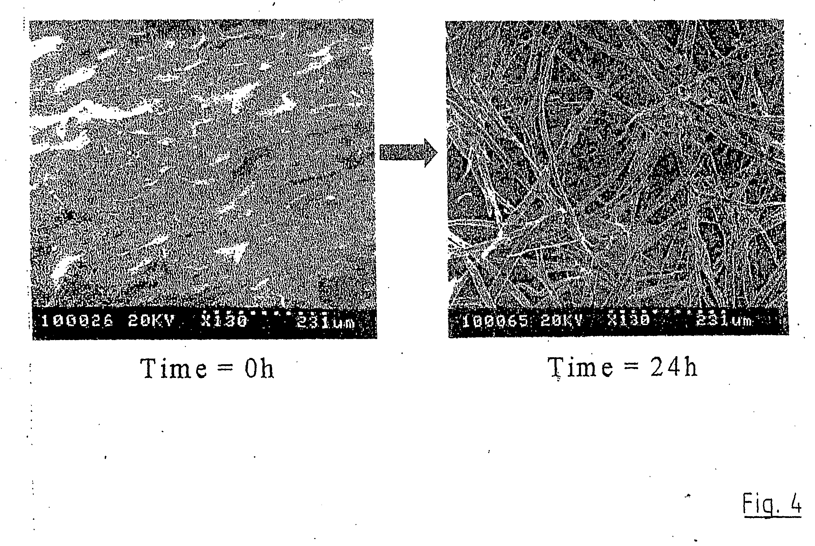

Prior to any pre-treatment biodegradable packing showed the strongest susceptibility towards enzymatic hydrolysis followed by tissue paper, paper cups and corrugated cardboard. The paper cup-induced enzyme system, functions optimally, releasing maximum sugar levels from paper waste, at a temperature of 50°C, pH 4.5, at an enzyme dosage of 4 mL/g substrate and while shaking at 37 rpm. Homogenisation of the paper cups increased the level of hydrolysis by 2.3-fold. Under these experimental conditions (enzyme dosage of 36 FPU) (filter paper units) a total %hydrolysis of 85% was achieved, with glucose accounting for ∼80% of the reducing sugars released. Glucose and xylose were the main products released (see Fig. 3). However, decreasing the enzyme dosage to 9 FPU effected an overall hydrolysis of ∼76%. Electron microscopy demonstrated the excellent hydrolytic properties of this cocktail (Fig. 4).

Heat treatment increased cardboard conversion by the same enzyme system, by a factor of 34% ( an overall carbohydrate hydrolysis based on reducing sugars released of ∼88%), while the combination of both heat treatment and homogenisation increased the reducing sugars released by 80% yielding 1.47 mg/ml glucose (31.7% of the total sugars released). Paper plates were rapidly degraded by the paper cup-induced enzyme cocktail with glucose accounting for ∼67% of the total sugars released.

Enzymatic saccharification of paper and food wastes have been investigated in Sequential Hydrolysis and Saccharification (SHF), i.e. enzyme pre-treatment followed by yeast fermentation to produce ethanol, and in Simultaneous Hydrolysis and Saccharification (SSF), where feedstock is continuously generated and immediately fermented by yeast. The enzymatic pre-treatment reaction temperatures are different in both processes, i.e. higher in SHF as the hydrolysate is cooled prior to fermentation, and at a temperature close to ambient temperatures for yeast growth and fermentation in. SSF. While the T. emersonii IMI393751enzymes are more efficient and higher reaction rates, and pasteurization are achieved (and less enzyme is required), the T. emersonii enzymes still work quite well at 25-37°C and compare well with commercial enzyme preparations from other fungal sources. The sugar-rich feedstocks produced were found to be suitable for biofuel (bioethanol and biogas) production.

### (c) Bioconversion of Softwood residues for Bioethanol production

Woody residues and wastes from primary and secondary sources (e.g. bark, thinings, and processing wastes, such as shavings and sawdust) represent a vast resource with as much as 65-70% of the dry weight comprising complex carbohydrates such as hemicellulose (∼19-28% and mainly xylans and mannans with some other polysaccharides) and cellulose (∼39-46%), which are encased in lignin.

T. emersonii IMI 393751 was grown in liquid or solid state fermentation, on woody residues, such as sitka spruce sawdust and ash shavings to generate enzyme systems with the appropriate profile of enzymes for conversion of the target waste. Different reaction/pre-treatment temperatures and enzyme dosages were investigated. The enzyme systems evaluated included cocktails obtained during growth of T. emersonii on a variety of substrates. Reaction temperatures of 50°C, 60°C, 70°C and 80°C were investigated, and a number of different substrates were used, i.e. untreated and pre-treated woody residues. Enzyme loading was also investigated, with initial studies starting with a 60 FPU, later increased up to 200 FPU (FPU: filter paper units, a measure of total cellulase activity)..

**Table 10: Saccharification of woody residues by T. emersonii (WB/BP (1:1) cocktail**

| **Dosage** | **Temp** | **Sitka spruce sawdust** | | | **Roughly milled Sitka spruce sawdust** | | |
|---|---|---|---|---|---|---|---|
| | | **% Hyd** | **%Conv** | **Hexose (g)** | **% Hyd** | **%Conv** | **Hexose (g)** |
| 60 FPU | 50°C | 34.3 | 40.9 | 0.39 | 35.2 | 27.0 | 0.24 |
| 60 FPU | 80°C | 44.3 | 50.6 | 0.49 | 48.1 | 39.5 | 0.38 |
| 60 FPU of blend* | 50°C | 48.7 | 62.3 | 2.95 | 41.8 | 35.1 | 1.87 |
| 60 FPU of blend* | 80°C | 62.4 | 79.1 | 3.45 | 55.9 | 65.2 | 3.1 |

| | | | | | | | |
|---|---|---|---|---|---|---|---|
| Hexose content is given as g released from a 10 g starting batch in a 24 hreaction period *Blend = T. emersonii (WB/BP (1:1) cocktail + C. thermophile coffee waste induced Hydrolysis under unbuffered and buffered conditions was investigated, as was the effect of reaction moisture levels and enzyme dosage. | | | | | | | |

Effects of enzyme dosage on theoretical ethanol yields are presented in Table 11.

**Table 11: Effect of enzyme dosage on %Conversion and Theoretical ethanol yield**

| **Enzyme** | **Dosage (FPU)** | **Reaction temperature** | **Hexose released (g)** | **%Conversion** | **Theoretical Ethanol yield (L/ton raw material)** |
|---|---|---|---|---|---|
| Blend* | 60 | 70°C | 4.5 | 70.0 | 325.5 |
| Blend* | 200 | 70°C | 5.1 | 76.4 | 345.4 |

| | | | | | |
|---|---|---|---|---|---|
| *Blend = T. emersonii (WB/BP (1:1) cocktail + C. thermophile coffee waste induced; Hexose content is given as g released from a 10 g starting batch in a 24 h reaction period | | | | | |

### Example 8 : Saccharification of woody biomass

**Preparation of the test substrate** Spruce chips (2-10 mm in diameter) were impregnated with sulphur dioxide (3% w/v moisture) for 20 min at room temperature to an absorption rate of 2.5% w/w moisture. The SO₂ treated spruce was treated with steam at 215°C for 2-5 min. The hemicellulose content was almost completely hydrolysed; solid recovery was 60-65% of the starting raw material. **Enzymes** MGBG Thermozymes: Numbered MGBG 1, MGBG 2, MGBG 3 and MGBG 4. Commercial enzymes used were Celluclast 2L from T. reesei and Novozym 188 from A. niger (Novo Industri A/S, Bagsvaerd, Denmark).

### Evaluation of Enzymatic Hydrolysis

Standard enzymatic hydrolysis was carried out at 37°C, 50°C and 60°C in 300 mL, 1 L and 10 L reaction vessels with agitation at ∼130 rpm. The enzyme dosage was 32 FPU of each enzyme prepper g of cellulose in a buffered substrate solution ( Gilleran, 2004). The pH of the reaction buffer was adjusted to pH 5.0 for the MGBG enzymes and pH 4.8 for the commercial preparations. Samples were removed at timed intervals and enzymatic action was terminated by boiling each reaction mixture (and controls) for 10 min. At the lowest reaction temps (37-50°C), 2 of the enzyme preparations of the invention perform as well as the commercial Celluclast preparation, and (ii) the performance of 3 of the MGBG enzymes is in the same range as the commercial Celluclast (and Cellulclast/Novozym blend).

The glucose yield using the composition of the invention was similar to the optimized commercial preparations but they yield higher levels of additional fermentable sugars than the commercial enzymes.

The enzyme preparations of the invention out-performed the commercial enzymes/ enzyme blends at the higher reaction conditions (60-70°C), in terms of overall extent hydrolysis, product yield and enzyme stability. A lower enzyme dosage could be used at the higher reaction temperatures to attain similar hydrolysis performance (depending on the enzyme preparation, only 62.5-78% of the commercial enzyme loading required). They are also less affected by inhibitory substances present in the steam-pre-treated substrate and higher concentrations of glucose and cellobiose in the sugar-rich hydrolysates. They also yield a greater amount of sugar in 24 h at 60°C, than the commercial enzymes achieve in 72 h at 50°C, the optimum working temperature for the commercial enzymes.

The key results for the enzymatic hydrolysis are presented in Table 12, while the best reaction temperature/reaction time combinations for optimum %hydrolysis, for each of the commercial and enzyme compositions of the invention tested are given in Table 13

**Table 12: Summary of Results comparing the enzymatic performance/hydrolytic potential of the Commercial and MGBG enzyme preparations on pre-treated spruce**

| **Reaction temp. (°C)** | **Reaction time (h)** | **End-points / Products (g/L)** | **Cellulclast** | **Cell + Novozym (24:4)** | **MGBG 1** | **MGBG 2** | **MGBG 3** | **MGBG 4** |
|---|---|---|---|---|---|---|---|---|
| 50°C | 24 h | Hexose | 7.33 | 13.85 | 6.8 | 11.9 | 3.84 | 8.64 |
| | | Pentose | 0.83 | 0 | 1.1 | 1.93 | 0.59 | 1.33 |
| | | Cellobiose | 2.58 | 0 | 1.22 | 2.14 | 1.28 | 2.88 |
| | | %Hydrolysis* | 40.2 | 51.9 | 34.1 | 59.8 | 21.4 | 48.1 |
| | | | | | | | | |
| | 48 h | Hexose | 14.1 | 20.4 | 14.45 | 15.75 | 7.7 | 15.55 |
| | | Pentose | 1.59 | 1.1 | 2.34 | 2.55 | 1.18 | 2.52 |
| | | Cellobiose | 4.96 | 0.6 | 1.01 | 0.3 | 2.57 | 0.35 |
| | | %Hydrolysis | 77.3 | 82.7 | 66.6 | 69.6 | 42.9 | 69.0 |
| | | | | | | | | |
| | 72 h | Hexose | 12.98 | 20.8 | 21.25 | 21.48 | 9.16 | 20.84 |
| | | Pentose | 2.26 | 2.34 | 3.4 | 3.47 | 1.40 | 3.88 |
| | | Cellobiose | 1.33 | 0.3 | 0.93 | 0.12 | 3.05 | 0.16 |
| | | %Hydrolysis | 62.0 | 87.8 | 95.8 | 93.9 | 51.0 | 91.3 |
| | | | | | | | | |
| 60°C | 24 h | Hexose | 4.18 | 3.74 | 20.55 | 22.12 | 4.65 | 10.46 |
| | | Pentose | 0.47 | 0.31 | 2.1 | 1.87 | 0.71 | 1.49 |
| | | Cellobiose | 1.47 | 0.17 | 3.69 | 1.34 | 1.55 | 3.61 |
| | | %Hydrolysis | 22.9 | 15.8 | 98.6 | 94.8 | 25.9 | 58.3 |
| | | | | | | | | |
| | 48 h | Hexose | 8.04 | 11.86 | 22.71 | 23.02 | 9.32 | 17.38 |
| | | Pentose | 0.9 | 0.63 | 2.47 | 2.55 | 1.43 | 2.67 |
| | | Cellobiose | 2.83 | 0.34 | 1.31 | 0.39 | 3.11 | 5.80 |
| | | %Hydrolysis | 44.1 | 48.0 | ∼100.0 | 97.2 | 51.9 | 96.8 |
| | | | | | | | | |
| | 72 h | Hexose | 12.98 | 14.80 | 23.51 | 23.33 | 12.56 | 21.9 |
| | | Pentose | 2.26 | 1.33 | 2.53 | 2.67 | 1.92 | 2.43 |
| | | Cellobiose | 1.33 | 0.17 | 0.85 | 0.17 | 5.7 | 1.34 |
| | | %Hydrolysis | 62.0 | 61.3 | ∼100.0 | 98.0 | 75.6 | 96.1 |
| | | | | | | | | |
| 70°C | 24 h** | Hexose | 1.2 | 1.07 | 21.72 | 24.11 | 16.8 | 20.98 |
| | | Pentose | 0.14 | 0.09 | 2.39 | 2.07 | 2.58 | 2.73 |
| | | Cellobiose | 0.42 | 0.05 | 2.43 | 0.55 | 5.60 | 2.44 |
| | | %Hydrolysis | 6.6 | 4.5 | 99.4 | ∼100.0 | 93.5 | 97.9 |

| | | | | | | | | |
|---|---|---|---|---|---|---|---|---|
| *%Hydrolysis: total sugars released expressed as a %of the total available sugars in the substrate; **Later time-points were no shown as complete hydrolysis was attained by the MGBG enzymes | | | | | | | | |

**Table 13: Reaction temperature/reaction time combinations for maximum %hydrolysis, for each of the commercial and MGBG enzymes investigated**

| **Enzyme Prep** | **Optimum Reaction temp (°C)** | **Optimum Reaction time (h)** | **%Hydrolysis** | **g/L Hexose** |
|---|---|---|---|---|
| Celluclast | 50.0 | 48 h | 77.3 | 14.1 |
| | | | | |
| Cell + Novozym (24:4) | 50.0 | 48 h | 82.7 | 20.4 |
| | 50.0 | 72 h | 87.8 | 20.8 |
| | | | | |
| MGBG 1 | 50.0 | 48 h | 66.6 | 14.45 |
| | 50.0 | 72 h | 95.8 | 21.25 |
| | 60.0 | 24 h | 98.6 | 20.55 |
| | 70.0 | 24 h | 99.4 | 21.72 |
| | | | | |
| MGBG 2 | 60.0 | 24 h | 94.8 | 22.12 |
| | 70.0 | 24 h | ∼100.0 | 24.11 |
| | | | | |
| MGBG 3 | 70.0 | 24 h | 93.5 | 16.8 |
| | | | | |
| MGBG 4 | 70.0 | 24 h | 97.9 | 20.98 |
| | 70.0 | 48 h | 96.8 | 17.38 |

### Simultaneous Saccharification and Fermentation (SSF)

Batch SSF experiments with spruce hydrolysate were carried out to compare the performance of different enzyme preparations. Fermentation was carried out at a concentration of spruce fibres of 4%, the pre-treated material was diluted with sterile water to the desired concentration. The pH was maintained at 5.0 with the addition of 2M NaOH. The fermentation temperature was 37°C and the stirrer speed was 500 rpm. The reactor medium was sparged with nitrogen (600 ml/min) and the CO₂ content was measured with a gas analyser. The enzyme preparation was added directly to the fermentor at a loading of 25 filter paper units (FPU)/g cellulose. The fermentation medium was supplemented with nutrients; 0.5 g/l (NH₄)₂HPO₄, 0.025 g/l MgSO₄.7H₂O and 1.0 g/l of yeast extract. The concentration of yeast (baker's yeast, Saccharomyces cerevisiae) cell mass added was 5 g/l arid all SSF experiments were carried out at 37°C for 72 hours. Samples were withdrawn at various time intervals, were centrifuged in 1.5 ml microcentrifuge tubes at 14,000 g for 5 minutes (Z 160 M; Hemle Labortechnik, Germany), the supernatant was then prepared for HPLC analysis.

Products of hydrolysis generated during the degradation of lignocellulosic materials were analysed by HPLC. Cellobiose, glucose, xylose, galactose, mannose, HMF and furfural were separated on a polymer column (Aminex HPX-87P) at 85°C, the mobile phase was millipore water at a flow rate of 0.5 ml/min. Concentrations of ethanol, glycerol and acetate were determined using an Aminex HPX-87H column at 60°C, using a Shimadzu HPLC system equipped with a refractive index detector. The mobile phase was a 5 mM aqueous solution of H₂SO₄ at a flow rate of 0.5 ml/min. Ethanol produced was also determined using an enzyme-linked assay (r-Biopharm, Germany).

Yeast growth takes place in two phases. Carbon dioxide is an important by-product of the ethanol fermentation process as anaerobic fermentation of one mole of glucose yields one mole of ethanol and two moles of carbon dioxide. Therefore, measurement of the carbon dioxide concentration in the outlet gas, is an indirect measurement of the fermentation rate. In the first growth phase the available glucose is consumed and ethanol is formed, and the initial fast response to the glucose present is represented by a surge in the evolution of CO₂.

The results obtained during SSF are given in Table 3. As mentioned previously, the total time taken for SSF, for each enzyme preparation was 72 h, and the temperature used for SSF was 37°C. The fermentation efficiency was determined by dividing the actual concentration of Ethanol produced (g/L) by the total theoretical ethanol (g/L) that would be produced if all of the available substrate was converted to soluble, fermentable sugar and all of the sugar was converted to ethanol, and multiplying by 100.

Based on the data obtained, the ethanol yields for each enzyme/SSF combination are given in L Ethanol/dry tonne, and the corresponding US units, gallons Ethanol/dry US ton.

**Table 14: Summary of SSF experiments**

| **Enzyme Prep** | **g/L Ethanol** | **Fermentation efficiency (%)** | **Yield Ethanol (L/tonne)** | **Yield Ethanol (US gallons/ton)** | **Enzyme cost (US $/ton)** |
|---|---|---|---|---|---|
| Cell + Novo (24:4) #1 | 5.83 | 54.0 | 178.78 | 51.85 | Not available |
| | | | | | |
| Cell + Novo (24:4) #2 | 9.10 | 84.3 | 279.05 | 80.52 | Not available |
| | | | | | |
| MGBG 1* | 5.02 | 46.5 | 153.88 | 44.62 | ∼17 US $ |
| MGBG 1 | 7.33 | 67.9 | 224.62 | 65.14 | ∼21.76 US $ |
| | | | | | |
| MGBG 2* | 7.24 | 67.1 | 221.89 | 64.35 | ∼16.8 US $ |
| MGBG 2 | 7.89 | 73.1 | 241.79 | 70.12 | ∼23 US $ |
| | | | | | |
| MGBG 3** | 8.62 | 79.9 | 264.46 | 76.69 | ∼17.4 US $ |
| | | | | | |
| MGBG 4** | 9.67 | 89.6 | 296.37 | 85.95 | ∼15.4 US $ |

| | | | | | |
|---|---|---|---|---|---|
| *Enzyme loading of 21.3 FPU used instead of 32.0 FPU **More optimized blends of MGBG 3 and MGBG 4. | | | | | |

### Example 9 Comparison of the commercial and MGBG enzymes in a Sequential Hydrolysis and Fermentation (SHF) strategy for bioethanol production.

Bioethanol yields obtained by sequential hydrolysis and fermentation, were investigated for the commercial and enzyme preparations of the invention. One advantage of SSF, is that the process consists of an initial rapid fermentation and metabolism of monomeric sugars resulting from the pre-treatment step. Once glucose is released from the substrate by the action of the hydrolytic enzymes that have been added, fermentation is rapid, which means that, in SSF, the concentration of free sugars always remains low. In SSF, the fermentation rate eventually decreases as a result of either a decrease in the rate of substrate conversion by the enzymes, or inhibition of yeast metabolism, whichever is rate limiting. The data obtained in these experiments are summarized in Table 15.

**Table 15: Summary of SHF experiments**

| **Enzyme Prep** | **Hydrolysis temp & time** | **g/L Ethanol** | **Fermentation efficiency (%)** | **Yield Ethanol (L/tonne)** | **Yield Ethanol (US gallons/ton)** | **Enzyme cost (US $/ton)** |
|---|---|---|---|---|---|---|
| Celluclast | 50°C for 48 h | 7.21 | 66.8 | 220.94 | 64.8 | Not available |
| Cell + Novo (24:4)# 1 | 50°C for 48 h | 9.35 | 86.7 | 286.7 | 83.15 | Not available |
| Cell + Novo (24:4)#2 | 50°C for 48 h | 10.42 | 96.6 | 319.53 | 92.66 | Not available |
| | | | | | | |
| MGBG 1 | 60°C for 24 h | 10.50 | 93.37 | 321.98 | 93.37 | ∼21.76 US $ |
| MGBG 1 * | 70°C for 24 h | 11.10 | ∼100.0 | 340.38 | 98.71 | ∼21.76 US $ |
| MGBG 2 | 60°C for 24 h | 11.30 | ∼100.0 | 346.51 | 100.5 | ~21.8 US $ |
| MGBG 2* | 70°C for 24 h | 12.32 | >100.0** | 377.79 | 109.6 | ∼21.8 US $ |
| MGBG3^{%} | 70°C for 24 h | 8.59 | 79.6 | 263.26 | 76.3 | 21.4 US $ |
| MGBG 4 | 70°C for 24 h | 10.72 | 99.4 | 328.73 | 95.33 | ∼21.4 US $ |

| | | | | | | |
|---|---|---|---|---|---|---|
| *Enzyme preparation used at higher reaction temperature. ^{%}This enzyme cocktail is particularly effective in releasing fermentable sugars from hemicellulose-rich substrates and would not be expected to be as effective on a substrate that has a significant part of the hemicellulose fraction removed (pre-treatment step). | | | | | | |

HPLC analysis confirmed that the main products formed are monosaccharides (single sugars) with very small amounts of higher oligomers formed (cellobiose, which is a disaccharide, being the main, or only, higher chain sugar present in hydrolysates),

### Example 10 Animal feeds Applications:

Each enzyme composition was evaluated in individual target applications, with model studies conducted at laboratory scale with 5-25 g of the substrate (cereal, cereal flour or other plant residue) in 50-250 mL final reaction volumes, at pH 2.5-7.0 and 37-85°C, with or without shaking. Enzyme performance was evaluated with and without substrate pre-treatment, i.e. gentle steam pre-treatment (105°C, 8 p.s.i for 5 min), grinding using a mortar and pestle, homogenization in a Parvalux or Ultraturrax homogenizer. For soft fruit and vegetable tissues/residues, the substrate was macerated roughly by mixing, and incubated with enzyme, without pre-treatment. Substrate hydrolysis was monitored by (i) measurement of reducing sugars released and assays to detect and quantify individual sugars, (ii) confirmatory TLC and HPLC analysis of the sugar products of hydrolysis, (iii) analysis of weight/volume reduction of the residue, (iv) comparison of cellulose, hemicellulose, starch and pectin contents before and after enzymatic treatment, and (v) physical analysis of substrate degradation by scanning electron microscopy (SEM) for fibrous substrates such as paper and woody wastes.

**Table 16**

| **Cocktail** | **Cocktail composition (%)** | **Target application** | **Substrate** | **Optimum treatment temp (°C)** | **Products** |
|---|---|---|---|---|---|
| MGBG 15 | CBH I (25-30%) | Enhanced digestibility | Wheat, Oats | >60°C (upto 75°C) | Mainly glucooligosaccharides, xylooligosaccharides, and some other minor amounts of diverse sugars |
| | CBH II (1-5%) | | | | |
| | β(1,3)4-glucanase (10-15%) | | | | |
| | β-glucosidase (2-10%) | | | | |
| | Xylanase (44-48%) | | | | |
| | 0.1-1.5 %β-Xylosidase, | | | | |
| | 0.1-2.0 % α-Glucuronidase | | | | |
| | 0.1-1.5 % α-L-arabinofuranosidase; | | | | |
| | 2-5% pectinolytic enzymes, and esterases | | | | |
| | 7-15% protease | | | | |
| MGBG 16 | CBH I (10-15%) | Enhanced digestibility | Wheat; Oats; Rye | >60°C (upto 80-85°C) | Galactooligosaccharides, glucooligosaccharides, and xylooligosaccharides |
| | CBH II (10-15%) β(1,3)4-glucanase (25-30%) | | | | |
| | β-glucosidase (2-8%) | Feedstuffs enriched in | | | |
| | Xylanase (25-30%) | Galacto- and | | | |
| | 1-2.0 %β-Xylosidase, | Glucooligosaccharides | | | |
| | 1-2.0 % α-Glucuronidase | | | | |
| | 0.1-2.0 % α-L-arabinofuranosidase; | | | | |
| | 5-10% pectinolytic enzymes, ∼5% starch modifying activity; 5-10% | Antioxidant release | | | |
| | oxidoreductase/oxidase and esterases 10-15% protease | | | | |
| MGBG 17 | CBH I (5-10%) | Enhanced digestibility | Wheat; Oats; Rye; | >60°C (upto 80°C) | Glucooligosaccharides, and xylooligosaccharides |
| | CBH II (5-10%) | | Maize; Barley; | | |
| | β(1,3)4-glucanase (20-30%) | | Canola | | |
| | β-glucosidase (2-8%) | | | | |
| | Xylanase (30-35%) | | | | |
| | 1-2.0 %β-Xylosidase, | | | | |
| | 1-3.0 % α-Glucuronidase | | | | |
| | 1.5-4.0 % α-L-arabinofuranosidase; 5-10% pectinolytic enzymes, 5-8% starch modifying activity; 1-4% oxidoreductase/oxidase and esterases, 18-25% protease | | | | |
| MGBG 13 | CBH I (5-10%) | Enhanced digestibility | Wheat; Oats; Rye; | >60°C (upto 85/90°C) | Glucooligosaccharides, and xylooligosaccharides, some monosaccharides and disaccharides from pectin polymers (e.g. arabinooligosaccharides, galacturonic acid, rhamnose, etc.) |
| | CBH II (5-10%) | | Maize; Barley; | | |
| | β(1,3)4-glucanase (25-40%) | Antioxidant release | Canola | | |
| | β-glucosidase (~5%) | | | | |
| | Xylanase (18-25%) | | | | |
| | 1-2.0%β-Xylosidase, | | | | |
| | 1-3.0%; Exoxylanase, | | | | |
| | 8-10%, α-Glucuronidase | | | | |
| | 1.5-5.0 % α-L-arabinofuranosidase; | | | | |
| | 10-15% pectinolytic enzymes, | | | | |
| | 5-7% starch modifying activity; | | | | |
| | 5-10% other hemicellulases, | | | | |
| | 1-4%,oxidoreductase/oxidase and esterases | | | | |
| | 8-12% protease | | | | |
| MGBG 19 | CBH I (10-15%) | Enhanced digestibility; | Sorghum; Canola; | >60°C (upto 80/85°C) | Glucooligosaccharides, and xylooligosaccharides, some monosaccharides or some other smaller amounts of sugars from pectic polymers |
| | CBH II (10-15%) | | also other cereals, | | |
| | β(1,3)4-glucanase (25-30%) | Flexibility to use different feestuffs | e.g. wheat; Oats; | | |
| | β-glucosidase (-10-15%) | | Rye; Maize; Barley | | |
| | Xylanase (20-27%) | | | | |
| | 4.0-7.0%β-Xylosidase, | | | | |
| | 5-10%; Exoxylanase, | | | | |
| | 1-4% α-Glucuronidase | | | | |
| | 2.0-5,0 % α-L-arabinofuranosidase; , | | | | |
| | 5% pectinolytic enzymes, | | | | |
| | ∼5% starch modifying activity; | | | | |
| | ∼5% other hemicellulases, | | | | |
| | 1-4%" oxidoreductase/oxidase and esterases | | | | |
| | ∼25% protease | | | | |
| MGBG 20 | CBHI (5-10%) | Enhanced digestibility; | Wheat, barley & rye | >60oC (upto 80/85oC) | Glucooligosaccharides, and xylooligosaccharides, some monosaccharides or some other smaller amounts of sugars from pectic polymers |
| | CBH II (5-10%) | | | | |
| | β(1,3)4-glucanase (20-32%) | | | | |
| | β-glucosidase (∼2-9.5%) | | | | |
| | Xylanase (15-25%) | | | | |
| | 15-20.0%β-Xylosidase, | | | | |
| | 10-15%; Exoxylanase, | | | | |
| | 2-5%, α-Glucuronidase | | | | |
| | 1.5-5% α-L-Arabinofuranosidase; | | | | |
| | 10-15% pectinolytic enzymes, | | | | |
| | ∼10% starch modifying activity; | | | | |
| | ∼5% other hemicellulases, | | | | |
| | 2-5%" oxidoreductase/oxidase and esterases | | | | |
| | ∼7-10% protease | | | | |
| MGBG 21 | CBH I (5-10%) | Enhanced digestibility; | Wheat, barley & maize; also beet pulp | >60oC (upto 80/85oC) | Glucooligosaccharides, xylooligosaccharides, some monosaccharides or some other smaller amounts of sugars from pectic polymers and fructooligosaccharides |
| | CBH II (5-10%) | | | | |
| | β(1,3)4-glucanase (15%) | Fructooligosaccharide | | | |
| | β-glucosidase (-2-10%) | release | | | |
| | Xylanase (50-55%) | | | | |
| | 1-5%-Xylosidase; | | | | |
| | 5-10%; Exoxylanase, | | | | |
| | 1-4%, α-Glucuronidase | | | | |
| | 2-5% α-L-Arabinofuranosidase; | | | | |
| | 20-25% pectinolytic enzymes, | | | | |
| | 4-6% starch modifying activity; | | | | |
| | 7-10% other hemicellulases, | | | | |
| | 2-4%" oxidoreductase/oxidase and esterases | | | | |
| | ∼10% protease | | | | |
| MGBG 22 | CBH I (∼5%) | Enhanced digestibility; | Wheat, barley, rye | >60oC (upto 85/90oC) | Glucooligosaccharides, xylooligosaccharides, some monosaccharides or some other smaller amounts of sugars, etc. |
| | CBH II (∼5%) | | | | |
| | β(1,3)4-glucanase (27-45%) | Glucooligosaccharide formation | | | |
| | β-glucosidase (∼1-5%) | | | | |
| | Xylanase (16-25%) | | | | |
| | 0.5-4%-Xylosidase, | | | | |
| | ∼10%; Exoxylanase, | | | | |
| | 0.5-4%, α-Glucuronidase | | | | |
| | 1-5% α-L-Arabinofuranosidase; | | | | |
| | 5-10% pectinolytic enzymes, | | | | |
| | 4-8% starch modifying activity; | | | | |
| | 8-10% other hemicellulases, | | | | |
| | 1-4%, oxidoreductase/oxidase and esterases | | | | |
| | ∼5-6% protease | | | | |
| MGBG 23 | CBH I (∼3-6%) | Enhanced digestibility; | Wheat, barley, rye | >60°C (upto 85/90°C) | Low DP Glucooligosaccharides, xylooligosaccharides,wit h significant monosaccharides or some other smaller amounts of sugars, etc. |
| | CBH II (∼3-6%) | | | | |
| | β(1,3)4-glucanase (35-45%) | Glucooligosaccharide, xylooligosaccharides | | | |
| | β-glucosidase (∼3-6%) | | | | |
| | Xylanase (∼30%) | | | | |
| | 0.5-2.0%-Xylosidase, | High anti-oxidant release potential | | | |
| | ∼2-5%; Exoxylanase, | | | | |
| | 0.5-2%, α-Glucuronidase | | | | |
| | 1-5% α-L-Arabinofuranosidase; | | | | |
| | 5-10% pectinolytic enzymes, | | | | |
| | ∼2-4% starch modifying activity; | | | | |
| | ∼10% other hemicellulases, | | | | |
| | 6-10%" oxidoreductase/oxidase and esterases | | | | |
| | ∼8-10% protease | | | | |

| | | | | | |
|---|---|---|---|---|---|
| For feedstuffs enriched in key oligosaccharides, e.g. Galactooligosaccharides - MGBG 16 enzyme cocktail is best Glucooligosaccharides - MGBG 16 and 22 are best Fructooligosaccharides - MGBG 21 cocktail is best | | | | | |

For antioxidant-enriched feedstuff preparations, the best cocktails to use for treatment are those prepared on: MGBG 13, 16 and 23.

### Example 11 Monogastric animal feedstuff applications:

Studies were conducted at temperatures over the range 50-85°C (with shaking at 140 rpm), with crude cereal fractions (1-5 g lots). Hydrolysis of carbohydrate in the substrate, by each enzyme preparation (0.5 IU maximum dosage per g substrate), was monitored over 24 h by quantifying reducing sugars released, and products formed in samples removed from the reaction mixture at periodic intervals.

**Table 17**

| **Cocktail** | **Cocktail composition (%)** | **Target application** | **Substrate** | **Optimum treatment temp (°C)** | **Products** |
|---|---|---|---|---|---|
| MGBG 24 | CBH I (1-5%) | Enhanced digestibility. | Barley, Millet, Wheat, Oats. | >60°C (up to 85°C) | Mainly glucooligosaccharides, xylooligosaccharides, and some other minor amounts of diverse sugars. Some of the oligosaccharide (and peptide) products would have health-boosting properties. |
| | CBH II (1-5%) | Production of low-pentose, easily digested cereal-based feedstuffs for poultry and pigs, in which the water-binding properties of viscous β-1,3;1,4-glucans has been reduced by enzymatic fragmentation. | | | |
| | β(1,3)4-glucanase (27-43%) | | | | |
| | β-glucosidase (2-10%) | | Barley-soybean diets. Maize-wheat-soybean diets. Wheat-rye-soybean diets. | | |
| | Xylanase (44-48%) | | | | |
| | 0.1-0.4% β-Xylosidase, | | | | |
| | 0.1-3.0 % α-Glucuronidase | | | | |
| | 0.1-0.4% α-L-Arabinofuranosidase; | | | | |
| | 5-10% pectinolytic enzymes, and esterases | | | | |
| | 20-25% protease | | | | |
| MGBG 17 | As before | | | | |
| MGBG 25 | CBH I (0.5-2.5%) | Enhanced digestibility. | Barley, Oats, Rye | >60°C (up to 85°C) | Mainly glucooligosaccharides, xylooligosaccharides (DP 2-6) |
| | CBH II (0.5-2.5%) | Production of low- pentose, easily digested cereal-based feedstuffs for poultry and pigs. | | | |
| | β(1,3)4-glucanase (15-20%) | | | | |
| | β-glucosidase (4-10%) | | | | |
| | Xylanase (70-88%) | | | | |
| | 0.1-0.4% β-Xylosidase, | >30% hydrolysis of the non-cellulosic β-glucan present in Rye, which is accompanied by a marked decrease in viscosity | | | |
| | 0.1-2.0 % α-Glucuronidase | | | | |
| | 0..1-1.0 % α-L-Arabinofuranpsidase; | | | | |
| | 1-6% pectinolytic enzymes, and esterases | | | | |
| | 8-10% protease | | | | |

Significant hydrolysis of the xylan and non-cellulosic ß-glucan fraction was observed, which resulted in the formation of medium to longer chain oligosactharide products of hydrolysis, which would also be suitable fermentation substrates for probiotic microorganisms.

### Example Biogas production

Two 10 L upflow anaerobic hybrid reactor (UAHR; Reynolds, 1986), one maintained at 37°C and the other at 55°C were used for biogas production by individual mixed populations of mesophilic and thermophilic bacteria, respectively. The sugar-rich hydrolysate was pumped up through the sludge bed and degraded by the communities of microorganisms present. A separating device at the top of the reactor was used to separate the gas produced from any sludge particles that might have become dislodged during anaerobic digestion. Both reactors were evaluated continuously throughout at 650 day operation period by monitoring the efficiency of COD removal (APHA, 1992), total carbohydrate reduction (Dubois method) and methane production. Fatty acids production, an indicator of sugar metabolism, and methane production were monitored by gas chromatography (GC).

Biofuel can be produced from a number of feedstocks. Many of these require the use of different enzyme cocktails. MGBG 16 is the best cocktail for production of feedstocks from the food and vegetable wastes listed below for biogas production by Anaerobic digestion.

**Table 18**

| **Treatment Cocktail** | **Waste residue used** | **Main sugars released** | **Mesophilic Anaerobic Digestion % Carb Reduction** | **Thermophilic Anaerobic Digestion % Carb Reduction** | **Mesophilic Anaerobic Digestion % methane in biogas** | **Thermophilic Anaerobic Digestion % methane in biogas** |
|---|---|---|---|---|---|---|
| MGBG 16 | Carob | Xylose, glucose mannose, cellobiose | 99-100 | 94.0-96.6 | 58.0 | 49.0 |
| | Bread | Xylose, xylobiose, Glucose, cellobiose | 96.1-99.7 | 96.0-99.3 | 71.9 | 69.0 |
| | Apple Pomace | Glucose, | 96.0-99.3 | 95.4-99.5 | 58.0 | 52.0 |
| | | Galacturonic acid, | | | | |
| | | Fructose, | | | | |
| | | Arabinose, | | | | |
| | | Galactose, | | | | |
| | | Xylose | | | | |
| | Cardboard | Xylose, | 95.4-96.9 | 94.0-96.6 | 63.0 | 49.0 |
| | | Glucose (trace higher oligos) | | | | |
| MGBG 18 | Mixed veg/fruit waste (restaurant) | Xylose | | | | |
| | | Glucose, | | | | |
| | | Galacturonic acid, | | | | |
| | | Fructose; | | | | |
| | | Arabinose, | | | | |
| | | Galactose, | | | | |
| | | Rhamnose | | | | |

| | | | | | | |
|---|---|---|---|---|---|---|
| (*%Methane in biogas is normally 50-65% max.); | | | | | | |

The data in Table 18 were achieved at retention times of only 3 days, in contrast with reports in the literature, in which retention times are normally between 9-30 days. Retention times indicates the period taken (or lag phase) for metabolism of the carbohydrate feedstock and production of biogas.

### Example 12 Bioethanol production

Standard enzymatic hydrolysis was carried out at 37°C, 50°C and 60°C in 300 mL and 1 L vessels with agitation at ∼130 rpm. Enzyme dosage was 32 FPU of each enzyme preparation per gram of cellulose in a buffered substrate solution (as described by Gilleran, (NUI, Galway, Ph.D. Thesis, 2004)total weight processed = 100 g in laboratory-scale studies). The pH of the reaction buffer was adjusted to pH 5.0. Samples were removed at timed intervals and enzymatic action was terminated by boiling for 10 min.Over a 0-72 h period the following were measured:
• Release of reducing sugars and detection and quantification of individual sugars (expressed as g/L)
• HPLC analysis of the sugar products of hydrolysis (product quantities expressed in g/L)
• Weight/volume reduction of the residue
• Cellulose fibre content before and after enzymatic treatment

Scanning electron microscopy (SEM) of substrate integrity before and after enzyme treatment Production of key fatty acids during the metabolism of sugars by the anaerobic bacteria, and the production of methane, were monitored by gas chromatography (GC). Bioethanol produced by yeast fermentation was measured using two approaches, an enzyme-linked assay kit for quantification of ethanol (r-Biopharm, Germany) and also by high performance liquid chromatography (HPLC).

Sitka spruce hydrolysate, paper waste and woody residues (mixture of coniferous residues, mainly sitka spruce) were tested in laboratory-scale studies. Two ethanol production formats were investigated, SHF (**S**equential **H**ydrolysis and **F**ermentation) and SSF (Simultaneous Saccharification and Fermentation).

**Table 19: Enzyme cocktail composition for Bioethanol production**

| **Enzyme** | **MGBG 1^{a} %** | **MGBG 2^{b} %** | **MGBG 3^{c} %** | **MGBG 4^{d} %** |
|---|---|---|---|---|
| CBH I | 20-28 | 15-20 | 15-17 | 12-15 |
| CBH II | 15-20 | 20-28 | 22-26 | 24-30 |
| β-(1,3)4-glucanase | 20-25 | 20-26 | 25 | 20-22 |
| β-glucosidase | 10-12 | 10-11 | 11-15 | ∼10.0 |
| Xylanase | 20-25 | 18-30 | 24-27 | 20-30 |
| β-Xylosidase, | 5-10 | 8-10 | 10-12 | 5-8 |
| α-Glucuronidase | 5-8 | 8-10 | 6-8 | 8-10 |
| α-L-Arabinofurano- sidase (Other hydrolases, including Pectinolytic enzymes, Phenolic acid and acetyl(xylan) esterases, Protease; Lignin-modifying oxidase activities) | 0.5-2.0 | 0.5-2.0 | 2-4.0 | 1.5-3.0 |
| | 8-15 | 6-17 | 12-15 | 10-15 |

| | | | | |
|---|---|---|---|---|
| Data from 1 L laboratory-scale studies in SHF and SSF; Hydrolysis values are based on the available cellulose and any residual hemicellulose in substrate | | | | |

**Table 20: Comparison of Bioethanol production from spruce hydrolysate generated by compositions of the invention and Commercial enzymes**

| **Enzyme Prep** | **Optimum Reaction time (h)** | **%Hydrolysis*** | **g/L Hexose** | **g/L Ethanol SSF (37°C)** | **Fermentation efficiency SSF (37°C)** | **Optimum Reaction temp (°C) for SHF** | **g/L Ethanol SHF** | **Fermentation efficiency SHF** |
|---|---|---|---|---|---|---|---|---|
| Celluclast | 48 h | 74.5 | 13.59 | 4.69 | 43.44 | 50.0 | 6.93 | 64.21 |
| | | | | | | | | |
| Cell + Novozym (24:4) | 48 h | 78.9 | 19.46 | nd | nd | 50.0 | 8.97 | 83.18 |
| | 72 h | 82.3 | 19.50 | 5.70 | 52.80 | 50.0 | nd | nd |
| | | | | | | | | |
| MGBG 1 | 48 h | 57.9 | 12.56 | 4.83 | 44.74 | 50.0 | nd | nd |
| | 72 h | 87.2 | 19.34 | nd | nd | 50.0 | nd | nd |
| | 24 h | 88.1 | 19.14 | nd | nd | 60.0 | 10.29 | 91.50 |
| | 24 h | 90.4 | 19.75 | 6.89 | 63.82 | 70.0 | 10.51 | 93.46 |
| | | | | | | | | |
| MGBG 2 | 24 h | 89.3 | 20.84 | 7.04 | 65.25 | 60.0 | 10.39 | 92.39 |
| | 24 h | 91.2 | 22.32 | 8.01 | 74.21 | 70.0 | 10.53 | 93.64 |
| | | | | | | | | |
| MGBG 3 | 24 h | 87.3 | 15.69 | 8.58 | 79.53 | 70.0 | 8.71 | 80.71 |
| | | | | | | | | |
| MGBG 4 | 24 h | 92.2 | 19.76 | 9.55 | 88.49 | 70.0 | 10.65 | 98.75 |
| | 48 h | 89.9 | 16.14 | nd | nd | 70.0 | nd | nd |

**Table 21: Bioethanol production from Paper waste and Softwood residues generated by the T. emersonii thermostable enzyme cocktails in comparison" Commercial enzymes (data from 1 L laboratory-scale studies in SHF and SSF; Hydrolysis values are based on the available cellulose and any residual hemicellulose in substrate)**

| **Enzyme Prep** | **Optimum Reaction temp (°C)** | **Optimum Reaction time (h)** | **%Hydrolysis*** | **g/L Hexose** | **g/L Ethanol SHF** | **Fermentation efficiency SHF** | **g/L Ethanol SSF (37°C)** | **Fermentation efficiency SSF (37°C)** |
|---|---|---|---|---|---|---|---|---|
| Paper Waste | | | | | | | | |
| MGBG 1 | 70.0 | 24 h | 76.5 | 13.59 | 8.36 | 74.3 | 7.18 | 66.5 |
| MGBG 3 | 70.0 | 24 h | 79.2 | 17.57 | 7.89 | 70.2 | 8.08 | 74.6 |
| MGBG 4 | 70.0 | 24 h | 83.4 | 19.46 | 7.54 | 69.9 | 8.22 | 76.2 |
| | | | | | | | | |

| Softwood residues | | | | | | | | |
|---|---|---|---|---|---|---|---|---|
| MGBG 1 | 70.0 | 24 h | 63.8 | 11.21 | 7.57 | 67.3 | 7.03 | 65.1 |
| MGBG 3 | 70.0 | 24 h | 74.9 | 13.09 | 7.92 | 70.4 | 7.53 | 69.8 |
| MGBG 4 | 70.0 | 24 h | 77.9 | 13.57 | 7.74 | 71.8 | 7.82 | 72.5 |

**Table 22: Other cocktails for production of fermentable sugar-rich hydrolysates from Vegetable/Fruit waste streams**

| **Cocktail** | **Cocktail composition (%)** | **Target application** | **Substrate** | **Optimum treatment temp (°C)** | **Products sugar** |
|---|---|---|---|---|---|
| MGBG 18 | CBH I (5-10%) | Production of fermentable sugars for manufacture of biofuel and/or other high value products, e.g. (bio)chemicals/chemical feedstocks, carotenoids, antibiotics, probiotics, etc. | Mixed veg/fruit waste (restaurant) | >60°C (upto 80°C) | Xylose |
| | CBH II (5-10%) | | | | Glucose, |
| | β(1,3)4-glucanase (25-40%) | | | | Galacturonic |
| | β-glucosidase (∼5%) | | | | acid, |
| | Xylanase (18-25%) | | | | Fructose, |
| | + [1-2.0%β-Xylosidase, 1-3.0%; Exoxylanase, 8-10%, α-Glucuronidase 1.5-5.0 % α-L-Arabinofuranosidase; 10-15% pectinolytic enzymes, including β-galactosidase, rhamnogalacturonase, polygalacturonase, exogalacturonase and galactanase, 5-7% starch modifying activity; 5-10% other hemicellulases, including β-galactosidase, 1-4%,oxidoreductase/ oxidase and esterases + 8-12% protease] | | | | Arabinose, |
| | | | | | Galactose, |
| | | | | | Rhamnose |
| MGBG 32 | CBH I (1-5%) | Production of fermentable sugars for manufacture of biofuel and/or other high value products, e.g. (bio)chemicals/chemical feedstocks, carotenoids, antibiotics, probiotics, | Mixed veg/fruit waste (restaurant); | >60°C (upto 80°C) | Xylose |
| | CBH II (1-5%) | | | | Glucose, |
| | β(1,3)4-glucanase (20-25%) | | | | Galacturonic |
| | β-glucosidase (8-12%) | | Mixed soft fruit also | | acid, |
| | Xylanase (30-35%) | | | | Fructose, |
| | + [0.5-1.5 %β-Xylosidase, 1-2.5 % α-L-Arabinofuranosidase; 18-25% pectinolytic enzymes, including β-galactosidase, rhamnogalacturonase, polygalacturonase, exogalacturonase and galactanase, ∼5-10% starch modifying activity; 8-15% oxidoreductase/oxidase and esterases + 10-15% protease] | | | | Arabinose, |
| | | | | | Galactose, |
| | | aroma and flavour precursors, etc. | | | Rhamnose |
| | | Can be used also for enhanced juice extraction and production of 'health beverages' | | | |
| MGBG 6 (4% CarrotBP; 1:1) | CBH I (1-5%) | Production of fermentable sugars for manufacture of biofuel and/or other high value products, e.g. (bio)chemicals/chemical feedstocks, carotenoids, antibiotics, probiotics, aroma and flavour precursors, etc. Can be used also for enhanced juice extraction and production of 'health beverages' | Mixed veg/fruit waste (restaurant) | >60°C (upto 85°C) | Xylose |
| | CBH II (1-5%) | | | | Glucose, |
| | β(1,3)4-glucanase (22-28%) | | | | Galacturonic |
| | β-glucosidase (10-15%) | | Mixed soft fruit | | acid, |
| | Xylanase (25-30%) | | also | | Fructose, |
| | + [0.7-2.1 %β-Xylosidase, 2-4 % α-L-Arabinofuranosidase; 20-25% pectinolytic enzymes, including β-galactosidase, rhamnogalacturonase, polygalacturonase, exogalacturonase and galactanase, ∼5-8% starch modifying activity; 12-15% oxidoreductase/ oxidase and esterases + 8-12% protease] | | | | Arabinose, |
| | | | | | Galactose, |
| | | | | | Rhamnose |

**Table 23: Cocktails for production of fermentable sugar-rich hydrolysates from cellulose-rich hospital waste streams**

| **Cocktail** | **Cocktail composition (%)** | **Target application** | **Substrate** | **Optimum treatment temp (°C)** | **Products sugar** |
|---|---|---|---|---|---|
| MGBG 1 | CBH I (20-28%0 | Production of fermentable sugars for manufacture of biofuel | Sterilized cellulose-rich hospital waste stream) | 65°C (upto 85.°C) | Xylose Glucose, as the main sugars, with some galactose |
| | CBH II (15-20%) | | | | |
| | β(1,3)4-glucanase (20-25%) | | | | |
| | β-glucosidase (10-12%) | | | | |
| | Xylanase (20-25%) | | | | |
| | β-Xylosidase (5-10%) | | | | |
| | α-Glucuronidase (5-8%) | | | | |
| | α-L-Arabinofuranosidase (0.5-2.0%) | | | | |
| | (8-15%: Other hydrolases, including Pectinolytic enzymes phenolic acid and acetyl(xylan)esterases Protease; Lignin-modifying oxidase activities) | | | | |
| MGBG 2 | CBH I (15-20%) | Production of fermentable sugars for manufacture of biofuel | Sterilized cellulose-rich hospital waste stream) | 60°C (upto 85°C) | Glucose and xylose as the main sugars, with some mannose and galactose |
| | CBH II (20-28%) | | | | |
| | β(1,3)4-glucanase (20-26%) | | | | |
| | β-glucosidase (10-11%) | | | | |
| | Xylanase (18-30%) | | | | |
| | β-Xylosidase (8-10%) | | | | |
| | α-Glucuronidase (8-10%) | | | | |
| | α-L-Arabinofuranosidase (0.5-2.0%) | | | | |
| | (6-17%: Other hydrolases, including Pectinolytic enzymes Phenolic acid and acetyl(xylan)esterases Protease; Lignin-modifying oxidase activities) | | | | |
| MGBG 4 | CBHI (12-15%) | Production of fermentable | Sterilized cellulose-rich | 60°C (upto 85°C) | Glucose and xylose as the main sugars, with some |
| | CBH II (24-30%) | | | | |
| | β(1,3)4-glucanase (20-22%) | | | | |
| | β-glucosidase (~10%) | sugars for manufacture of biofuel | hospital waste stream) | | |
| | Xylanase (20-30%) | | | | mannose and galactose |
| | β-Xylosidase (5-8%) | | | | |
| | α-Glucuronidase (8-10%) | | | | |
| | α-L-Arabinofuranosidase (1.5-3.0%) (10-15%: Other hydrolases, including Pectinolytic enzymes Phenolic acid and acetyl(xylan)esterases Protease; Lignin-modifying oxidase activities) | | | | |
| MGBG 27 | CBH I (50-55%) | Production of fermentable sugars for manufacture of biofuel | Sterilized cellulose-rich hospital waste stream) | 60°C (upto 85°C) | Glucose mainly; minor amount of xylose |
| | CBH II (20-25%) | | | | |
| | β(1,3)4-glucanase (12-20%) | | | | |
| | β-glucosidase (5-8%) | | | | |
| | Xylanase (∼5%) | | | | |
| | β-Xylosidase (0.1-0.5%) | | | | |
| | α-L-Arabinofuranosidase (0.5-2.0%) | | | | |
| | (5-8%: Other hydrolases, including selected Pectinolytic enzymes, esterases; 0.5-1.5%Protease; 0.5-1.5% oxidase activities) | | | | |
| MGBG 31 | CBH I (5-10%) | Production of | Sterilized cellulose- | 60°C (upto 85°C) | Glucose and xylose as the main sugars, with some mannose, galactose and galacturonic acid |
| | CBH II (∼15%) | fermentable sugars | rich hospital waste | | |
| | β(1,3)4-glucanase (28-32%) | for manufacture of | stream) | | |
| | β-glucosidase (8-12%) | biofuel | | | |
| | Xylanase (22-30%) | | | | |
| | β-Xylosidase (10-15%) | | | | |
| | α-Glucuronidase (2-5%) | | | | |
| | α-L-Arabinofuranosidase (1-3.0%) | | | | |
| | (20-25%: Other hydrolases, including Pectinolytic enzymes Phenolic acid and acetyl(xylan)esterases Protease; starch-modifying enzymes, Lignin-modifying oxidase activities) | | | | |
| MGBG 34 | CBH I (20-25%) | Production of fermentable sugars for manufacture of biofuel | Sterilized cellulose-rich hospital waste stream) | 60°C (upto 85°C) | Glucose and xylose as the main sugars, with some mannose, galactose and galacturonic acid |
| | CBH II (10-15%) | | | | |
| | β(1,3)4-glucanase (24-28%) | | | | |
| | β-glucosidase (10-15%). | | | | |
| | Xylanase (25-30%) | | | | |
| | β-Xylosidase (5-8%) | | | | |
| | α-Glucuronidase (1-3%) | | | | |
| | α-L-Arabinofuranosidase (2-3.0%) | | | | |
| | (5-10%: Other hydrolases, including Pectinolytic enzymes Phenolic acid and acetyl(xylan)esterases Protease; starch-modifying enzymes, Lignin-modifying oxidase activities) | | | | |
| MGBG 35 | CBH I (10-12%) | Production of fermentable sugars for manufacture of biofuel | Sterilized cellulose-rich hospital waste stream) | 60°C (upto 85°C) | Glucose and xylose as the main sugars, with some mannose, galactose and galacturonic acid |
| | CBH II (∼15%) | | | | |
| | β(1,3)4-glucanase (30-40%) | | | | |
| | β-glucosidase (5-10%) | | | | |
| | Xylanase (15-28%) | | | | |
| | β-Xylosidase (2-5%) | | | | |
| | α-Glucuronidase (1-3%) | | | | |
| | α-L-Arabinofuranosidase (1-3.0%) | | | | |
| | (∼15%: Other hydrolases, including Pectinolytic enzymes Phenolic acid and acetyl(xylan)esterases Protease; starch-modifying enzymes, Lignin-modifying oxidase activities) | | | | |
| MGBG 35 | CBH I (10-12%) | Production of fermentable sugars for manufacture of biofuel | Sterilized cellulose-rich hospital waste stream) | 60°C (upto 85°C) | Glucose and xylose as the main sugars, with some mannose, galactose and galacturonic acid |
| | CBH II (∼15%) | | | | |
| | β(1,3)4-glucanase (30-40%) | | | | |
| | β-glucosidase (5-10%) | | | | |
| | Xylanase (15-28%) | | | | |
| | β-Xylosidase (2-5%) | | | | |
| | α-Glucuronidase (1-3%) | | | | |
| | α-L-Arabinofuranosidase (1-3.0%) | | | | |
| | (∼15%: Other hydrolases, including Pectinolytic enzymes such as β-galctosidase, rhamnogalacturonase, polygalacturonase, exo-galacturonase, mannan-degrading enzymes such as α-galactosidase; Phenolic acid and acetyl(xylan)esterases Protease; starch-modifying enzymes, Lignin-modifying oxidase activities), | | | | |
| MGBG 37 | CBH I (15-20%) | Production of fermentable sugars for manufacture of biofuel | Sterilized cellulose-rich hospital waste stream) | 60°C (upto 85°C) | Glucose and xylose as the main sugars, with some mannose, galactose and galacturonic acid |
| | CBH II (35-40%) | | | | |
| | β(1,3)4-glucanase (20-25%) | | | | |
| | β-glucosidase (5-10%) | | | | |
| | Xylanase (15-20%) | | | | |
| | β-Xylosidase (1-3%) | | | | |
| | α-Glucuronidase (0.5-2%) | | | | |
| | α-L-Arabinofuranosidase (1-3.0%) | | | | |
| | (12-15%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | | | | |
| MGBG 38 | CBH I (25-30%) | Production of fermentable sugars for manufacture of biofuel and other high-value product, including antibiotics, carotenoids, food flavours and aroma compounds, chemical feedstocks, etc. | Cotton-rich textiles, especially on highly purified forms of cotton | 60°C (upto 85°C) | Glucose, some mannose, galactose and galacturonic acid. Minor amounts of xylose |
| | CBH II (15-20%) | | | | |
| | β(1,3)4-glucanase (15-20%) | | | | |
| | β-glucosidase (2-8%) | | | | |
| | Xylanase (25-30%) | | | | |
| | β-Xylosidase (1-3%) | | | | |
| | α-Glucuronidase (0.5-2%) | | | | |
| | α-L-Arabinofuranosidase (1-3.0%) | | | | |
| | (15-20%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | | | | |
| MGBG 39 | CBH I (20-25%) | Production of fermentable sugars for manufacture of biofuel and other high-value product, including antibiotics, carotenoids, food flavours and | Cotton-rich textiles, including more mixed fibres | 65°C (upto 85°C) | Glucose mainly, with some xylose and mannose |
| | CBH II (20-25%) | | | | |
| | β(1,3)4-glucanase (20-25%) | | | | |
| | β-glucosidase (8-12%) | | | | |
| | Xylanase (20-25%) | | | | |
| | β-Xylosidase (2-5%) | | | | |
| | α-Glucuronidase (1-3%) | | | | |
| | α-L-Arabinofuranosidase (1-3.0%) | | | | |
| | (8-12%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | aroma compounds, chemical feedstocks, etc. | | | |
| MGBG 40 | CBH I (18-25%) | Production of fermentable sugars for manufacture of biofuel | Sterilized cellulose-rich hospital waste stream | 60°C (upto 85°C) | Glucose and xylose as the main sugars, with some mannose and galactose |
| | CBH II (15-20%) | | | | |
| | β(1,3)4-glucanase (25-30%) | | | | |
| | β-glucosidase (5-10%) | | | | |
| | Xylanase (20-25%) | | | | |
| | β-Xylosidase (0.5-2%) | | | | |
| | α-Glucuronidase (0.5-2%) | | | | |
| | α-L-Arabinofuranosidase (1.5-4.0%) | | | | |
| | (20-25%: Other hydrolases, including Pectinolytic enzymes such as β-galctosidase, rhamnogalacturonase, polygalacturonase, exo-galactfronase, mannan-degrading enzymes such as α-galactosidase, Phenolic acid and acetyl(xylan)esterases Protease; starch-modifying enzymes, Lignin-modifying oxidase activities) | | | | |
| MGBG 43 | CBH I (10-15%) | Production of fermentable sugars for manufacture of biofuel | Sterilized cellulose-rich hospital waste stream) | 60°C (upto 85°C) | Glucose and xylose as the main sugars, with some mannose |
| | CBH II (20-25%) | | | | |
| | β(1,3)4-glucanase (25-30%) | | | | |
| | β-glucosidase (5-10%) | | | | |
| | Xylanase (25-30%) | | | | |
| | β-Xylosidase (5-10%) | | | | |
| | α-Glucuronidase (1-3%) | | | | |
| | α-L-Arabinofuranosidase (1-3.0%) | | | | |
| | (10-14%: Other hydrolases, including Pectinolytic enzymes Phenolic acid and acetyl(xylan)esterases Protease; starch-modifying enzymes, Lignin-modifying oxidase activities) | | | | |

**Table 24: Cocktails for production of fermentable sugar-rich hydrolysates from textile waste streams**

| **Cocktail** | **Cocktail composition (%)** | **Target application** | **Substrate** | **Optimum treatment temp (°C)** | **Products sugar** |
|---|---|---|---|---|---|
| MGBG 34 | CBH I (20-25%) | Production of fermentable sugars for manufacture of biofuel and other high-value product, including antibiotics, carotenoids, food flavours and aroma compounds, chemical feedstocks, etc. | Cotton-rich textiles | 60°C (upto 85°C) | Glucose mainly with some xylose, galactose and galacturonic acid |
| | CBH II (10-15%) | | | | |
| | β(1,3)4-glucanase (24-28%) | | | | |
| | β-glucosidase (10-15%) | | | | |
| | Xylanase (25-30%) | | | | |
| | β-Xylosidase (5-8%) | | | | |
| | α-Glucuronidase (1-3%) | | | | |
| | α-L-Arabinofuranosidase (2-3.0%) | | | | |
| | (5-10%: Other hydrolases, including Pectinolytic enzymes Phenolic acid and acetyl(xylan)esterases Protease; starch-modifying enzymes, Lignin-modifying oxidase activities) | | | | |
| MGBG 37 | CBH I (15-20%) | Production of fermentable sugars for manufacture of biofuel and other high-value product, including antibiotics, carotenoids, food flavours and aroma compounds, chemical feedstocks, etc. | Cotton-rich textiles, including more mixed fibres and more processed. cotton textiles | 65°C (upto 85°C) | Glucose mainly, some smaller amounts of xylose and mannose |
| | CBH II (35-40%) | | | | |
| | β(1,3)4-glucanase (20-25%) | | | | |
| | β-glucosidase (5-10%) | | | | |
| | Xylanase (15-20%) | | | | |
| | β-Xylosidase (1-3%) | | | | |
| | α-Glucuronidase (0.5-2%) | | | | |
| | α-L-Arabinofuranosidase (1-3.0%) | | | | |
| | (12-15%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | | | | |
| MGBG 38 | CBH I (25-30%) | Production of fermentable sugars for manufacture of biofuel and other high-value product, including antibiotics, carotenoids, food flavours and aroma compounds, chemical feedstocks, etc. | Cotton-rich textiles, especially on highly purified forms of cotton | 60°C (upto 85°C) | Glucose, some mannose, galactose and galacturonic acid. Minor amounts of xylose |
| | CBH II (15-20%) | | | | |
| | β(1,3)4-glucanase (15-20%) | | | | |
| | β-glucosidase (2-8%) | | | | |
| | Xylanase (25-30%) | | | | |
| | β-Xylosidase (1-3%) | | | | |
| | α-Glucuronidase (0.5-2%) | | | | |
| | α-L-Arabinofuranosidase (1-3.0%) | | | | |
| | (15-20%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | | | | |
| MGBG 39 | CBH I (20-25%) | Production of fermentable sugars for manufacture of biofuel and other high-value product, including antibiotics, carotenoids, food flavours and aroma compounds, chemical feedstocks, etc. | Cotton-rich textiles, including more mixed fibres | 65°C(upto 85°C) | Glucose mainly, with some xylose and mannose |
| | CBH II (20-25%) | | | | |
| | β(1,3)4-glucanase (20-25%) | | | | |
| | β-glucosidase (8-12%) | | | | |
| | Xylanase (20-25%) | | | | |
| | β-Xylosidase (2-5%) | | | | |
| | α-Glucuronidase (1-3%) | | | | |
| | α-L-Arabinofuranosidase (1-3.0%) | | | | |
| | (8-12%: Other hydrolases, including Pectinolytic enzymes such as β-galctosidase, rhamnogalacturonase, polygalacturonase, exo-galacturonase, mannan-degrading enzymes such as α-galactosidase, Phenolic acid and acetyl(xylan)esterases Protease; starch-modifying enzymes, Lignin-modifying oxidase activities) | | | | |

**Table 25: Cocktails for production of fermentable sugar-rich hydrolysates from cereals, including whole crops, grains, processing residues and brewing wastes**

| **Cocktail** | **Cocktail composition (%)** | **Target application** | **Substrate** | **Optimum treatment temp (°C)** | **Products sugar** |
|---|---|---|---|---|---|
| MGBG 5 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes - especially barley, wheat, Rye, maize, malt/mash residues (including distillers' grains) | >60°C.(upto 80°C) | Xylose, arabinose Glucose, Cellobiose, xylobiose, Galactose, Glucuronic acid, some phenolic acids, small amounts of glucuronic acids, Galacturonic acid and Rhamnose and higher oligosaccharides |
| | CBH II (8-15%) | | | | |
| | β(1,3)4-glucanase (18-22%) | | | | |
| | β-glucosidase (6-10%) | | | | |
| | Xylanase (30-35%) | | | | |
| | + [5-12%β-Xylosidase, 10-15%; Exoxylanase, 3-5%, α-Glucuronidase 1.5-5.0 % α-L-Arabinofuranosidase; 8-10% pectinolytic enzymes, including β-galactosidase, rhamnogalacturonase, polygalacturonase, exogalacturonase and galactanase, 5-7% starch modifying activity; 4-7% other hemicellulases, including α-galactosidase, 1-4%,oxidoreductase/ oxidase and esterases + 2-6% protease] | | | | |
| MGBG 6 | CBH I (1-5%) | Production of fermentable sugars for manufacture of biofuel | Cereals, including whole crops, | >60°C (upto 85°C) | Xylose, Arabinose, Glucose mainly, small amounts of glucuronic |
| | CBH II (1-5%) | | | | |
| | β(1,3)4-glucanase (22-28%) | | | | |
| | β-glucosidase (10-15%) | and/or other high value products, e.g. (bio)chemicals/chemical feedstocks, carotenoids, antibiotics, probiotics, aroma and flavour precursors, natural sweeteners, etc. | grains, processing residues and brewing wastes - especially barley, wheat, and oats | | |
| | Xylanase (25-30%) | | | | acids, Galactose, Galacturonic acid and Rhamnose and disaccharides (cellobiose and xylobiose) |
| | + [0.7-2.1.%β-Xylosidase, 2-4 % α-L-Arabinofuranosidase; 20-25% pectinolytic enzymes, including β-galactosidase, rhamnogalacturonase, polygalacturonase, exogalacturonase and galactanase, ∼5-8% starch modifying activity; 12-15% oxidoreductase/ oxidase and esterases + 8-12% protease] | | | | |
| MGBG 7 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Residues from Barley, Millet, Wheat, Oats. | >60°C (upto 85°C) | Mainly Xylose, arabinose Glucose, Cellobiose, xylobiose, Glucuronic acid, some Galactose, phenolic acids, Galacturonic acid |
| | CBH II (8-12%) | | | | |
| | β(1,3)4-glucanase (25-30%) | | | | |
| | β-glucosidase (5-10%) | | | | |
| | Xylanase (40-45%) | | | | |
| | + [0.5-2.0% β-Xylosidase, 2-5.0 % α-Glucuronidase, 0.5-2.0% α-L-Arabinofuranosidase; 8-15% pectinolytic enzymes, mainly β-galactosidase and exogalacturonase, 2-4% esterases + 10-15% protease] | | | | |
| MGBG 8 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes especially Wheat, oats, barley, malt/mash residues (including distillers' grains) | >60°C (upto 80°C) | Xylose, arabinose Glucose, Cellobiose, xylobiose; smaller amounts of glucuronic acid, phenolic acids, and galactose. |
| | CBH II (5-10%) | | | | |
| | β(1,3)4-glucanase (∼20%) | | | | |
| | β-glucosidase (4-8%) | | | | |
| | Xylanase (25-30%, including 5-10%; Exoxylanase) + [1-2.0%β-Xylosidase, 5-8%, α-Glucuronidase, 0.5-3.0 % α-L-Arabinofuranosidase; 8-12% pectinolytic enzymes, including β-galactosidase, rhamnogalacturonase, polygalacturonase, exogalacturonase and galactanase, 8-12% starch modifying activity; 5-10% other hemicellulases, including α-galactosidase, 1-3%,oxidoreductase/ oxidase and esterases + 10-12% protease] | | | | |
| MGBG 9 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes - especially Wheat, oats, barley, sorghum and more novel adjunct residues, malt/mash residues (including distillers' grains) | >60°C (upto 80°C) | Xylose, arabinose Glucose, Cellobiose, xylobiose; smaller amounts of glucuronic acid, phenolic acids, and galactose. |
| | CBH II (5-10%) | | | | |
| | β(1,3)4-glucanase (20-25%, including high lichenanase) | | | | |
| | β-glucosidase (8-10%) | | | | |
| | Xylanase (25-30%, including ∼8-12%; Exoxylanase) + [2.5.0%β-Xylosidase, 1-4%, α-Glucuronidase, 2-5.0% α-L-Arabinofuranosidase; 5-10% pectinolytic enzymes, including α-galactosidase, rhamnogalacturonase, polygalacturonase, exogalacturonase and galactanase, 5-10% starch modifying activity; 3-6% other hemicellulases, including β-galactosidase, 2-4% oxidoreductase/ oxidase and esterases + 5-10% protease] | | | | |
| MGBG 10 | CBH I (3-6%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes - especially Wheat, oats, barley, maize, rye malt/mash residues (including distillers' grains) | >60°C (upto 80°C) | Xylose, arabinose Glucose, Cellobiose, xylobiose; smaller amounts of glucuronic acid, phenolic acids, and galactose. Some higher oligosaccharides. |
| | CBH II (5-10%) | | | | |
| | β(1,3)4-glucanase (25-40%, including high lichenanase) | | | | |
| | β-glucosidase (2-5%) | | | | |
| | Xylanase (∼25-30%, including ∼10-15%; Exoxylanase) + 5-12%β-Xylosidase, 2-5%, α-Glucuronidase, 2-5.0% α-L-Arabinofuranosidase; ∼5-8% pectinolytic enzymes, including β-galactosidase, rhamnogalacturonase, polygalacturonase, exogalacturonase and galactanase, 2-5% starch modifying activity; 2-5% other hemicellulases, including β-galactosidase, 1-3% oxidoreductase/ oxidase and esterases + 3-6% protease] | | | | |
| MGBG 11 | CBHI (1-5%) CBH II (5-8%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes - especially Wheat, maize, oats, barley, maize, rye malt/mash residues (including distillers' grains) | >60°C (upto 80°C) | Xylose, arabinose Glucose, smaller amounts of glucuronic acid, phenolic acids, galactose, and rhamnose. Some higher oligosaccharides. |
| | | | | | |
| | β(1,3)4-glucanase (20-25%, including high activity against high and medium DP mixed-linkage glucans) | | | | |
| | β-glucosidase (20-30%) | | | | |
| | Xylanase (∼15-20%, including -3-6%; Exoxylanase) | | | | |
| | + 2-6%β-Xylosidase, 1-3%, | | | | |
| | α-Glucuronidase, | | | | |
| | 1.5-3.0% α-L-Arabinofuranosidase; ∼8-12% pectinolytic enzymes, includingβ-galactosidase, rhamnogalacturonase, polygalacturonase, exogalacturonase and galactanase, 5-10% starch modifying activity; 6-11% other hemicellulases, including α-galactosidase, 3-7% oxidoreductase/ oxidase and esterases +10-15% protease] | | | | |
| MGBG 13 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Cereals, including whole crops, grains, processing residues and brewing wastes - especially oats, barley, wheat, malt/mash residues (including distillers' grains) | >60°C (upto 80°C) | Xylose, arabinose Glucose, Cellobiose xylobiose, Galactose, Glucuronic acid, some phenolic acids, Galacturonic acid and Rhamnose |
| | CBH II (5-10%) | | | | |
| | β(1,3)4-glucanase (25-40%) | | | | |
| | β-glucosidase (∼5%) | | | | |
| | Xylanase (18-25%) | | | | |
| | + [1-2.0%β-Xylosidase, 1-3.0%; Exoxylanase, 8-10%, α-Glucuronidase 1.5-5.0 % α-L-Arabinofuranbsidase; 10-15% pectinolytic enzymes, including β-galactosidase, rhamnogalacturonase, polygalacturonase, exogalacturonase and galactanase, 5-7% starch modifying activity; 5-10% other hemicellulases, including α-galactosidase, 1-4%,oxidoreductase/ oxidase and esterases + 8-12% protease] | | | | |

**Table 26: Cocktails for production of fermentable sugar-rich hydrolysates from non-cereal based Horticultural wastes, including grasses (and silage), leaves, pruning clippings and florists wastestreams**

| **Cocktail** | **Cocktail composition (%)** | **Target application** | **Substrate** | **Opt treatment temp (°C)** | **Products sugar** |
|---|---|---|---|---|---|
| MGBG 2 | CBH I (15-20%) | Production of fermentable sugars for manufacture of biofuel and/or other high value products, e.g. (bio)chemicals/chemical feedstocks, carotenoids, antibiotics, probiotics, natural sweeteners, antioxidant molecules, etc. | Horticultural wastes, including grasses, leaves, pruning clippings and mixed florists wastestreams; especially good for more woody materials | 60°C (upto 85°C) | Glucose and xylose as the main sugars, with some arabinose, mannose and galactose, glucuronic and galacturonic acids, some phenolic acids, and Rhamnose, as well as some higher DP oligosaccharides |
| | CBH II (20-28%) | | | | |
| | β(1,3)4-glucanase (20-26%) | | | | |
| | β-glucosidase (10-11%) | | | | |
| | Xylanase (18-30%) | | | | |
| | β-Xylosidase (8-10%) | | | | |
| | α-Glucuronidase (8-10%) | | | | |
| | α-L-Arabinofuranosidase (0.5-2.0%) | | | | |
| | (6-17%: Other hydrolases, including Pectinolytic enzymes Phenolic acid and acetyl(xylan)esterases Protease; Lignin-modifying oxidase activities) | | | | |
| MGBG 13 | CBH I (5-10%) | Production of fermentable sugars for manufacture of biofuel and/or other high value products, e.g. (bio)chemicals/chemical feedstocks, carotenoids, antibiotics, probiotics, natural sweeteners, antioxidant molecules, etc. | Horticultural wastes, including grasses, leaves, pruning clippings and mixed florists wastestreams; especially good for grasses and silage | >60°C (upto 80°C) | Xylose, arabinose Glucose, Cellobiose, xylobiose, Galactose, mannose, Glucuronic acid, some phenolic acids, Galacturonic acid and Rhamnose. Some higher oligosaccharides |
| | CBH II (5-10%) | | | | |
| | β(1,3)4-glucanase (25-40%) | | | | |
| | β-glucosidase (∼5%) | | | | |
| | Xylanase (18-25%) + [1-2.0%β-Xylosidase, 1-3.0%; Exoxylanase, 8-10%, α-Glucuronidase 1.5-5.0 % α-L-Arabinofuranosidase; 10-15% pectinolytic enzymes, including α-galactosidase, rhamnogalacturonase, polygalacturonase, exogalacturonase and galactanase, 5-7% starch modifying activity; 5-10% other hemicellulases, including α-galactosidase, 1-4%,oxidoreductase/ oxidase and esterases + 8-12% protease] | | | | |
| MGBG 21 | CBH I (5-10%) | Production of fermentable sugars for manufacture of biofuel and/or other high value products, e.g. (bio)chemicals/chemical feedstocks, carotenoids, antibiotics, probiotics, aroma and flavour precursors, antioxidant molecules, etc. | Horticultural wastes, including grasses, leaves, pruning clippings and mixed florists wastestreams; also good for bark | >60°C (upto 80/85°C) | Xylose, Glucose, Arabinose, Galactose, mannose Galacturonic acid, some Fructose, Rhamnose; some phenolic acids and sucrose; some higher oligosaccharides |
| | CBH II (5-10%) | | | | |
| | β(1,3)4-glucanase (15%) | | | | |
| | β-glucosidase (∼2-10%) | | | | |
| | Xylanase (50-55%) + [1-5%-Xylosidase, 5-10%; Exoxylanase, 1-4%, α-Glucuronidase, 2-5% α-L-Arabinofuranosidase; 20-25% pectinolytic enzymes, 4-6% starch modifying activity; 7-10% other hemicellulases, 2-4%, oxidoreductase/oxidase and esterases + ∼10% protease] | | | | |
| MGBG 32 | CBH I (1-5%) | Production of fermentable sugars for manufacture of biofuel and/or other high value products, e.g. (bio)chemicals/chemical feedstocks, carotenoids, antibiotics, probiotics, aroma and flavour precursors, antioxidant molecules, etc. | Horticultural wast including grasses, leaves, pruning clippings and mixe florists wastestrear | >60°C (upto 80°C) | Xylose, Glucose, Arabinose, Galactose, Galacturonic acid, some Fructose, Rhamnose; some phenolic acids |
| | CBH II (1-5%) | | | | |
| | β(1,3)4-glucanase (20-25%) | | | | |
| | β-glucosidase (8-12%) | | | | |
| | Xylanase (30-35%) + [0.5-1.5 %β-Xylosidase, 1-2.5 % α-L-Arabinofuranosidase; 18-25% pectinolytic enzymes, including α-galactosidase, rhamnogalacturonase, polygalacturonase, exogalacturonase and galactanase, ∼5-10% starch modifying activity; 8-15% oxidoreductase/oxidase and esterases + 10-15% protease] | | | | |
| MGBG 34 | CBH I (20-25%) | Production of fermentable sugars for manufacture of biofuel and other high-value product, including antibiotics, carotenoids, food flavours and aroma compounds, chemical feedstocks, etc. | Horticultural wastes, including grasses, leaves, pruning clippings and mixed florists wastestreams | 60°C (upto 85°C | Glucose, arabinose and xylose mainly with some galactose and galacturonic acid; phenolic acids, rhamnose, and some Oligosaccharide: |
| CBH | CBH II (10-15%) | | | | |
| | β(1,3)4-glucanase (24-28%) | | | | |
| | β-glucosidase (10-15%) | | | | |
| | Xylanase (25-30%) | | | | |
| | β-Xylosidase (5-8%) | | | | |
| | α-Glucuronidase (1-3%) | | | | |
| | α-L-Arabinofuranosidase (2-3.0%) | | | | |
| | (5-10%: Other hydrolases, including Pectinolytic enzymes Phenolic acid and acetyl(xylan)esterases Protease; starch-modifying enzymes, Lignin-modifying oxidase activities) | | | | |

**Table 27: Cocktails for production of fermentable sugar-rich hydrolysates from paper wastestreams**

| | | | | | |
|---|---|---|---|---|---|
| The paper wastes include: black and white newsprint, coloured newsprint, glossy magazines, paper cups, paper plates, tissues/mediwipes, brown paper, corrugated cardboard, white office paper, cellophane, kitchen towel, Kleenex^{™} and household cotton bandages | | | | | |

| **Cocktail** | **Cocktail composition (%)** | **Target application** | **Substrate** | **Optimum treatment temp (°C)** | **Products sugar** |
|---|---|---|---|---|---|
| MGBG 26 | CBH I (5-10%) | Production of fermentable sugars for manufacture of biofuel and other high-value product, including antibiotics, carotenoids, food flavours and aroma compounds, chemical feedstocks, etc. | Paper cups, brown paper, Kleenex™ and related paper waste products | 60°C (upto 85°C) | Glucose, arabinose and xylose, some galactose and galacturonic acid; some oligosaccharides |
| | CBH II (45-50%) | | | | |
| | β(1,3)4-glucanase (10-15%) | | | | |
| | β-glucosidase (0.5-2.0%) | | | | |
| | Xylanase (15-20%) | | | | |
| | β-Xylosidase (0.2-1.0%) | | | | |
| | α-Glucuronidase (0.5-2.0%) | | | | |
| | α-L-Arabinofuranosidase (0.5-1.5%) | | | | |
| | Other hydrolases, including 3-6% Pectinolytic enzymes, 0.2-1.0 Phenolic acid and acetyl(xylan)esterases 1-5% Protease; 5-10% starch-modifying enzymes, 2-5 oxidoreductase/ oxidase activities) | | | | |
| MGBG 27 | CBH I (50-55%) | Production of fermentable sugars for manufacture of biofuel and other high-value product, including antibiotics, carotenoids, food flavours and aroma compounds, chemical feedstocks, etc. | White office paper, paper plates and related products | 60°C (upto 85°C) | Glucose, smaller amounts of arabinose and xylose, galactose and galacturonic acid; some oligosaccharides, but mainly monosaccharides |
| | CBH II (20-25%) | | | | |
| | β(1,3)4-glucanase (12-20%) | | | | |
| | β-glucosidase (5-8%) | | | | |
| | Xylanase (∼5%) | | | | |
| | β-Xylosidase (0.1-0.5%) | | | | |
| | α-L-Arabinofuranosidase (0.5-2.0%) | | | | |
| | (5-8%: Other hydrolases, including selected Pectinolytic enzymes, esterases; 0.5-1.5%Protease; 0.5-1.5% oxidase activities) | | | | |
| MGBG 28 | CBH I (10-15%) | Production of fermentable sugars for manufacture of biofuel and other high-value product, including antibiotics, carotenoids, food flavours and aroma compounds, chemical feedstocks, etc. | White office paper, paper plates and related products | 60°C (upto 85°C) | Glucose, smaller amounts of arabinose and xylose, galactose and galacturonic acid; some oligosaccharides, but mainly monosaccharides |
| | CBH II (30-35%) | | | | |
| | β(1,3)4-glucanase (15-20%) | | | | |
| | β-glucosidase (2-5%) | | | | |
| | Xylanase (20-25%) | | | | |
| | β-Xylosidase (0.5-2.0%) | | | | |
| | α-L-Arabinofuranosidase (0.5-2.0%) (17-26%: Other hydrolases, including selected Pectinolytic and starch-modifying enzymes, 2-5% esterases; 8-10%Protease; 2-5% oxidase activities) | | | | |
| MGBG 29 | CBH I (8-10%) | Production of fermentable sugars for manufacture of biofuel and other high-value product, including antibiotics, carotenoids, food flavours and aroma compounds, chemical feedstocks, etc. | Mediwipes, cotton bandages, cardboard and cellophane | 60°C (upto 85°C) | Glucose, smaller amounts of arabinose and xylose, galactose and galacturonic acid; some oligosaccharides, but mainly monosaccharides |
| | CBH II (8-10%) | | | | |
| | β(1,3)4-glucanase (20-30%) | | | | |
| | β-glucosidase (25-30%) | | | | |
| | Xylanase (20-30%) | | | | |
| | β-Xylosidase (0.5-2.0%) | | | | |
| | β-L-Arabinofuranosidase (0.5-2.0%) | | | | |
| | Hydrolases, including selected 5-10%Pectinolytic and 5-8% starch-modifying enzymes, 1-3% esterases; 5-10%Protease; 2-5% oxidases | | | | |

**Table 28: Cocktails for production of fermentable sugar-rich hydrolysates from biodegradable packaging wastes/residues**

| **Cocktail** | **Cocktail composition (%)** | **Target application** | **Substrate** | **Opt.treat temp (°C)** | **Products sugar** |
|---|---|---|---|---|---|
| MGBG 30 | CBH I (20-25%) | Production of fermentable sugars for manufacture of biofuel and other high-value product, including antibiotics, carotenoids, food flavours and aroma compounds, chemical feedstocks, etc. | Biodegradable packaging wastes/residues | 60°C (upto 85°C) | Xylose, glucose, galactose, small amounts of mannose, galacturonic acid; and some oligosaccharides, but mainly monosaccharides |
| | CBH II (20-25%) | | | | |
| | β(1,3)4-glucanase (15-20%) | | | | |
| | β-glucosidase (1-5%) | | | | |
| | Xylanase (25-30%) | | | | |
| | β-Xylosidase (5-10%) | | | | |
| | α-L-Arabinofuranosidase (0.6-2.0%) (Other hydrolases, including selected 8-12% Pectinolytic enzymes, 4-8% starch modifying enzymes, 1-3% mannan-degrading enzymes, 1-5% esterases; 3-6.0%Protease; 2-5% oxidase activities) | | | | |
| MGBG 35 | CBH I (10-12%) | Production of fermentable sugars for manufacture of biofuel and other high-value product, including antibiotics, carotenoids, food flavours and aroma compounds, chemical feedstocks, etc. | Biodegradable packaging wastes/residues | 60°C (upto 85°C) | Glucose and xylose as the main sugars, with some mannose, galactose and galacturonic acid |
| | CBH II (∼15%) | | | | |
| | β(1,3)4-glucanase (30-40%) | | | | |
| | β-glucosidase (5-10%) | | | | |
| | Xylanase (15-28%) | | | | |
| | β-Xylosidase (2-5%) | | | | |
| | α-Glucuronidase (1-3%) | | | | |
| | α-L-Arabinofuranosidase (1-3.0%) (∼15%: Other hydrolases, including Pectinolytic enzymes such as α-gaalctosidase, rhamnogalacturonase, polygalacturonase, exo-galacturonase, mannan-degrading enzymes such as β-galactosidase, Phenolic acid and acetyl(xylan)esterases Protease; starch-modifying enzymes, Lignin-modifying oxidase activities) | | | | |
| MGBG 29 | CBH I (8-10%) | Production of fermentable sugars for manufacture of biofuel and other high-value product, including antibiotics, carotenoids, food flavours and aroma compounds, chemical feedstocks, etc. | Biodegradable packaging wastes/residues | 60°C (upto 85°C) | Glucose, smaller amounts of arabinose and xylose, galactose and galacturonic acid; mainly monosaccharides |
| | CBH II (8-10%) | | | | |
| | β(1,3)4-glucanase (20-30%) | | | | |
| | β-glucosidase (25-30%) | | | | |
| | Xylanase (20-30%) | | | | |
| | β-Xylosidase (0.5-2.0%) | | | | |
| | α-L-Arabinofuranosidase (0.5-2.0%) (Hydrolases, including selected 5-10%Pectinolytic and 5-8% starch-modifying enzymes, 1-3% esterases; 5-10%Protease; 2-5% oxidase ctivities) | | | | |

**Table 29: Cocktails for production of fermentable sugar-rich hydrolysates from Fungal biomass (including post-fermentation spent biomass) and marine algal biomass**

| **Cocktail** | **Cocktail composition (%)** | **Target application** | **Substrate** | **Optimum treatment temp (°C)** | **Products sugar** |
|---|---|---|---|---|---|
| MGBG 9 | CBH I (5-10%) | Production of fermentable sugars for manufacture of biofuel and/or other high value products, e.g. (bio)chemicals/ chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (5-10%) | | | | |
| | β(1,3)4-glucanase (20-25%, including high lichenanase) | | | | |
| | β-glucosidase (8-10%) | | | | |
| | Xylanase (25-30%, including ∼8-12%; Exoxylanase) + [2-5.0%β-Xylosidase, 1-4%, α-Glucuronidase, 2-5.0% α-L-Arabinofuranosidase; 5-10% pectinolytic enzymes, including β-galactosidase, rhamnogalacturonase, polygalacturonase, exogalacturonase and galactanase, 5-10% starch modifying activity; 3-6% other hemicellulases, including α-galactosidase, 2-4% oxidoreductase/ oxidase and esterases + 5-10% protease) | | | | |
| MGBG 13 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (5-10%) | | | | |
| | β(1,3)4-glucanase (25-40%) | | | | |
| | β-glucosidase (∼5%) | | | | |
| | Xylanase (18-25%) + [1-2.0%β-Xylosidase, 1-3.0%; Exoxylanase, 8-10%, α-Glucuronidase 1.5-5.0 % α-L-Arabinofuranosidase; 10-15% pectinolytic enzymes, including β-galactosidase, rhamnogalacturonase, polygalacturonase, exogalacturonase and galactanase, 5-7% starch modifying activity; 5-10% other hemicellulases, including α-galactosidase, 1-4%,oxidoreductase/ oxidase and esterases + 8-12% protease] | | | | |
| MGBG 44 | CBH I (2-5%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (2-5%) | | | | |
| | β(1,3)4-glucanase (20-25%) | | | | |
| | (1,3)6-glucanase (20-25%) | | | | |
| | 2-5% N-Acetylglucosaminidase and/or chitinase | | | | |
| | β-glucosidase (5-10%) | | | | |
| | Xylanase (10-15%) + [5-10%β-Xylosidase, 1-5%, α-Glucuronidase 1-5.0 % α-L-Arabinofuranosidase; 2-5% pectinolytic enzymes, including α-galactosidase, and galactanase, 2-5% starch modifying activity; 3-5% other hemicellulases, including mannanase, 2-5%, oxidoreductase/ oxidase and 2-4% esterases + 25-30% protease] | | | | |
| MGBG 45 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (5-10%) | | | | |
| | β(1,3)4-glucanase (15-20%) | | | | |
| | (1,3)6-glucanase (25-30%). | | | | |
| | 5-10% N-Acetylglucosaminidase and/or chitinase | | **Or** | | |
| | β-glucosidase (5-10%) | | Algal biomass and processing residues | | |
| | Xylanase (1-4%) + [2-5%β-Xylosidase, 5-10% pectinolytic enzymes, including β-galactosidase, and galactanase, 12-15% starch modifying activity; 2-5% other hemicellulases, including mannanase, 2-5%, oxidoreductase/ oxidase and 2-4% esterases + 10-15% protease | | | | |
| MGBG 46 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBR II (5-10%) | | | | |
| | β(1,3)4-glucanase (25-30%) | | | | |
| | β-glucosidase (5-10%) | | | | |
| | Xylanase (20-25%) + [2-5%β-Xylosidase, 1-4% α-Glucuronidase 2-5% α-L-Arabinofuranosidase; 5-8% pectinolytic enzymes, including β-galactosidase, and galactanase, 5-10% starch modifying activity; 1-2% other hemicellulases, including mannanase, 2-5%, oxidoreductase/ oxidase and 2-5% esterases + 30-35% protease] | | | | |
| MGBG 47 | CBH I (2-5%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (2-5%) | | | | |
| | β(1,3)4-glucanase (10-15%) | | | | |
| | (1,3)6-glucanase (40-45%) | | | | |
| | β-glucosidase (8-10%) | | | | |
| . | Xylanase (2-5%) + [1-3%β-Xylosidase, 1-2% α-Glucuronidase 2-5% α-L-Arabinofuranosidase; 2-5% pectinolytic enzymes, including β-galactosidase, and galactanase, 2-5% starch modifying activity; ∼5% other hemicellulases, including mannanase, 2-5%, oxidoreductase/ oxidase and 1-3% esterases] | | | | |
| MGBG 48 | CBH I (5-10%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (5-10%) | | | | |
| | β(1,3)4-glucanase (40-45%) | | | | |
| | (1,3)6-glucanase (10-15%) | | | | |
| | β-glucosidase (8-12%) | | | | |
| | Xylanase (10-15%) + [5-8%β-Xylosidase, 0.5-1.5% α-Glucuronidase 0.5-1.5% α-L-Arabinofuranosidase; 2-5% pectinolytic enzymes, including β-galactosidase, and galactanase, 2-5% starch modifying activity; ∼5% other hemicellulases, including mannanase, 1-3%, oxidoreductase/ oxidase and 1-3% esterases] | | | | |
| MGBG 49 | CBH I (0.4-2%) | Production of fermentable sugars for manufacture of biofuel and/or other high value products, e.g. (bio)chemicals/chemical feedstocks, carotenoids, antibiotics, probiotics, natural sweeteners, etc. | Yeast and filamentous fungal biomass | >60°C (upto 80°C) | Glucose, Cellobiose, mannose, Galactose, and some oligosaccharides mainly |
| | CBH II (0.4-1.0%) | | | | |
| | β(1,3)4-glucanase (7.0-15.0%) | | | | |
| | (1,3)6-glucanase (12-15%) | | | | |
| | β-glucosidase (3.0-5.0%) | | | | |
| | Xylanase (75.0-78.0%) +[0.4-2.0%β-Xylosidase, 0.5-1.5% α-Glucuronidase 0.2-1.5% α-L-Arabinofuranosidase; 2-5% pectinolytic enzymes, including β-galactosidase, and galactanase, 2-5% starch modifying activity; ∼4.0-7.0% other hemicellulases, including mannanase, 1-3%, oxidoreductase/ oxidase and 1-3% esterases] | | | | |

**Tabel 30: Cocktails for production of fermentable sugar-rich hydrolysates from Sugar Beet pulp, sugar cane and residues thereof**

| **Cocktail** | **Cocktail composition (%)** | **Target application** | **Substrate** | **Optimum treatment temp (°C)** | **Products sugar** |
|---|---|---|---|---|---|
| MGBG 6 | CBH I (1-5%) | Production of fermentable sugars for manufacture of biofuel and/or other high value products, e.g. (bio)chemicals/chemical feedstocks, carotenoids, antibiotics, probiotics, aroma and flavour precursors, etc. | Sugar beet, including tops, beet pulp and whole plant, as well as sugar cane and processing wastes | >60°C (upto 85°C) | Xylose |
| | CBH II (1-5%) | | | | Glucose, |
| | β(1,3)4-glucanase (22-28%) | | | | Galacturonic acid, |
| | β-glucosidase (10-15%) | | | | Fructose, |
| | Xylanase (25-30%) + [0.7-2.1 %β-Xylosidase, 2-4 % α-L-Arabinofuranosidase; 20-25% pectinolytic enzymes, including β-galactosidase, rhamnogalacturonase, polygalacturonase, exogalacturonase and galactanase, -5-8% starch modifying activity; 12-15% oxidoreductase/ oxidase and esterases + 8-12% protease] | | | | Arabinose, |
| | | | | | Galactose, |
| | | | | | Rhamnose |
| | | | | | Some oligosaccharides; phenolic acid released |
| MGBG 20 | CBH I (5-10%) | Production of fermentable sugars for manufacture of biofuel and/or other high value products, e.g. (bio)chemicals/chemical feedstocks, carotenoids, antibiotics, probiotics, etc. | Sugar beet, including tops, beet pulp and whole plant, as well as sugar cane and processing wastes | >60°C (upto 80/85°C) | Xylose Glucose, |
| | CBH II (3-10%) | | | | |
| | β(1,3)4-glucanase (20-32%) | | | | Galacturonicacid, |
| | | | | | Fructose, Sucrose |
| | β-glucosidase (∼2-10%) | | | | Arabinose, |
| | Xylanase (15-25%) + [15-20.0%β-Xylosidase, 10-15%; Exoxylanase, 2-5%, α-Glucuronidase 1.5-5% α-L-Arabinofuranosidase; 10-15% pectinolytic enzymes, ∼10% starch modifying activity; -5% other hemicellulases, 2-5%,, oxidoreductase/oxidase and esterases + ∼7-10% pretease | | | | Galactose, |
| | | | | | Rhamnose |
| | | | | | Some oligosaccharides; phenolic acid released |
| | | | | | |
| MGBG 18 | CBH I (5-10%) | Production of fermentable sugars for manufacture of biofuel and/or other high value products, e.g. (bio)chemicals/chemical feedstocks, carotenoids, antibiotics, probiotics, etc. | Sugar beet, including tops, beet pulp and whole plant, as well as sugar cane and processing wastes | >60°C (upto 80°C) | Xylose |
| | CBH II (5-10%) | | | | Glucose, |
| | β(1,3)4-glucanase (25-40%) | | | | Galacturonic acid, |
| | β-glucosidase (∼5%) | | | | Fructose, Sucrose |
| | Xylanase (18-25%) + [1-2.0%β-Xylosidase, 1-3.0%; Exoxylanase, 8-10%, α-Glucuronidase 1.5-5.0 % α-L-Arabinofuranosidase; 10-15% pectinolytic enzymes, including β-galactosidase, rhamnogalacturonase, polygalacturonase, exogalacturonase and galactanase, 5-7% starch modifying activity; 5-10% other hemicellulases, including α-galactosidase, 1-4%,oxidoreductase/ oxidase and esterases + 8-12% protease] | | | | Arabinose, |
| | | | | | Galactose, |
| | | | | | Rhamnose |
| | | | | | Some oligosaccharides; phenolic acid released |

Several of the cocktails listed above have applications in a wide range of other applications. In these applications, the key differences in the use of the cocktails lies in (a) enzyme dosage, or quantity used in each treatment, and (b) the duration of the incubation. For example, MGBG 18 is very well suited to the treatment of certain waste streams, as well as in nutraceutical applications. In the 'waste' treatment/saccharification steps, a higher dosage of enzyme is used and the reaction time is ∼18-24 h (∼25-32 Filter paper units). The ultimate goal is to achieve extensive breakdown of the target residue to fermentable monosaccharides. In contrast, where production of bioactive oligosaccharides (either glucoligosaccharides and xylooligosaccharides) is required, and/or a textural change to breads, a lower enzyme concentration is required and the modification (or reaction) time may take no longer than 1 (max 2) h to achieve the desired end-point.

Where the target substrate is primarily cellulose-rich, enzyme concentrations have been based on 'filter paper units or FPU'. Where a bioactive oligosaccharide (e.g. non-cellulosic β-glucooligosaccharide) is being produced, enzyme concentrations are based on the main activity required to fragment the target susbtrate (e.g. non-cellulosic, mixed-linkage β-1,3;1,4-glucans or β-1,3;1,6-glucans from fungal or algal sources).

### Example 13 Enzyme production by T. emersonii strains during liquid fermentation

*Talaromyces emersonii* strains examined were:
IMI (Imperial Mycological Institute (CABI Bioscience))393751 (Patent strain), IMI 393753 (CBS(Centraal Bureau voor Schimmelcultures) 180.68), IMI 393755 (CBS 355.92), IMI 393756 (CBS 393.64), IMI 393757 (CBS 394.64), IMI 393758 (CBS 395.64), IMI 393759 (CBS 397.64), IMI 393760 (CBS 472.92), IMI 393752 (CBS 549.92), IMI 393761 (CBS 759.71).

Liquid Fermentation; Replicate liquid cultures of the individual *T. emersonii* were grown at 45°C, in the medium described by Moloney *et al*., (1983) and Tuohy & Coughlan (1992), under pH uncontrolled conditions. The four carbon sources selected were: glucose (monosaccharide), oat spelts xylan (arabinoglucuronoxylan), carob powder and a 1:1 tea leaves/paper plates mixture (fragmented in a blender for ∼15-20 seconds, culture supernatants were recovered (Tuohy & Coughlan, 1992; Murray *et al*., 2002) and used to analyze extracellular enzyme production. Enzyme assays; Enzyme activity was expressed in International Enzyme Units (IU) per gram of inducing carbon source. One unit IU releases 1 micromole of product (reducing sugar, 4-nitrophenol etc.) per minute. All exoglycosidase and endo-hydrolase enzyme assays were conducted as described previously (Tuohy & Coughlan, 1992; Tuohy *et al*., 1994, 2002; Murray *et al*., 2002; Gilleran, 2004). Unless otherwise stated, all initial activity measurements were conducted at 50°C and pH 5.0. Exoglycosidase activities included: β-Glucosidase, α-Glucosidase, β-Xylosidase, β-Galactosidase, β-Mannosidase, β-Fucosidase, α-Arabinofuranosidase, N-Acetylglucosaminidase, α-Rhamnopyranosidase, α-Galactosidase, α-Fucosidase, α-Arabinopyranosidase, α-Mannosidase, and α-Xylosidase. α-Glucuronidase activity was assayed by a reducing sugar method using a mixture of reduced aldouronic acids as substrate (Megazyme International Ltd). This substrate contained reduced aldotriouronic, aldotetrauronic and aldopentauronic acids in an approx. ratio of 40:40:20. Activity was measured at pH 5.0 with a 5 mg/ml stock of this mixture. The reducing groups liberated during a 30 min incubation period were detected by the DNS method As some of the enzyme samples contain appreciable β-xylosidase activity that could liberate xylose residues from the aldo-uronic acids, the assay was repeated and xylose included in the reaction mixture to inhibit β-xylosidase activity.

Additional exo-acting xylanolytic enzymes such as: α-arabinoxylan arabinofuranohydrolase (release of arabinose from wheat straw arabinoxylan measured using an enzyme-linked assay), acetyl esterase (using 4-nitrophenyl and 4-methylumbelliferyl acetate substrates), acetyl xylan esterase activity (monitoring the release of acetate from acetylated beechwood xylan), ferulic acid esterase (spectrophotometric and HPLC assay methods) were also measured.

Endohydrolase activities included: β-D-(1,3;1,4)-Glucanase (β-glucan from barley (BBG) or lichenan as assay substrates), Xyloglucanase (tamarind xyloglucan), Laminarinase (laminaran from *Laminaria digitata*), endo-1,4-β-glucanase, (referred to as CMCase), based on activity against the commercial substrate carboxymethylcellulose, β-mannanase (carob galactomannan)pectinase and polygalacturonase, rhamnogalacturonase (soybean rhamnogalacturonan), galactanase (lupin and potato pectic galactans as substrates), arabinanase (sugar beet arabinan), amylase, glucoamylase, and dextrinase.

Temperature / pH optima and stabilities; The optimum temperature for activity was determined by carrying out the appropriate standard assays at temperature increments over the range 30- 100°C, in normal assay buffer (100 mM NaOAc, 5.0). Variation of pH with temperature was taken into consideration. pH Optima were determined using the following buffers pH 2.2-7.6 : McIlvaine-type constant ionic strength citrate-phosphate buffer; pH 7 - pH 10 Tris-HCl buffer. All buffers regardless of pH were adjusted to the same ionic strength with KCl.

Temperature and pH stabilities were determined as described previously (Tuohy *et al*., 1993; Gilleran, 2004; Braet, 2005)

Protein Determination; Protein concentration in enzyme samples (crude culture samples) was estimated by the Bensadoun and Weinstein modification of the method of Lowry (Bensadoun and Weinstein, 1976; Lowry *et al*., 1951) using BSA fraction V as a standard (Murray *et al*., 2001). Electrophoresis and Zymography; To determine the profile of proteins present in culture filtrates, a known volume of each sample was concentrated by lyophilization and analyzed by Native and/or renaturing SDS-PAGE or isoelectric focusing (IEF; Tuohy & Coughlan, 1992). Endoglycanase-active bands in the renatured SDS-PAGE gels and IEF gels were identified using a modification of the gel overlay technique of MacKenzie & Williams (1984), (Tuohy & Coughlan, 1992). To detect exoglycosidase activity, gels were incubated immediately in 50-100 pM solution of the appropriate 4-methylumbelliferyl glycoside derivative (reaction period of 2-30 min). Enzyme active band(s) were visualised under UV light using a Fluor-S^{™} Multimager (Bio-Rad).

### Results:

Liquid cultivation of the strains was repeated at least 3 times, in independent experiments conducted in different time periods. Culture filtrates harvested (120 h) from replicate flasks (for each strain/carbon source combination) were assayed for enzyme activity; multiple replicates were assayed at a range of enzyme dilutions. In addition, the results were validated by intra and interassay measurements, and independence of volume tests. There were clear differences between the cultures in terms of culture appearance and growth pattern. For example, the IMI393751 strain rapidly yielded quite a dense, filamentous culture on glucose, whereas the several of the other strains (e.g. CBS180.68, CBS355.92, CBS393.64, CBS395.64, CBS397.64, CBS 549.92 and CBS 759.71) displayed limited growth and atypical morphology i.e. absence of normal filamentous growth and formation of a slimy looking, limited culture mass. Strain CBS394.64 yielded a lower mycelia biomass, but did grow as a filamentous culture, while CBS472.92 adopted pellet morphology under identical growth conditions. A 120 h growth time-point was selected, as previous studies have shown that extracellular exoglycosidase and endoglycanase activities are present in significant quantities during growth of *T. emersonii* on most carbon sources (in pH uncontrolled conditions, 'peaking and troughing' of key activities has been observed. However, maximum activity is generally detected towards the end of the fermentation cycle).

Utilization of glucose was only approximately 15-25% by 120 h for many of the strains. Strain CBS 394.64 utilized ∼50-55% while CBS472.92 (which displayed pellet morphology) utilized ∼20-25% of the glucose in the medium. In contrast, ∼95% of the glucose in the culture medium was utilized by the strain of the invention (IMI 393751) by 72 h and no glucose was detected in the culture medium at the harvest timepoint of 120 h.

Tables 31A-D show the production of selected exoglycosidases by the strains. As the results reveal, clear distinctions can be seen between the strain of the invention and other *T. emersonii* strains with respect to exoglycosidase production.

Glucose does not completely repress exoglycosidase production by the *T. emersonii* strains (Table 31A). Strain 393751 produces significantly higher levels of β-glucosidase (BGase) than the other strains and the second highest levels of *N*-acetylglucosaminidase (NAGase) during growth on glucose. The production pattern obtained for the 393751 strain contrasts markedly with that for the CBS549.92 (previously CBS814.70) strain. Production of several exoglycosidase activities by the latter strain appears to be repressed by glucose. It should be noted that exoglycosidase activity levels were measured in undialyzed and dialyzed culture filtrates in case residual glucose in the medium was inhibiting BGase and/or NAGase present (similar patterns were noted in the dialyzed samples).

Carob induces differential production of extracellular glycosidases by the strains (Table 31B). Strain CBS 394.64 produces relatively no exoglycosidase activity apart from α-arabinofuranosidase. Low levels of all exoglycosidases were produced by strain CBS 393.64, CBS 395.64 and CBS 549.92. The 393751 strain produced significant levels of a broad range of exoglycosidases (highest levels of certain activities, e.g. the pectin modifying exoglycosidase β-ucosidase).

### Endoglycanase production by T. emersonii strains

A. Xylanase production: Two of the major type of endohydrolase activities required for conversion of plant biomass and waste residues rich in non-starch polysaccharides are glucanase and xylanase. Of all of the polymeric glycan degrading activities assayed, glucanase and xylanase were the predominant glycanase activities present.

Tables 32A-D present values for production of xylanase by all strains on the same carbon sources. Previous studies have shown that the wild type (CBS814.70) and other mutant strains produce a complex xylanolytic enzyme system (Tuohy *et al.,* 1993; 1994), with multiple endoxylanases. Several of the isolated xylanases display selective specificity towards different types of xylans, e.g. arabinoxylans, arabinoglucuronoxylans, glucurononxylans, more substituted xylans *versus* nonsubstituted xylans (from previous results and ongoing results with the enzymes from the strain of invention). As the results show, glucose is a strong repressor of xylanase expression in all *T. emersonii* strains. Significant levels of xylanase activity active against Oat spelts arabinoxylan (OSX) is expressed by the 393751 strain. This component is not active against rye or wheat arabinoxylans.

Carob, which is mainly rich in galactomannans (contains some xylan), is a potent inducer of very high levels of xylanase activity against all xylan substrates by the393751 strain. The role of carob as an inducer of potent xylanase acitivty would not be expected based on knowledge of its composition. The appearance of the cultures obtained for a number of the CBS strains (after 120 h) was significantly different and clear morphological differences could be observed between the 393751 and CBS549.92 strains, i.e. dense mycelial (filamentous) growth for IMI 393751 and formation of a slimy looking, limited (non-filamentous) culture mass for the CBS549.92 strain. As shown in Table 31B, the xylanase levels are significantly higher for the 393751 strain than any other In contrast to the 393751 strain, carob is a very poor inducer of xylanase in CBS394.64, CBS395.64, and CBS549.92 strains. Another marked difference can be seen in the type of xylanase activity induced by carob. In the 393751 strain potent activity is produced against all xylans. In the other strains, in general, very little or no activity against Rye and wheat arabinoxylans is produced. Only two strains other than the 393751 strain produce appreciable levels of activity against both of these xylans, i.e. CBS472.92 and CBS759.71. Zymogram analysis of the 393751 strain culture filtrate revealed high levels of multiple xylanase-active bands, including a new bi-functional xylanase which has been isolated.

The Tea leaves/paper plates (TL/PPL) mixture also proved to be a potent inducer of xylanase activity in the 393751 strain (Table 32C), and while this mixture did induce xylanase expression in the other strains, the levels were significantly lower. As observed with Carob, potent activity was produced by the 393751 strain against all xylans, with almost 1:8-fold greater activity against rye arabinoxylan (Rye AX) being obtained and lower activity against OSX and Birchwood xylan. This suggests differential expression of individual xylanases by the TL/PPL and Carob inducers, which was subsequently supported by zymogram analysis. TL/PPL also induces a multicomponent xylanolytic enzyme system, and complementary esterase and oxidase/peroxidase activities in the 393751 strain and not in the other strains. These complementary activities enhance the effectiveness of polysaccharide hydrolases in enzyme cocktails optimized for key biomass degradation applications (e.g. cereals, plant wastes, woody residues, paper products). In comparison with Carob, TL/PPL induces higher xylanase production by all strains (especially activity against the arabinoxylans), but levels are much lower than for the 393751 strain. Although OSX is a known inducer of xylanase in fungi and did induce enzyme production by all strains, the most pronounced induction was with the 393751 strain. However, in contrast to the 393751 strain, only OSX induced high xylanase activity and TL/PPL was a poor inducer of xylanase production by the 472.92 strain. The pattern of enzyme production on OSX is different to that obtained with carob and TL/PPL. Overall, the results for xylanase production on OSX, suggest that the 393751 strain can metabolize the crude substrates very rapidly and effectively to generate soluble inducers of xylanase. Hemicellulose in more complex crude substrates is more accessible to this strain. The results also suggest that the cocktails of enzymes produced by the 393751 strain on such complex substrates would be more suitable for hydrolysis of complex crude plant materials and residues. Model studies and applications investigated to date (e.g. woody biomass conversion, saccharification of carbohydrate-rich food and vegetable wastes and OFMSW and cereals) have confirmed the potential of these cocktails.

Finally, Figures 32A-D compare and contrast the production of xylanase active against the different assay substrates (i.e. OSX, Rye AX, etc.) by the 393751 strain and the parent strain CBS549.92 (also CBS814.70) on all four carbon sources.

B. Glucanase and Mannanase production: Previous studies have shown that the wild type (CBS814.70) and other mutant strains produce a complex glucanolytic enzyme system (Murray *et al*., 2001, 2004; Tuohy *et al.,* 2002; McCarthy *et al.,* 2003, 2005), which includes cellulases and an array of non-cellulolytic β-glucan modifying activities. As noted for the xylanolytic system, multiple endoglucanases are produced, depending on the carbon source. Several of the isolated β-glucanases display selective specificity towards different types of β-glucans.

Tables 7A-D show activity against the modified commercial β-1,4-glucan CMC (Sigma Aldrich), β-1,3;1,4-glucans from barley (BBG; Megazyme) and the lichen *Cetraria islandica* (Lichenan; Sigma Aldrich), xyloglucan (β-1,4-glucan backbone; Megazyme) from Tamarind and galactomannan from carob (Megazyme). Non-cellulosic β-glucans are present in significant concentrations in the non-starch polysaccharide component of a number of plant residues, especially those derived from cereals Tables 7A-D illustrate differential induction of the respective activities in the strains, with the pattern of induction being completely different on the more complex (crude) carbon sources.

Glucose is a potent repressor of glucanase and mannanase production in almost all of the strains (Table 33A). For all samples, activities were measured on dialyzed and un-dialyzed samples.

As the results reveal, Carob is a potent inducer of high levels of β-1,3;1,4-glucanase (against BBG and lichenan) in the 393751 strain, the highest levels for all of the strains tested (Table 33B). Levels of β-1,4-glucanase (against CMC) produced by the 393751 strain were ∼10-fold lower than activity against BBG (the level of CMCase was higher for this strain when compared with other strains). Even lower xyloglucanase was detected, with the levels obtained for the 393751 strain being the highest. Zymogram analysis confirmed that the types ofendoglucanase components, expression pattern and relative levels of expression of glucanase components in the respective *T. emersonii* cultures filtrates are markedly different. Low levels of (galacto)mannanase were produced by all strains during growth on carob.

The TL/PPL mixture was an even more potent inducer of β-1,3,1,4-glucanase (both BBGase and lichenanase), and (galacto)mannanase, by the 393751 strain (Table 33C). Overall these results highlight the non-equivalence of β-glucanase production by the *T. emersonii* strains and confirm that the 393751 strain is an excellent source of different β-glucanase activities and TL/PPL mixture induces a potent cocktail of these activities.

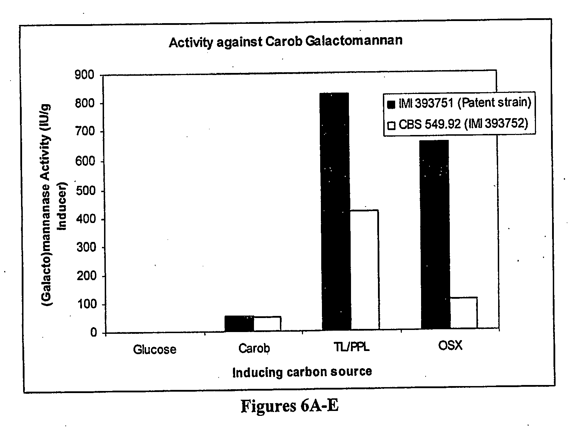

OSX (Table 33D), as expected, induced much lower levels of β-glucanase than either carob or TL/PPL. The pattern of enzyme production is different for the 393751 and CBS 549.92 strains. In Table 33D, β-1,4-Glucanase levels (Carboxymethylcellulase activity) were lower for most strains except CBS 397.64, which produced 2-fold higher levels than the 393751 strain (on OSX as inducer), and was not detected in culture filtrates of four strains (i.e. CBS 393.64, CBS 394.64, CBS 395.64 and CBS 549.92). Significant (galacto)mannanase activity was produced during growth on OSX by the 393751 strain (lower than with TL/PPL as inducer) and CBS 472.92. Figure 6A-E compare and contrast glucanase and mannanase production under the experimental conditions outlined by the 393751 and CBS814.70 strains.

In conclusion, the results indicate that:
the 393751 strain is a potent producer of high levels of a range of very important enzyme activities, the 393751 strain is the only *T. emersonii* strain that produces very high levels of both xylanase and β-1,3;1,4-glucanase on two key depolymerising hemicellulase activities, on low-cost inducers (carob and TL/PPL), and
highest activity levels were obtained on crude carbon sources thus providing demonstrating that the 393751 strain is a cost-effective source of a potent array of enzyme cocktails.

**Table 31A: Exoglycosidase production following growth for 120 h on glucose as carbon source.**

| **Glucose as inducer** | ***Talaromyces emersonii* strains (IU/g Inducer)** | | | | | | | | | |
|---|---|---|---|---|---|---|---|---|---|---|
| Exoglycosidase activity | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IMI 393761) |
| | | | | | | | | | | |
| α-Arabinofuranosidase | 3.69 | 5.83 | 6.82 | 5.17 | 6.00 | 6.33 | 2.64 | 4.73 | 4.18 | 3.08 |
| β-Xylosidase | 0.00 | 0.00 | 1.49 | 0.00 | 0.00 | 1.60 | 0.00 | 1.63 | 0.00 | 2.40 |
| β-Glucosidase | 31.90 | 1.96 | 15.42 | 0.99 | 0.19 | 10.67 | 2.20 | 2.15 | 8.97 | 9.30 |
| α-Glucosidase | 0.44 | 0.67 | 0.09 | 0.43 | 1.71 | 0.00 | 0.00 | 0.00 | 0.00 | 1.71 |
| β-Galactosidase | 0.28 | 1.16 | 0.00 | 0.55 | 0.86 | 0.00 | 0.00 | 0.00 | 0.00 | 1.49 |
| β-Mannosidase | 1.30 | 2.64 | 1.97 | 1.60 | 1.51 | 1.63 | 1.10 | 1.63 | 0.00 | 2.15 |
| β-Fucosidase | 0.00 | 0.11 | 0.58 | 0.22 | 0.00 | 0.09 | 0.00 | 7.37 | 0.00 | 1.10 |
| NAcetylGlucosaminidase | 86.08 | 156.97 | 27.83 | 33.72 | 0.00 | 43.56 | 59.57 | 74.91 | 6.22 | 34.87 |

**Table 31B: Exoglycosidase production following growth for 120 h on Carob as carbon source.**

| **Carob as inducer** | ***Talaromyces emersonii* strains IU/g Inducer)** | | | | | | | | | |
|---|---|---|---|---|---|---|---|---|---|---|
| Exoglycosidase activity | IMI 393751) | CBS 180.68 (IMI 393753) | CBS 355.92 (IMI 393755) | CBS 393.64 (IMI 393756) | CBS 394.64 (IMI 393757) | CBS 395.64 393758) | CBS 397.64 (IMI 393759) | CBS 472.92 (IMI 393760) | CBS 549.92 (IMI 393752) | CBS 759.71 (IMI 393761) |
| | | | | | | | | | | |
| α-Arabinofuranosidase | 0.72 | 0.22 | 3.30 | 1.49 | 7.04 | 1.45 | 1.32 | 1.05 | 2.55 | 1.49 |
| β-Xylosidase | 7.15 | 0.39 | 10.29 | 1.65 | 0.00 | 0.77 | 2.37 | 16.45 | 2.60 | 11.66 |
| β-Glucosidase | 23.65 | 17.22 | 67.54 | 6.55 | 0.00 | 10.04 | 24.53 | 85.36 | 11.25 | 74.14 |
| α-Glucosidase | 0.99 | 0.00 | 0.44 | 2.26 | 0.00 | 0.91 | 6.27 | 0.22 | 1.64 | 1.43 |
| β-Galactosidase | 2.97 | 4.24 | 1.71 | 3.52 | 0.00 | 1.95 | 3.30 | 1.49 | 1.73 | 3.03 |
| β-Mannosidase | 1.16 | 0.77 | 1.32 | 2.42 | 0.00 | 0.98 | 3.19 | 11.17 | 1.31 | 7.04 |
| β-Fucosidase | 36.58 | 0.00 | 4.79 | 1.76 | 0.00 | 0.50 | 3.19 | 10.12 | 0.62 | 6.55 |
| NAcetylGlucosaminidase | 31.02 | 15.95 | 19.91 | 3.85 | 0.00 | 7.98 | 14.85 | 44.55 | 6.73 | 18.15 |

**Table 31C: Exoglycosidase production following growth for 120 h on Tea leaves/Paper plates as carbon source.**

| **Tea Leaves/Paper plates** | ***Talaromyces emersonii* strains (IU/g Inducer)** | | | | | | | | | |
|---|---|---|---|---|---|---|---|---|---|---|
| Exoglycosidase activity | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IMI 393761) |
| | | | | | | | | | | |
| α-Arabinofuranosidase | 3.30 | 1.10 | 8.53 | 0.00 | 25.74 | 23.93 | 3.08 | 0.88 | 1.87 | 2.53 |
| β-Xylosidase | 6.93 | 0.00 | 3.08 | 0.00 | 12.49 | 12.82 | 1.98 | 4.18 | 1.93 | 1.43 |
| β-Glucosidase | 44.61 | 20.19 | 22.72 | 61.16 | 9.13 | 41.20 | 20.74 | 32.86 | 15.51 | 34.32 |
| α-Glucosidase | 0.44 | 0.00 | 3.41 | 0.00 | 18.10 | 7.65 | 0.83 | 0.00 | 0.61 | 0.44 |
| β-Galactosidase | 2.81 | 0.00 | 1.01 | 0.00 | 7.26 | 6.27 | 3.19 | 0.00 | 0.88 | 2.42 |
| β-Mannosidase | 1.16 | 0.00 | 3.25 | 0.00 | 12.60 | 12.27 | 2.48 | 0.00 | 1.44 | 1.98 |
| β-Fucosidase | 18.87 | 0.00 | 3.58 | 0.00 | 12.05 | 10.51 | 2.15 | 14.96 | 6.88 | 1.82 |
| NAcetylGlucosaminidase | 27.23 | 18.15 | 9.85 | 46.04 | 13.20 | 45.05 | 16.78 | 56.71 | 25.65 | 25.91 |

**Table 31D: Exoglycosidase production following growth for 120 h on Oat Spelts Xylan as carbon source.**

| **Oat Spelts Xylan** | ***Talaromyces emersonii* strains (IU/g Inducer)** | | | | | | | | | |
|---|---|---|---|---|---|---|---|---|---|---|
| Exoglycosidase activity | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IMI 393761 |
| | | | | | | | | | | |
| α-Arabinofuranosidase | 15.18 | 2.97 | 4.07 | 5.83 | 23.60 | 8.91 | 0.00 | 1.10 | 0.00 | 5.39 |
| β-Xylosidase | 9.63 | 0.00 | 2.26 | 0.66 | 0.00 | 2.10 | 11.77 | 14.91 | 12.16 | 6.93 |
| β-Glucosidase | 32.84 | 16.83 | 42.30 | 24.97 | 0.00 | 26.13 | 15.73 | 67.27 | 3.14 | 61.04 |
| α-Glucosidase | 0.92 | 0.00 | 1.05 | 0.77 | 0.00 | 0.17 | 1.76 | 0.00 | 0.44 | 0.84 |
| β-Galactosidase | 4.68 | 0.00 | 0.83 | 1.49 | 0.00 | 1.30 | 1.43 | 0.00 | 0.00 | 0.00 |
| β-Mannosidase | 0.00 | 0.00 | 2.09 | 2.97 | 0.39 | 2.75 | 2.53 | 0.00 | 0.11 | 0.48 |
| β-Fucosidase | 19.64 | 0.00 | 0.00 | 1.60 | 0.00 | 1.82 | 1.65 | 6.82 | 0.77 | 1.26 |
| NAcetylGlucosaminidase | 82.12 | 20.85 | 30.86 | 40.87 | 0.99 | 68.15 | 40.21 | 118.80 | 10.89 | 28.16 |

**Table 32A: Production of extracellular xylanase by T. emersonii strains on glucose as sole carbon source**

| **Glucose as inducer** | ***Talaromyces emersonii* strains (IU/g Inducer)** | | | | | | | | | |
|---|---|---|---|---|---|---|---|---|---|---|
| Xylan assay substrate | IMI 393751 | CBS 180.68 393753) | CBS 355.92 (IMI 393755) | CBS 393.64 393756) | CBS 394.64 393757) | CBS 395.64 (IMI 393758) | CBS 397.64 (IMI 393759) | CBS 472.92 393760) | CBS 549.92 (IMI 393752) | CBS 759.71 (IMI 393761 |
| | | | | | | | | | | |
| Oat Spelts Xylan | 513.10 | 28.71 | 0.00 | 0.00 | 0.00 | 0.00 | 0.00 | 0.00 | 0.00 | 0.00 |
| Rye Arabinoxylan | 0.00 | 0.00 | 0.00 | 0.00 | 0.00 | 0.00 | 0.00 | 0.00 | 0.00 | 0.00 |
| Wheat Arabinoxylan | 0.00 | 0.00 | 0.00 | 0.00 | 0.00 | 0.00 | 0.00 | 0.00 | 0.00 | 0.00 |
| Birchwood xylan | 254.49 | 155.98 | 736.78 | 147.79 | 279.13 | 779.90 | 0.00 | 452.87 | 116.99 | 0.00 |

**Table 32B: Extracellular xylanase activity in 120 h T. emersonii culture filtrates on Carob powder as sole carbon source**

| **Carob as inducer** | ***Talaromyces emersonii* strains (IU/g Inducer)** | | | | | | | | | |
|---|---|---|---|---|---|---|---|---|---|---|
| Xylan assay substrate | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IM I 393761) |
| | | | | | | | | | | |
| Oat Spelts Xylan | 3168.55 | 177.87 | 610.23 | 266.81 | 17.77 | 217.53 | 238.10 | 727.65 | 283.20 | 361.19 |
| Rye Arabinoxylan | 1212.20 | 0.00 | 0.00 | 15.07 | 0.00 | 0.00 | 0.00 | 135.47 | 90.31 | 201.30 |
| Wheat Arabinoxylan | 2464.00 | 21.89 | 116.27 | 108.08 | 0.00 | 0.00 | 49.28 | 365.31 | 9.57 | 271.15 |
| Birchwood xylan | 2520.27 | 474.76 | 607.48 | 513.10 | 373.45 | 428.23 | 348.92 | 751.30 | 348.92 | 636.35 |

**Table 32C: Extracellular xylanase activity in 120 h T. emersonii culture filtrates during growth on Tea leaves/Paper plates as sole carbon source**

| **Tea Leaves/Paper plates** | ***Talaromyces emersonii* strains (IU/g Inducer)** | | | | | | | | | |
|---|---|---|---|---|---|---|---|---|---|---|
| Xylan assay substrate | IMI 393751 | CBS 180.68 (IMI 393753) | CBS 355.92 (IMI 393755) | CBS 393.64 (IMI 393756) | CBS 394.64 (IMI 391757) | CBS 395.64 (IMI 393758) | CBS 397.64 (IMI 393759) | CBS 472.92 (IMI 393760) | CBS 549.92 (IMI 393752) | CBS 759.71 (IMI 393761 |
| | | | | | | | | | | |
| Oat Spelts Xylan | 2696.65 | 124.52 | 198.39 | 618.20 | 166.93 | 467.94 | 280.50 | 287.32 | 335.23 | 264.06 |
| Rye Arabinoxylan | 2398.55 | 102.63 | 180.62 | 522.67 | 260.15 | 399.52 | 441.93 | 281.88 | 114.95 | 254.49 |
| Wheat Arabinoxylan | 2525.60 | 53.35 | 123.15 | 499.40 | 123.20 | 351.62 | 305.09 | 261.25 | 170.34 | 236.01 |
| Birchwood xylan | 2127.40 | 417.29 | 458.37 | 771.65 | 457.00 | 626.45 | 727.65 | 617.10 | 647.13 | 521.29 |

**Table 32D: Extracellular xylanase activity in 120 h T. emersonii culture filtrates during growth on Oat Spelts Xylan as sole carbon source**

| **Oat Spelts Xylan** | ***Talaromyces emersonii* strains (IU/g Inducer)** | | | | | | | | | |
|---|---|---|---|---|---|---|---|---|---|---|
| Xylan assay substrate | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CB S 759.71 (IMI 393761 |
| | | | | | | | | | | |
| Oat Spelts Xylan | 2725.25 | 216.15 | 287.87 | 295.35 | 235.35 | 262.68 | 389.95 | 2126.30 | 789.47 | 305.09 |
| Rye Arabinoxylan | 2009.15 | 284.51 | 220.28 | 384.45 | 372.13 | 247.67 | 351.62 | 1543.30 | 723.80 | 328.35 |
| Wheat Arabinoxylan | 2722.50 | 180.62 | 265.43 | 297.00 | 191.51 | 197.01 | 220.28 | 1641.75 | 577.50 | 281.88 |
| Birchwood xylan | 2196.15 | 548.68 | 567.82 | 695.20 | 550.00 | 489.83 | 584.21 | 1908.50 | 871.53 | 623.92 |

**Table 33A: Extracellular glucanase and mannanase present in 120 h culture filtrates from the T. emersonii strains during growth on glucose as sole carbon source**

| **Glucose as inducer** | ***Talaromyces emersonii* strains (IU/g Inducer)** | | | | | | | | | |
|---|---|---|---|---|---|---|---|---|---|---|
| Glucanase/Mannanase Substrate | IMI 393751 | CBS 180.68 (IMI 393753) | CBS 355.92 (IMI 3937551 | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IMI 393761) |
| | | | | | | | | | | |
| Carboxymethyl cellulose | 0.00 | 0.00 | 0.00 | 0.00 | 0.00 | 0.00 | 0.00 | 0.00 | 0.00 | 0.00 |
| Barley β-glucan | 0.00 | 0.00 | 0.00 | 0.00 | 77.00 | 69.25 | 137.78 | 95.98 | 0.00 | 0.00 |
| Lichenan | 0.00 | 0.00 | 0.00 | 0.00 | 202.46 | 112.64 | 0.00 | 0.00 | 0.00 | 0.00 |
| Xyloglucan | 0.00 | 0.00 | 0.00 | 0.00 | 0.00 | 0.00 | 0.00 | 0.00 | 0.00 | 0.00 |
| Carob Galactomannan | 0.00 | 0.00 | 0.00 | 0.00 | 0.00 | 0.00 | 0.00 | 0.00 | 0.00 | 0.00 |

**Table 33B: Extracellular glucanase and mannanase present in 120 h culture filtrates from the T. emersonii strains during growth on Carob as sole carbon source**

| **Carob as inducer** | ***Talaromyces emersonii* strains (IU/g Inducer)** | | | | | | | | | |
|---|---|---|---|---|---|---|---|---|---|---|
| Glucanase/Mannanase Substrate | IMI393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 393759) | CBS 472.92 (IMI 393760) | CBS 549.92 (IMI 393752) | CBS 759.71 (IMI 393761 |
| | | | | | | | | | | |
| Carboxymethyl cellulose | 318.34 | 76.89 | 246.84 | 34.93 | 0.00 | 64.96 | 109.40 | 166.54 | 7.21 | 221.71 |
| Barley β-glucan | 3420.45 | 145.64 | 1120.35 | 81.29 | 0.00 | 60.06 | 281.77 | 1150.05 | 45.65 | 1702.47 |
| Lichenan | 2162.60 | 135.52 | 429.39 | 47.36 | 0.00 | 32.01 | 203.45 | 431.97 | 19.91 | 914.65 |
| Xyloglucan | 180.24 | 40.15 | 89.82 | 45.71 | 0.00 | 43.45 | 39.16 | 48.68 | 10.45 | 112.97 |
| Carob Galactomannan | 54.18 | 108.08 | 90.15 | 107.42 | 110.99 | 61.05 | 107.09 | 94.71 | 49.01 | 101.86 |

**Table 33C: Extracellular glucanase and mannanase present in 120 h culture filtrates from the T. emersonii strains during growth on Tea leaves/Paper plates as sole carbon source**

| **Tea Leaves/Paper plates** | ***Talaromyces emersonii* strains (IU/g Inducer)** | | | | | | | | | |
|---|---|---|---|---|---|---|---|---|---|---|
| Glucanase/Mannanase Substrate | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 3937581 | CBS 397.64 (IMI 393759) | CBS 472.92 (IMI 393760) | CBS 549.92 (IMI 393752) | CBS 759.71 (IMI 393761) |
| | | | | | | | | | | |
| Carboxymethyl cellulose | 368.61 | 90.75 | 147.02 | 282.10 | 0.00 | 131.56 | 196.90 | 396.72 | 257.95 | 109.40 |
| Barley β-glucan | 4341.15 | 393.47 | 783.20 | 1818.30 | 100.54 | 783.64 | 804.65 | 3113.00 | 1512.50 | 626.89 |
| Lichenan | 2735.15 | 452.54 | 581.90 | 1239.70 | 164.56 | 631.40 | 611.05 | 2268.75 | 1356.85 | 559.79 |
| Xyloglucan | 192.34 | 88.83 | 26.46 | 171.44 | 6.16 | 146.63 | 77.06 | 177.65 | 82.94 | 39.82 |
| Carob Galactomannan | 825.44 | 845.68 | 130.96 | 347.44 | 313.78 | 210.65 | 321.97 | 339.90 | 421.52 | 846.34 |

**Table 33D: Extracellular glucanase and mannanase present in 120 h culture filtrates fom the T. emersonii strains during growth on Oat Spelts Xylan as sole carbon source**

| **Oat Spelts Xylan** | ***Talaromyces emersonii* strains (IU/g Inducer)** | | | | | | | | | |
|---|---|---|---|---|---|---|---|---|---|---|
| Glucanase/Mannanase Substrate | IMI 393751 | CBS 180.68 (IMI 393753) | CBS 355.92 (IMI 393755) | CBS 393.64 (IMI 393756) | CBS 394.64 (IMI 393757) | CBS 395.64 (IMI 393758) | CBS 397.64 (IMI 3937591 | CBS 472.92 (IMI 393760) | CBS 549.92 (IMI 393752) | CBS 759.71 (IMI 393761) |
| | | | | | | | | | | |
| Carboxymethyl cellulose | 101.86 | 32.62 | 22.22 | 0.00 | 0.00 | 0.00 | 213.24 | 64.35 | 0.00 | 25.14 |
| Barley β-glucan | 375.82 | 100.27 | 100.87 | 44.72 | 21.23 | 72.49 | 283.42 | 282.76 | 52.58 | 117.87 |
| Lichenan | 477.68 | 176.33 | 197.56 | 114.62 | 203.72 | 135.52 | 353.27 | 360.80 | 115.61 | 840.40 |
| Xyloglucan | 35.26 | 44.39 | 0.00 | 0.00 | 3.91 | 6.88 | 230.84 | 25.14 | 0.00 | 38.23 |
| Carob Galactomannan | 653.84 | 344.80 | 56.82 | 85.53 | 174.02 | 95.48 | 298.38 | 641.30 | 110.72 | 139.10 |

### Example 14: Thermozymes from T. emersonii IMI393751 with potential for bioenergy production

The object was to reduce the biodegradable component of sterilized cellulose-rich clinical waste, thus reducing the volume of waste to landfill, and to recover the sugar-enriched liquid output after enzyme treatment and to recover energy in the form of biofuel.

The waste stream contained a high proportion of cellulose (>50%) and consisted mainly of paper, tissues, medical swabs and cotton-rich bandages and cloths, cotton wool, etc. The main 'fibre' in the wastestream is cellulose, but many products are 'finished' with polysaccharide coatings, binding agents and fillers, so a mixture of accessory enzymes (viz. hemicellulase, pectinase and starch-degrading enzymes) is essential to enhance cellulose accessibility and improve waste reduction or conversion to simple, soluble sugars (e.g. glucose, galactose, xylose, etc.).

### Experimental approach:

The profile of endohydrolase and exoglycosidase enzyme activities in each of 10 thermozyme cocktails, derived from the 393751 strain, were determined (Tuohy & Coughlan 1992; Tuohy *et al*., 1993, 1994, 2002; Murray *et al.,* 2001, 2004). Table 34 summarizes the relative levels of key activities determined in a selection of the cocktails. The enzyme preparations were added in different concentrations to 100 g batches of STG treated cellulose-rich waste, at 50°C and 70°C, and incubated for 24-48 h (at moisture levels of 50-60%). Samples of the sugar-rich liquor (and cellulose-rich materials, e.g. tissue, etc.) were removed periodically over 48 h and analyzed for (i) weight and volume reduction, (ii) volume of sugar rich liquor recovered, (iii) reducing sugars released, (iv) physical structure of substrate following enzymatic treatment (using scanning electron microscopy), (v) qualitative analysis of the types of sugars released by TLC, (vi) quantitative analysis of the sugars produced by HPLC, GC-MS and ESI-Q-TOF-MS, (vii) substances potentially toxic to fermentation microorganisms (bioenergy production), (viii) sterility of hydrolysates, (ix) bioethanol production, and (x) biogas production, as described by Tuohy *et al.*, 1993,1994, 2002; Murray *et al.*, 2001, 2004, Gilleran (2004) and Braet (2005).

### Results:

### A. Weight and volume reduction, volume of sugar rich liquor recovered, reducing sugars released

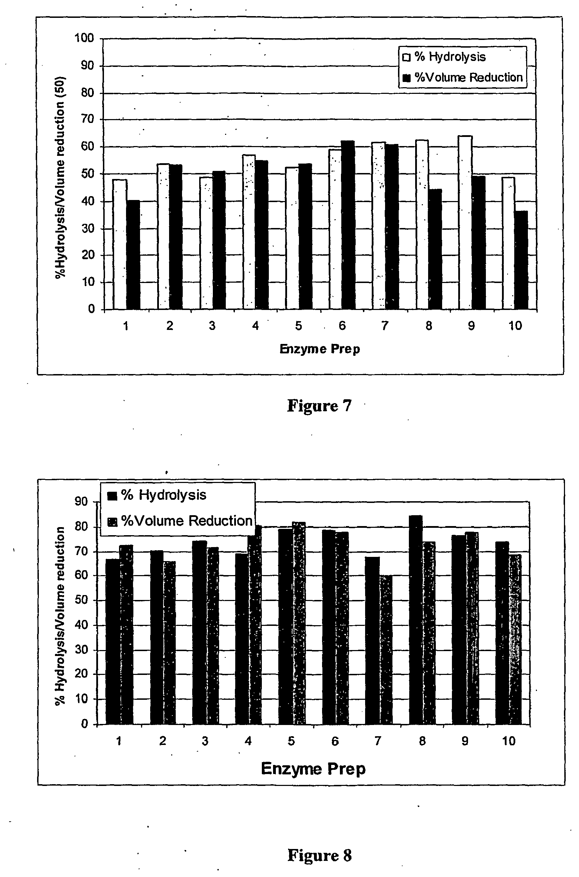

Pre- and post enzyme treatment weights were recorded and estimates of reduction in volume recorded for all of the enzyme preparations where made. Volume reduction data and reducing sugars obtained at 50°C, using the same reaction parameters (enzyme loading, 60% moisture content and a reaction time of 24 h), are illustrated in Figure 7.

In summary, 70°C for 24 h @ 60% moisture yielded the best results in terms of volume reduction for all of the cocktails. In repeated studies with the 6 selected cocktails (and with replicate tests), the volume reduction was consistently between ∼60-75% depending on the cocktail (see Figure 8). Reducing sugars released were converted to %hydrolysis or conversion of the cellulose present, which was between 66-82% of the cellulose-rich fraction present in the initial sterilized waste. A 60-75% volume reduction was obtained in laboratory tests.

Per 100 g batch, approximately 70-100 mL of added moisture (H₂O) were added to bring the final moisture content to 50-60%. Liquid recovery volumes; post enzymatic hydrolysis, ranged from 69-105 mL. Experiments were also completed at reaction temperatures of 75-85°C and different pH values. Above 70°C a marginal increase (∼2-5.5%) in the overall hydrolysis was noted, when compared with values obtained at 70°C, and while pH 3.5-4.0 yielded optimum levels of hydrolysis, values were not significantly greater (<5%) than obtained using H₂O.

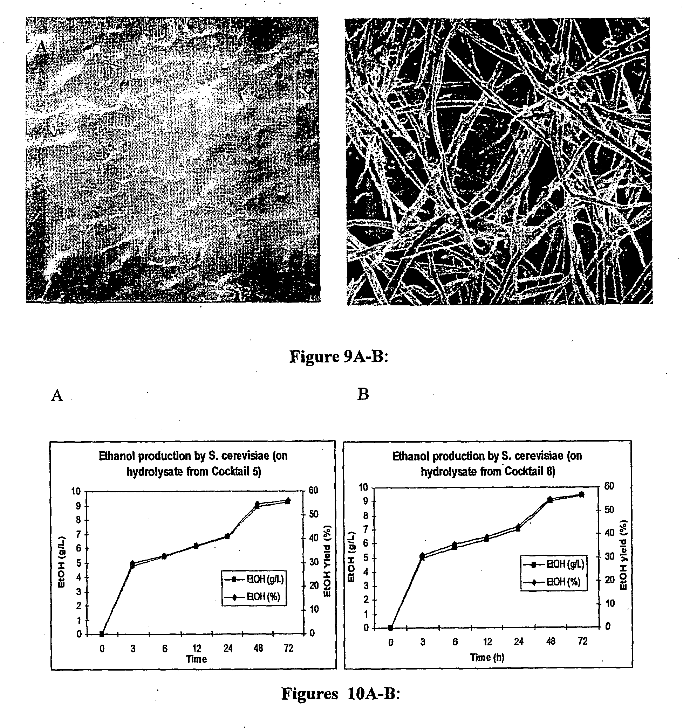

### B. Physical loss of substrate integrity, sugar products generated and relative amounts of each type of sugar. SEM demonstrated significant loss in cellulose fibre structure (Figure 9B)

### Hydrolysis Products: Qualitative analysis by TLC ; Quantitative analysis by HPLC

>76-92% of the sugar released was monosaccharide for the 7 best cocktails, and this consisted mainly of glucose, with some galactose, mannose and xylose. For example, sugar levels ranged from 0.2-0.55 g/mL and the monosaccharide concentration ranges determined by HPLC were as follows (dependent on the thermozyme cocktail and waste batch):
Glucose: 43-70%; Mannose: 5-15%; Galactose: 4-10%; Xylose: 20-30%; Cellobiose: 4-12% and higher oligosaccharides: 5-26%.

### C. Screening for substances that are potentially toxic to fermentation microorganisms (bioenergy production), before and after fermentation, analysis sterility of hydrolysates, and bioethanol and biogas production

The liquid fraction recovered did not appear to be toxic to yeast species screened for fermentation of the sugar-rich hydrolysates to bioethanol, i.e. did not prevent growth of S. *cerevisiae* (baker's yeast), *Pachysolen tannophilus, Pichia sp., Candida shehatae* and *Kluveromyces marxianus.* In addition, analysis of ethanol production (using an enzyme-linked assay kit) indicated that the yeasts were producing ethanol.

Agar plates (containing the appropriate agar medium) were inoculated with samples of the sugar-rich liquors and residual wastes, incubated under the recommended conditions (for the microorganism) and analyzed for the presence of colonies (bacteria and yeast) and radial growth (filamentous fungi). No microbial growth occurred in plates inoculated with the sugar-rich liquors and waste samples from the **70°C** enzyme treatments, i.e. microbial spoilage (and sugar loss) of the waste hydrolysates did not occur.

### Bioethanol production

Bioethanol production from sugar-rich feedstocks by different yeast species e.g. *Saccharomyces cerevisiae, Pachysolen tannophilus, Pichia sp., Candida shehatae* and *Kluveromyces marxianus* (and strains) was evaluated. End-points measured included yeast growth (and yeast biomass), utilization of sugars, evolution of CO₂ and ethanol produced. Two of the 70°C enzyme digests (hydrolysates generated by cocktails 5 & 8 in Figure 8) were selected as the test feedstocks for bioethanol production by all of the yeast species in 1 L Laboratory-scale cultures.

Figures 10A and B illustrate the ethanol production profiles obtained with S. *cerevisiae.* Ethanol yields are similar with both feedstocks, even though thermozyme cocktail 8 yields marginally more simple sugars in the hydrolysate. However, the digest from cocktail 8 contains higher pentose (not fermented by *S*. *cerevisiae*) than that generated by thermozyme cocktail 5. The thermozyme cocktail 5 digest contains some cellobiose (and smaller amounts of cellooligosaccharides) which are easily metabolised by the yeast.

**Table 34 results for a number of yeast + sugar-rich digest combinations**

| **Yeast species** | **Thermozyme cocktail 5 digest** | **Thermozyme cocktail 8 digest** |
|---|---|---|
| *S. cerevisiae:* | 9.2 g/L Ethanol | 9.4 g/L Ethanol |
| *P. tannophilus* | 8.5 g/L Ethanol | 8.8 g/L Ethanol |
| *K. marxianus* | 9.6 g/L Ethanol | 8.4 g/L Ethanol |
| *A. pullulans* | 6.7 g/L Ethanol | 7.4 g/L Ethanol |
| *C. shehatae* | 6.3 g/L Ethanol | 5.4 g/L Ethanol |

### Analysis of potential toxic end-of-fermentation products.

A range of techniques (HPLC, MS), especially GC-MS, were used to determine the presence of potential toxic end-of-fermentation products. Almost complete sugar utilization was achieved (with the expception of digests rich in pentose sugars (xylose, arabinose) for S. *cerevisiae, and* normal end-of-fermentation products were detected, i.e. glycerol, acetate and some trace by-products (solvents).

### Biogas production

Larger batches of Cocktail 5 and 8 digests were prepared to feed to mesophilic and thermophilic Upflow Anaerobic Hybrid Reactor (UAHR) Anaerobic digestors. The total carbohydrate levels of the influents and effluents of both reactors were measured (Dubois *et al.* 1956; Laboratory protocol). Reducing sugars present in influent and effluent samples were determined (Tuohy *et al.,* 1994). To determine the Chemical Oxygen Demand (COD), a known volume of the hydrolysate was oxidized using potassium dichromate (concentrated sulphuric acid with silver sulphate as catalyst), over 2 hours (international test method; Laboratory protocol). The remaining dichromate was determined by titration with a standardized solution of ferrous ammonium sulphate. COD and carbohydrate removal efficiency were measured (daily samples) throughout the trial. The specific methanogenic activity (SMA) of the sludges were analysed using the pressure transducer technique (Colleran and Pistilli, 1994; Coates *et al.* 1996). A sample of sludge was removed from the sludge bed through an outlet port and tests were carried out at either 37°C (for mesophilic reactor sludge) or 55°C (for thermophilic reactor sludge)..

The sugars present in the enzymatically-generated digests were metabolized quickly by the bacteria in both mesophilic (37°C) and thermophilic (55°C) UAHRs, i.e. 95-97% reduction of carbohydrate at loading rates of 4.5 g COD/m³/day, under non-optimized conditions. Methane levels in the biogas stream obtained were between 55-61%, and the estimated retention time (days) taken to metabolise all of the sugar and reach maximum methane levels was ∼3.0-4.0 days. pH of the effluent was monitored and there was no noticeable change in the pH of effluents from either reactor.

### C. Optimization of the thermozyme systems for treatment of cellulose-rich clinical wastestreams

A combination of genomics and functional proteomics was used to identify the optimum growth conditions and substrates to use (based on information from the 10 cocktails used in the initial experiments) to obtain an enzyme cocktail that would have optimum levels of all of the key enzyme components. Two inducer combinations were selected: a 1:1 mixture of spent tea leaves and waste paper plates, and a 1:1 mixture of sorghum and unmolassed beet pulp. Additional blends of selected cocktails from the 10 used above were also prepared. The novel cocktail and the blends were characterized with respect to the component enzymes and their ability to catalyse extensive conversion of commercial celluloses, hemicelluloses, and sterilized cellulose-rich waste to simple, fermentable sugars. Optimum pH and temperature for maximum enzyme reactivity, thermostability of the optimized enzyme system and blended cocktails, and potential inhibition by reaction end-product(s), the simple sugars, or potential toxic molecules (phenolic compounds/benzene-derivatives) were determined.
**Temperature Optima:** 75-80°C, with >70-85% activity remaining at 85°C, depending on the enzyme preparation/blend (enzyme activity was still detected at 90-95°C)
**Thermostability:** No real loss of activity after 24 h at 50°C and <5-10% loss of activity after 1 week at the same temperature.
At 70°C, <2-20% loss of activity in the first 24 h, with <10% further loss of activity thereafter over a 5 day period.
**pH Optima:** While the enzymes were most active at between pH 4-5, >60% activity was observed at pH 3.0 and pH 6.8, with all enzymes still displaying activity at pH 7.0. The enzyme preparations were most stable between pH 3.5-6.0 (4-50°C, over a period of 1 week).

While the rate of reaction/substrate conversion to monosaccharides did decrease or reach a plateau where high concentrations of glucose and other monosaccharides were present, the enzyme preparations were still quite active in the presence of high concentrations (upto 100 mM) of monosaccharides (glucose, xylose, arabinose, galactose). In addition, although the phenolic compounds/benzene-derivatives did decrease the overall activity of the cocktails, the concentrations used in the tests were significantly greater than present potentially in the waste. Nonetheless, certain cocktails and the optimized enzyme cocktail displayed significant activity (>50-70%) in the presence of these compounds.

Overall, the concentrations of each enzyme required to achieve ∼65-75% hydrolysis values was surprisingly low (9-16 Cellulase units/10 g waste) and, while cocktail/blend dependent, higher dosages (up to 60 Cellulase units) did not increase the final degree of hydrolysis markedly. Scale-up of enzyme treatment to 100 and 10 Kg batches yielded a similar final degree of hydrolysis, and a similar profile and concentration of sugar products. The best Volume reduction values obtained for the Optimized cocktails were 73-80%, while the corresponding %hydrolysis values for conversion of the cellulosic fraction to simple sugars were 72-81%. Two of the optimized blends yielded similar end-points in terms of volume reduction and cellulose hydrolysis. The Ethanol yield obtained was 195-210 L/tonne with S. *cerevisiae* and 215-220 L/tonne with *P. tannophilus*. Approximately, 80-85% of the bio-ethanol could be recovered by distillation, but this could be improved.

### Example 15: Thermozymes from T. emersonii IMI393751 for generation of sugar-rich feedstocks, from cellulose-rich paper and tissue wastes

### Enzyme Activity Measurements:

Crude enzyme preparations were analyzed for a range of different lignocellulose-hydrolysing enzyme activities using 10-30 mg/mL concentrations of the relevant substrates for endo-acting enzymes, 50 mg filter paper/mL reaction volume for 'filter paperase' (general cellulase) or 1 mM of the appropriate 4-nitrophenyl-glycoside derivative (Tuohy *et al.,* 2002; Murray *et al.,* 2001). Assays were performed in triplicate. All results are representative of two identical experiments using different crude enzyme preparations.

### pH and Temperature requirements for activity and stability:

The pH and temperature requirements for enzyme activity and stability were evaluated over the pH range from 2.6-7.6, and over a temperature range from 30-90°C, using the normal assay procedures.

### Model-scale hydrolysis studies:

An aliquot of individual enzymes, containing 5-60 FPU (or 2-20 xylanase units, as appropriate), was incubated with 1 g of the target substrate, at the appropriate pH and reaction temperature, for up to 24 h. Samples were removed at timed intervals and the sugar content (reducing sugars) and composition analysed.

**Cellulose-rich substrates investigated:** mixed tissue and lavatory paper, office paper waste, brown paper, mixed newsprint.

**Summary of Results:** The thermozyme cocktails displayed high activity on a broad spectrum of carbohydrate substrates and therefore reflect the complexity and efficiency of enzyme production by *T. emersonii* IMI393751. Production of particular thermozymes reflected the inducing substrate composition and variation (Table 35).

**Table 35: Relative amounts of cellulose and hemicellulose hydrolyzing enzymes**

| | **Cocktails** | **IU/g Inducer** | | | | | |
|---|---|---|---|---|---|---|---|
| **Activity** | MGBG 2 | MGBG 3 | MGBG 4 | MGBG 5 | MGBG 6 | MGBG 7 | MGBG 8 |
| β-glucosidase | 26.4 | 240.35 | 90.75 | 134.2 | 114.4 | 119.9 | 36.85 |
| Endocellulase | 710.6 | 468.6 | 310.2 | 747.45 | 799.15 | 70.4 | 919.6 |
| Endo 1,3,1,4 glucanase | 4471.5 | 3199.9 | 2067.45 | 8004.7 | 9493 | 313.5 | 6207.85 |
| Endoxylanase | 1234.2 | 1646.15 | 1051.6 | 1429.45 | 1369.5 | 653.95 | 1731.4 |
| Filterpaperase | 216.7 | 205.7 | 271.15 | 155.65 | 146.85 | 105.6 | 172.15 |
| β-xylosidase | 8.25 | 4.125 | 9.24 | 11.77 | 11.715 | 5.17 | 5.335 |
| αArabinofuranosidase | 4.29 | 33.33 | 17.105 | 34.87 | 20.24 | 7.205 | 23.155 |
| β-Galactosidase | 10.175 | 137.5 | 6.82 | 42.79 | 13.695 | 1.98 | 115.5 |
| Pectinase | 173.25 | 358.6 | 119.9 | 168.85 | 157.3 | 19.25 | 429.55 |

MGBG 2, derived from 108h *T. emersonii* cultures grown on a 1:1 mixture of spent tea leaves/paper plates; MGBG 3, derived from 120 h*T. emersonii* cultures grown on a 1:1 mixture of sorghum/beet pulp; MGBG 4, derived from 120 h*T. emersonii* cultures grown on a 1:1 mixture of wheat bran/beet pulp; MGBG 5, derived from 120 h*T. emersonii* cultures grown on a 1:1 mixture of paper plates/beet pulp; MGBG 6, derived from 120 h*T. emersonii* cultures grown on a (2:1:1) mixture of brown paper/paper plates/beet pulp; MGBG 7, derived from 120 h*T. emersonii* cultures grown on a 1:1 mixture of rye flakes/wheat bran; MGBG 8, derived from 120 h*T. emersonii* cultures grown on a 1:1 mixture of beet pulp/spent tea leaves.

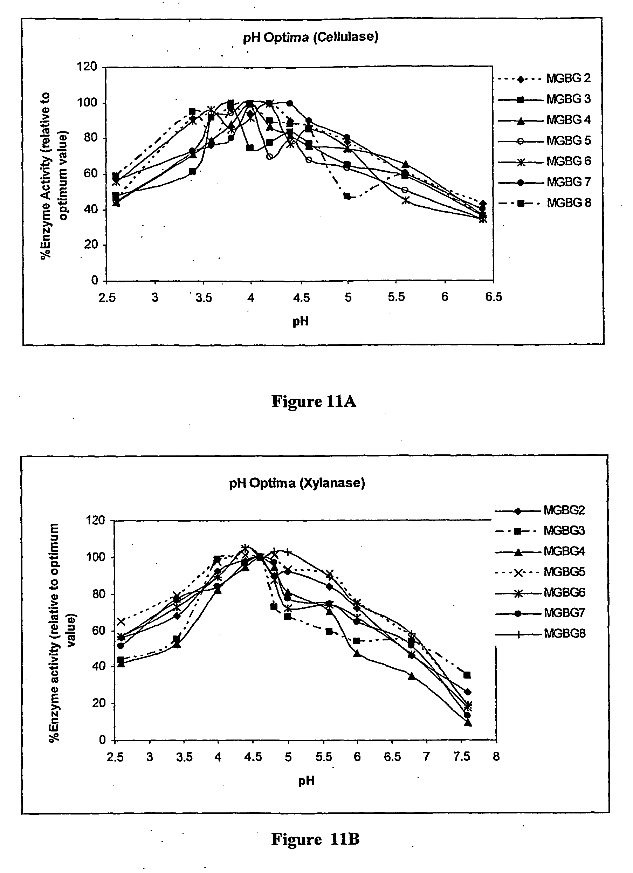

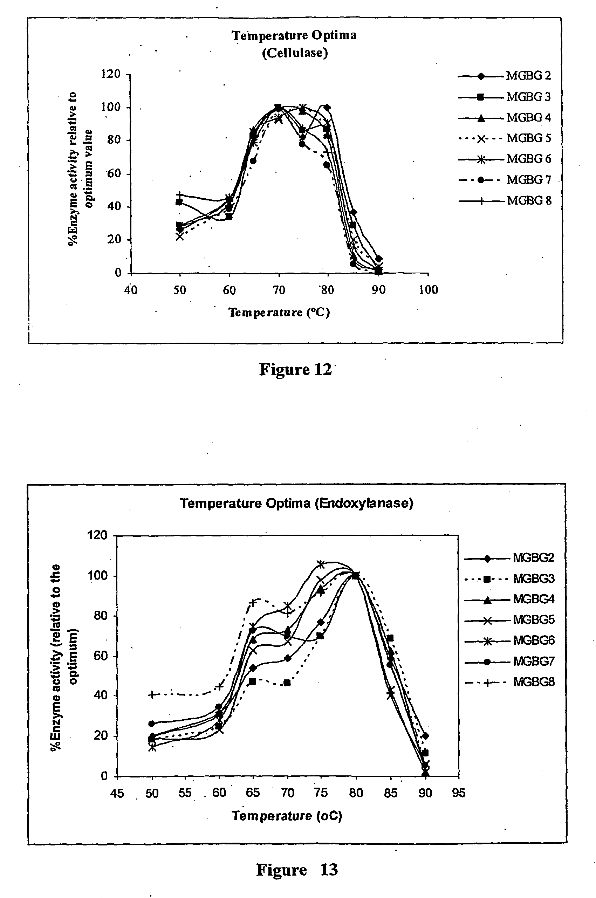

Cellulase in the thermozyme cocktails were most active around pH 4.0 (Figure 11A) and between 70°C and 80°C (Figure 12). The cellulase component(s) in each enzyme preparation are active over a broad pH range (<pH 2.6->pH 6.5). Xylanase activity present in the same cocktails was most active at pH 4.0-5.0 (Figure 11B) and between 75-85°C (Figure 13). However xylanase activity in the cocktails was active over a broad pH range (<(pH-2.6 to >pH 7.0), while similar activity levels were observed at 90°C and 50°C.

The thermal stability of several of the MGBG cocktails (listed in Table 35) was reflected in the long half-life values at 50°C and 70°C, i.e. effectively no or minimum loss in endoxylanase or endocellulase activity at 50°C after incubation in buffer only (pH 5.0). All of the cocktails were crude enzyme preparations and no stabilizers or enhancers were added. Xylanase activity present in cocktails 2, 5, 6 and 8 was particularly stable at 70°C (only ∼<2-20% loss of xylanase activity after 25 h). For example, the t½ value of cocktail 2 at 70°C was >6 days. The stabilities at 70°C of cocktails 3 and 4, and to a lesser extent cocktail 7, were lower due the presence of high levels of eqolisin protease (t½ values of 2 h, 12 h and 25 h). In the presence of substrate (i.e. crude waste), the stability of all three cocktails was markedly greater, i.e. (t½ values of 22 h, 46 h and 72 h). The general performance of the MGBG cocktails on cellulose and hemicellulose substrates was very high (Table 36). The level of hydrolysis increased significantly as the reaction temperature was increased from 50°C to 70°C (Table 37). At the higher reaction temperatures (e.g. 70°C), the %hydrolysis was achieved in 18 h and was similar to, and in some cases greater than, the %hydrolysis obtained after 24 h at 50°C (Table 37). This finding highlights the advantage of the thermozymes compared to the enzymes from mesophilic organisms, which would be active at lower temperatures.

**Table 36: %Hydrolysis of Cellulose-rich materials**

| | **Cocktail s** | **%Hydrolysis (50°C)** | | | | | |
|---|---|---|---|---|---|---|---|
| **Substrate** | **2** | **3** | **4** | **5** | **6** | **7** | **8** |
| Lavatory tissue | 37.9 | 85.6 | 9.8 | 9.7 | 47.4 | 22.4 | 22.6 |
| Paper cups | 17.6 | 20.0 | 16.1 | 27.5 | 34.2 | 16.3 | 26.2 |
| Filter paper | 18.3 | 23.7 | 7.2 | 17.4 | 18.8 | 0.0 | 20.7 |
| Barley β-glucan | 72.6 | 90.1 | 60.2 | 83.3 | 80.4 | 78.7 | 61.9 |

**Table 37: %Hydrolysis of Cellulose-rich tissue paper**

| | **MGBG 2** | **MGBG 3** | **MGBG 4** | **MGBG 5** | **MGBG 6** | **MGBG 7** | **MGBG 8** |
|---|---|---|---|---|---|---|---|
| 24 hours at 50°C | 70 | 87 | 51 | 79 | 80 | 66 | 71 |
| 18 hours at 70°C | 68 | 89 | 68 | 66 | 74 | 61 | 67 |

The products of the time-course hydrolysis of cellulose and hemicellulose, which were analysed by TLC, have confirmed the high conversion efficiency of MGBG thermozymes. The polysaccharide substrates were degraded initially to oligosaccharides and finally to glucose, which was almost the sole product of hydrolysis, while the cellulose present in cellulose-rich wastes, e.g. tissue paper were similarly hydrolysed almost completely to glucose.

The implications of these results for bio-ethanol based industry are significant and indicate the potential of the *T. emersonii* thermozyme systems.

### Example 16: Bioconversion of Beet Pulp to sugar-rich hydrolysates for Bioethanol production.

Eight thermozyme cocktails were selected from an initial range of 20 cocktails. The profile of endohydrolase and exoglycosidase enzyme activities in each of 8 thermozyme cocktails, derived from the 393751 strain, were determined (Tuohy & Coughlan, 1992, Tuohy *et al.*, 1993, 1994, 2002; Murray *et al.,* 2001, 2004).

The enzyme preparations were combined in different concentrations or dosages with 1 g batches of sugar beet fractions, prepared using different extraction methods. The fractions were as follows:
A. Sugar beet tops and stalks (1g dry weight / 10ml total volume)
B. Sugar beet pulp (1g dry weight / 10 ml total volume)
C. Sugar beet fruit (1g dry weight)
D. Sugar beet peel (1g dry weight)

Sugar beet fractions A-D were homogenized in a Waring blender (2 x 30 sec bursts). Aliquots of enzyme, i.e. 2ml and 5ml of enzyme solutions (1-8) were added to a final total reaction volume of 10 ml (with tap water, pH 7.2) and incubated at 63°C initially. Further experiments were conducted to optimize reaction temperature (75°C), pH (pH 4.0) and to reduce incubation time (16 h). Samples were taken at timed intervals over the 16-48h incubation, centrifuged, and enzyme action terminated by boiling at 100°C for 10 min. The supernatant fraction was analysed for reducing sugars released.

Qualitative analysis of the types of sugars released was analyzed by TLC, and quantitative analysis of the sugars produced was determined by HPLC. Sugar-rich feedstocks were evaluated for bioethanol production (Tuohy *et al.,* 1993, 1994, 2002; Murray *et al.,* 2001, 2004; Gilleran, 2004; Braet, 2005).

### Results:

The optimum reaction conditions were 75°C and pH 4.0. Table 38 presents the reducing sugars released after 16 h under optimum conditions. Increasing incubation to 48 h increased the %hydrolysis in the case of the peel and fruit fractions. Optimization of the enzyme loading conditions enabled the incubation time to be decreased by half (i.e. 24 h) with yields shown in Table 39.

**Table 38: %Hydrolysis of the carbohydrate present after 16 h at 75°C**

| | **Cocktails** | **%Hydrolysis (75°C) - after 16 h** | | | | | |
|---|---|---|---|---|---|---|---|
| **Substrate** | **2** | **3** | **4** | **5** | **6** | **7** | **8** |
| Pulp | 35.5 | 91.1 | 48.8 | 38.5 | 59.8 | 37.4 | 48.6 |
| Peel | 12.8 | 32.7 | 5.8 | 14.8 | 7.8 | 9.8 | 20.0 |
| Fruit | 13.8 | 6.4 | 3.0 | 26.0 | 11.5 | 3.0 | 28.7 |

**Table 39: %Hydrolysis of the carbohydrate present after 24 h at 75°C with optimized enzyme loadings**

| | **Cocktails** | **Hydrolysis (75°C) - mg RS after 48 h** | | | | | |
|---|---|---|---|---|---|---|---|
| **Substrate** | **2** | **3** | **4** | **5** | **6** | **7** | **8** |
| Pulp | 84.4 | 90.8 | 85.4 | 88.4 | 69.9 | 82.5 | 90.0 |
| Peel | 86.3 | 79.8 | 38.5 | 53.2 | 77.5 | 81.6 | 67.5 |
| Fruit | 66.6 | 75.5 | 59.1 | 104.1 | 79.5 | 85.4 | 102.2 |

Development and optimization of a enzyme cocktail to effect hydrolysis was undertaken using a combination of genomics and functional proteomics (based on information from the 8 cocktails used in the initial experiments). Two cocktails were produced, labeled MGBG SB#1 and MGBG SB#2. Hydrolysis of the total sugar beet plant and the total fruit component by the two optimized cocktails were compared with Cocktails 3 and 5 from the previous experiments. Reactions were conducted at the optimum conditions for the two novel cocktails, i.e. 71°C and pH 4.5. Table 40 gives the relative levels of enzyme included in each reaction per g substrate (i.e. total sugar beet plant or the total fruit component). Tables 41 and 42 summarize the hydrolysis results obtained.

**Table 40: Enzyme loadings per g sugar beet substrate**

| **Activity** | **Cocktail 3** | **Cocktail 5** | **MGBG SB#1** | **MGBG SB#2** |
|---|---|---|---|---|
| Xylanase | 4.8 | 4.98 | 0.68 | 0.20 |
| Cellulase (FPase) | 1.64 | 1.12 | 0.12 | 1.22 |
| Pectinase | 0.96 | 2.26 | 0.30 | 0.56 |

The main differences between the optimized cocktails and Cocktails 3 and 5 were in the relative amounts of key activities, especially exo-glycosidases.

**Table 41: %Hydrolysis based on Reducing sugars released (71°C, pH 4.5) after 24 h**

| | **Cocktails (%Hydrolysis) - RS** | | | |
|---|---|---|---|---|
| **Substrate** | **3** | **5** | **MGBG SB#1** | **MGBG SB#1** |
| Beet fruit | 56.8 | 46.9 | 65.6 | 58.3 |
| Total plant | 51.8 | 68.3 | 97.5 | 87.8 |

**Table 42: % Of the total sugar released that corresponds to glucose**

| | **Cocktails** | | | |
|---|---|---|---|---|
| **Substrate** | **3** | **5** | **MGBG SB#1** | **MGBG SB#1** |
| Beet | 116.8 | 90.3 | 112.0 | 97.9 |
| Total plant | 95.0 | 65.6 | 67.1 | 43.2 |

Both novel cocktails released high levels of reducing sugar from the total plant homogenate. Of the Reducing sugars released approximately 88-90% was monosaccharide of which approximately 43-67% was glucose.

### Fermentation to bioethanol

Studies were conducted to investigate production of bioethanol from the total plant and Beet hydrolysates. More than 90% of the glucose present in the total plant hydrolysate was converted to bioethanol by *S. cerevisiae* (from 40 g/L fermentable sugar, approximately 9.0 g/L ethanol were obtained). Other yeast species were investigated for production of bioethanol (aerobic conditions), e.g. *Pachysolen tannophilus*. The latter yeast utilized glucose and pentose sugars released (xylose and arabinose) in repeated fermentations (>92-95% metabolism). However, ethanol yields were slightly lower than with *S. cerevisiae* ((from 40 g/L fermentable sugar, approximately 6.7 g/L ethanol were obtained).

### Selected biochemical properties of the novel cocktails

**Temperature Optima:** 75-80°C, with >75-87% activity remaining at 85°C, depending on the cocktail.

**Thermostability:** No real loss of activity after 24 h at **50°C** and <10% loss after 3 weeks at **50°C.** At **71°C,** ∼4-9% loss of activity in the first 24 h, with less than 5-7% further loss over 5 days.

**pH Optima and stabilities:** While both cocktails were most active at pH 4.5, >55% activity was observed at pH <2.6 and >50-60% at pH 6.8; both enzymes displayed significant (>48-58%) activity at pH 7.0. The enzyme preparations were most stable between pH 3.5-6.0 (4-50°C, over 1 month).

### Example 17: Identification and selected properties of a novel bi-functional xylanase produced by Talaromyces emersonii IMI393751

*Talaromyces emersonii* secretes between 14-20 distinct endoxylanase components when grown on the appropriate carbon source. Thirteen of these endoxylanases have been purified to homogeneity and characterized with respect to catalytic properties. The molecular weights of the purified endoxylanases vary between 30-130 kDa. Xylanase and glucanase expression is not equivalent between 10 *T. emersonii* strains grown under identical conditions on the same nutrient medium and carbon inducers. A new low molecular weight xylanase has been identified, Xyn XII (17.5 kDa) from the xylan-degrading system *T. emersonii* IMI393751 strain. Secretion of xylanases with Mᵣ values less than or equal to 20 kDa has been reported for a number of other bacterial and fungal species, but not for *T. emersonii* before this.

### Enzyme purification and characterization

*T. emersonii* IMI393751 was grown on a 1:1 mixture of wheat bran and beet pulp for 120 h at 45°C, 210 rpm (or alternatively for 11 days in solid (static) fermentation, 33% substrate:67% moisture; 45°C). Xylanase and protein contents of crude and fractionated enzyme samples were analyzed as described previously (Tuohy & Coughlan, 1992; Tuohy *et al.,* 1993,1994; Murray *et al.,* 2001). Crude enzyme extract was harvested as described previously (Tuohy & Coughlan, 1992). Ultrafiltration using an Amicon DC2 system, equipped with a HIP 10-43 hollow-fibre dialyzer was used to separate the novel xylanase (permeate fraction) from the higher molecular weight xylanases (retentate). Xyn XII was purified to homogeneity using a combination of fractionation techniques, including 'salting-out' or precipitation with (NH₄)₂SO₄ (0-90% cut), gel permeation chromatography (GPC) on Sephacryl S-200 SF (100 mM NaOAc buffer, pH 5.0 as eluent), ion-exchange chromatography (IEC) on Whatman DE-52 (equilibrated with 30 mM NaOAc buffer, pH 5.0; xylanase was eluted by application of a linear buffered 0.0-0.3 M NaCl gradient), followed by hydrophobic interaction chromatography (HIC) on Phenyl Sepharose CL-4B (equilibrated with 15% (NH₄)₂SO₄ in 30 mM NaOc, pH5.0). Prior to HIC, the xylanase sample was 'salted-in' with (NH₄)₂SO₄ to a final concentration of 15 % (w/v). Buffer salts and (NH₄)₂SO₄ were removed by application of the sample to Sephadex G-25 (not shown here). Finally the xylanase-rich fractions were fractionated further by application to a second anion-exchange column of DE-52, at pH 7.0, followed by gel permeation chromatography on Sephacryl S-100 HR (100 mM NaOAc buffer, pH 5.0 used as equilibration and irrigation buffer) and a final fractionation step on DEAE-Sepharose, pre-equilibrated with 50 mM NH₄OAc buffer, pH 5.5 (0.0-0.2 M NaCl gradient used to elute xylanase). Pooled enzyme was de-salted by application to Sephadex G-25 or BioGel P-6 and lyophilized prior to electrophoretic analysis.

### Selected properties of the new bi-functional xylanase

### Selected physicochemical properties

The purity of the new enzyme was confirmed by SDS-polyacrylamide gel electrophoresis in 15% (acrylamide/bis-acrylamide) gels, according to the method of Laemmli. SDS-PAGE revealed a single protein band on silver-staining that corresponded to an estimated Mᵣ of 17.5 kDa. Furthermore, a single protein band was obtained on IEF corresponding to a pI value of pH 5.0 for Xyn XII. The temperature optimum for Xyn XII-catalyzed degradation of OSX was determined to be 75°C, and the optimum pH for activity was pH 4.0 - 4.5. However, unlike Xyn I - XI, which lost between 25-81% of the respective original activities during a 10 min incubation period at pH 3.0, Xyn XII was remarkably acid stable and retained over 91% of its original activity at pH 3.0 even on extended incubation.

### Selected catalytic properties

Suitably diluted aliquots of Xyn XII were incubated with a range of polysaccharides, including various xylans, β-glucans, pectic polymers and fructan (all at 1.0% (w/v) concentration). Reducing sugars released during an extended 30 min incubation period were quantified as described above. Activity against aryl-glycosides (1.0mM) was determined using a microassay method (Murray *et al.,* 2001). Preliminary studies to determine kinetic constants were carried out by varying [substrate], xylan, between 0.2 - 25 mg/ml, under the normal assay conditions.

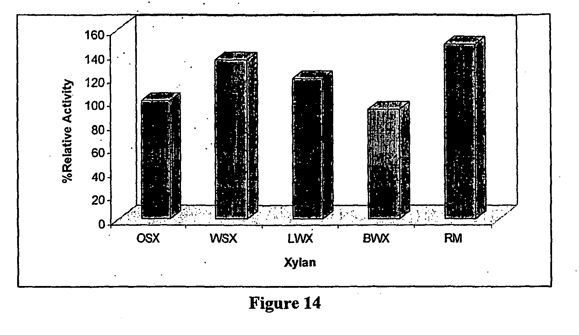

Results presented in **Figure 14** illustrate the relative reactivity of the new xylanase (Xyn XII) against different xylans. This enzyme is most active on a mixed linkage, unsubstituted xylan (1,3;1,4-β-D-xylan) known as rhodymenan from the red algae *Palmaria palmata.* In contrast, of the two cereal arabinoxylans, i.e. OSX and WSX, the enzyme displayed greatest activity against the more substituted WSX. The overall pattern of reactivity was: RM>WSX>LWX>OSX>BWX.

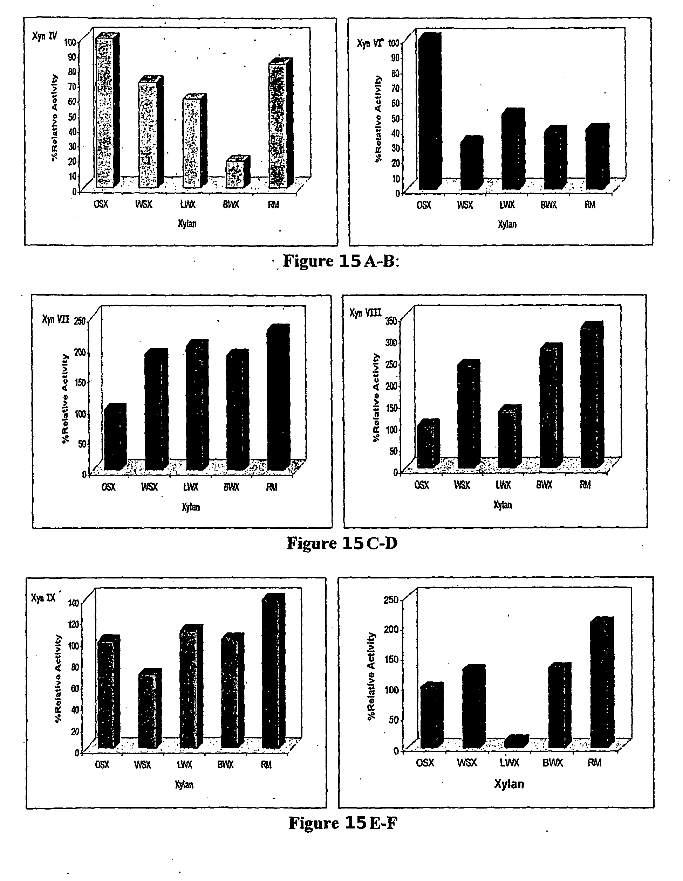

As **Figures 15A-G** demonstrate, the substrate preferences of a number of the other xylanases (Xyn IV to Xyn XI) are quite distinct from Xyn XII.

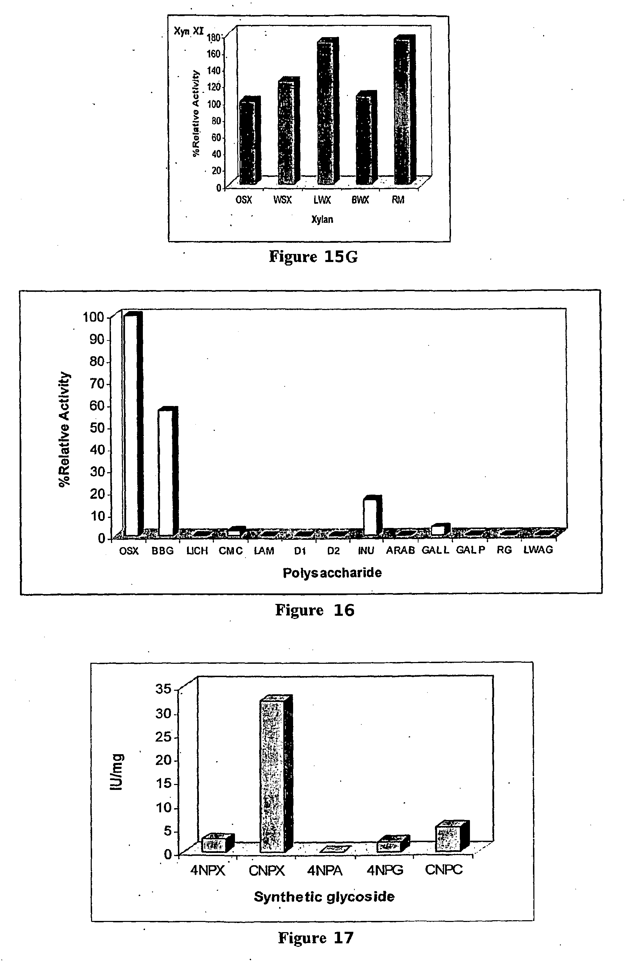

With Xyn IV, OSX>RM>WSX>LWX>>>BWX (Figure 15A). The order of reactivity with Xyn VI is OSX>LWX>RM≥BWX>WSX(**Figure 15B**), while that displayed by Xyn VII is RM>LWX>WSX≥BWX>>OSX (**Figure 15C**). The reactivity of Xyn VIII was RM>BWX>WSX>>LWX>OSX (**Figure 15D**), and Xyn IX was RM>LWX>BWX≥OSX>WSX (**Figure 15E**). Finally Xyn X displayed the following preference RM>>BWX∼WSX>OSX >>>LWX (**Figure 15**F) and Xyn XI RM≥LWX>WSX>BWX≥OSX (**Figure 15G**).

Furthermore, unlike Xyn I-XI which were strictly active against xylans only, the new bifunctional xylanase (Xyn XII) displayed substantial activity against mixed-linkage β-glucan from barley (1,3;1,4-β-D-glucan), i.e., over 55% activity relative to that observed with OSX, the normal assay substrate (**Figure 16**). In contrast to Xyn I-XI, the new bifunctional xylanase displayed activity against the aryl-β-xylosides 4-nitrophenyl β-D-xyloside (4NPX) and chloronitrophenylβ-D-xyloside (CNPX), with greater activity against the latter substrate (**Figure 17**). This finding may reflect the electron-withdrawing effect of the chloro moeity, making the chloronitrophenyl group a better 'leaving group'. However, other novel endoxylanases from the CBS814.70 strain of *T. emersonii* (Tuohy *et al.,* 1993; also produced by strain IMI393751, albeit differentially expressed) are significantly active against CNPX and display little or no activity against 4NPX. This phenomenon reflected partial 'exo'-acting characteristics of these enzymes, and may also reflect a similar characteristic for Xyn XII. In addition, low activity was observed against 4NP β-glucoside and CNP-β-cellobioside, which is not unexpected as Xyn XII is active against 1,3;1,4-β-D-glucan and if it possesses some exo-acting properties, as observed with the aryl β-xylosides, it might be expected to have corresponding activity against the aryl β-glucosides. Reactivity was not observed against any other α or β-linked aryl glycosides.

Thus, *"in vivo",* this new bifunctional xylanase may play a very important role for *T. emersonii* IMI393751 in providing access to plant cell wall hemicellulose, for example, by degrading mixed-link glucans in cereals and other plants, plant residues and wastes.

### Example 18: expression of key hydrolases and other accessory enzymes by T. emersonii strains.

### Expression of key enzymes on the same carbon source is different

Number, type and relative abundance of xylanase(s) produced by *T. emersonii* IMI393751 and other strains is different. As shown earlier, enzyme levels in culture filtrates are markedly different and suggest that the *T. emersonii* strains either produce different levels of the same xylanases, or express different isoforms. To illustrate this point, zymogram staining after gel SDS-PAGE electrophoresis (renatured as described by Tuohy & Coughlan (1992) and IEF was conducted with the IMI393751 and CBS549.92 strain culture filtrates. These studies confirmed that (i) different xylanases are expressed and (ii) the number of xylanase isoforms expressed are markedly different. The results obtained for xylanase expression on caroo are summarized as follows: Xylanase isoforms detected (see Tuohy *et al.,* (1994) for reference to the isoforms - pI and Mᵣ) **IMI393751:** Xyn IV, Xyn V, Xyn VI, Xyn IX and Xyn XI, Xyl I (β-xylosidase) and Xyl II **CBS549.92:** Xyn II, Xyn VII (note: Xyn VII is identical to the sequence published in an earlier patent (GenBank Accession Number AX403831) and very low amounts of Xyl I (β-xylosidase).

In addition, IMI393751 is the only strain that produces Xyn XII during growth on Tea leaves/paper plates (and on Tea Leaves only).

### Expression pattern on different carbon sources is different

The pattern of xylanase expression on different carbon sources is not-equivalent, i.e.
**IMI393751:**
   **Glucose** as carbon source: Xyn I, Xyn VIII, a smaller amount of Xyn XI and no β-xylosidase
   **Carob:** Xyn IV, Xyn V, Xyn VI, Xyn IX and Xyn XI, Xyl I (β-xylosidase) and Xyl II
   **TL/PPL:** Xyn III, Xyn V, Xyn IX, Xyn X, Xyn XII, Xyl I and Xyl II
**CBS549.92:**
   **Glucose:** low levels of a component that might be equivalent to Xyn IV and no β-xylosidase
   **Carob:** Xyn II, Xyn VII and very low amounts of Xyl I (β-xylosidase).
   **TL/PPL:** proteins similar to Xyn I, Xyn VII and Xyn IX, some Xyl I

In addition, other clear examples of differences in expression were observed, e.g. Xyl I expression No Xyl I is expressed by IMI393751, CBS549.92, CBS180.68, CBS393.64, CBS394.64 OR CBS397.64 during growth on glucose. In contrast, under identical conditions, Xyl I is expressed by CBS355.92, CBS395.64, CBS472.92 and CBS759.71.

Similarly on carob, Xyl I is expressed by all strains, albeit at markedly different levels, with the exception of CBS394.64. Xyl I is also expressed by all strains, except CBS180.68 and CBS394.64 during growth on TL/PPL. On OSX as carbon source, Xyl I was not expressed by CBS 180.68 and CBS394.64, but was expressed by CBS393.64 (low levels) and all other strains. Overall marked differences in Xyl I expression levels and the pattern of expression was noted (i.e. the strains expressing the greatest or lowest amounts of Xyl I) between all of the inducing substrates.

### Differences in specific activity of homologues produced by different strains.

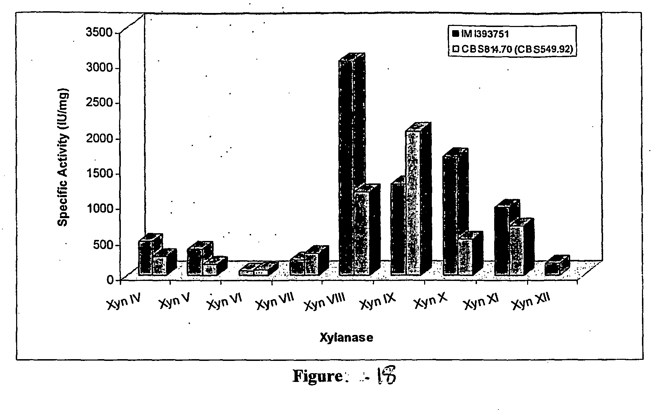

The different strains were compared with respect to expression of key xylanases as described above. However, in addition the xylanases present in induced cultures of IMI393751 and CBS549.92 were fractionated and the specific activity of the purified xylanase components compared. In the first instance, only IMI393751 appears to produce Xyn XII. Furthermore, when the specific activities were compared (results for OSX as assay substrate are presented below), some differences in specific activity of individual enzymes were clearly observed (**Figure 18**).

### Production of novel components by T. emersonii IMI393751 during growth on different

### carbon sources

*T. emersonii* IMI393751 was cultivated on a range of carbon sources and profiled by renaturing SDS-PAGE and IEF, following by zymogram staining, as outlined above. The following are a sample of some of the results obtained:
(i) **Tea leaves** as inducer: Xyn III, Xyn V, Xyn IX, some Xyn X and Xyn XII; Xyl I and Xyl II
(ii) **Carob:** Xyn IV, Xyn V, Xyn VI, Xyn IX and Xyn XI, Xyl I (β-xylosidase) and Xyl II
(iii) **Rye flakes:** Xyn IV, Xyn V, Xyn VI, Xyn IX, with an extra novel 'xylanase' component of pI 6.5; Xyl I (β-xylosidase) and Xyl II
(iv) **Retail flour:** Xyn III, Xyn VI, Xyn VIII, Xyn IX, Xyn X, Xyl I (β-xylosidase) and Xyl II, with additional novel 'xylanase' component of pI 5.8
(v) **Sorghum:** Xyn I, Xyn VIII, Xyn IX, Xyn X, Xyn XI, some Xyl I (β-xylosidase) and Xyl II
(vi) **Xylose:** Xyn I and Xyn VIII, Xyl I
(vii) **Glucose:** Xyn I, Xyn VIII, a smaller amount of Xyn XI and no ββ-xylosidase

### Example 19: Differential expression of other accessory enzymes by T. emersonii strains Glutathione peroxidase

Culture filtrate samples (unconcentrated and concentrated samples) were run on 7.5% native PAGE gels, soaked in reduced glutathione at 50°C followed by incubation with 0.002% H₂O₂. The gel was then stained with 1% ferric chlorine/1% Potassium ferricyanide. Zymogram staining was also complemented by enzyme assays on the culture filtrates.

IMI393751 produces extracellular Glutathione peroxidase (Mᵣ∼45 kDa), with differential expression being observed on a number of carbon inducers (the numbers 1-18 represent different inducers). In contrast no extracellular activity was observed for any of the other *T. emersonii* strains, even in the concentrated samples. Strain IMI393751 also produces significant levels of extracellular glutathione peroxidase during growth on TLIPPL.

### Catalase

Culture filtrate samples (unconcentrated and concentrated samples) were run on 7.5% native PAGE gels, followed by incubation with 3% H₂O₂. The gel was then stained with 1% ferric chloride/1% Potassium ferricyanide (Catalase bands appear as intense yellow bands). Zymogram staining was also complemented by enzyme assays on the culture filtrates, using the standard, published method.

IMI393751 produces extracellular Catalase (Mᵣ∼230 kDa), with differential expression being observed on a number of carbon inducers (the numbers 1-18 represent different inducers). In contrast no extracellular activity was observed for any of the other *T. emersonii* strains, even in the concentrated samples.

The presence of these enzyme activities in the culture filtrates of IMI393751 would be important potentially in affecting the structure of the substrate, but also in removing any compounds that might oxidase key hydrolase activities, e.g. xylanase or glucanase.

### Example 20:Comparison of the phenotype of 10 T. emersonii strains on different solid (agar) media

### B. Agar media

1. Sabouraud Dextrose Agar (Oxoid Ltd., UK)
2. Potato Maltose Agar (Oxoid Ltd., UK)
3. Czapek Dox (Oxoid Ltd., UK; pH not adjusted)
4. Cornmeal Agar (Oxoid Ltd., UK)
5. Malt Extract Agar (Oxoid Ltd., UK)
6. NutrientAgar (Difco Ltd., UK)
7. Emerson's Agar (Yeast potassium soluble starch; YpSs)
   The following ingredients were added to 1 L of distilled H₂O
   15.0 g Soluble starch (Sigma-Aldrich, Dublin, Ireland)
   4.0 g Yeast extract (Oxoid Ltd., UK)
   1.0 g Potassium *di*-hydrogen phosphate (KH₂PO₄)
   0.5 g Magnesium sulphate heptahydrate (MgSO₄.7H₂O)
   20.0 g Agar No.1 (Oxoid Ltd., UK)
8. Yeast Glucose Agar (YGA)
   The following ingredients were added to 1 L of distilled H₂O:
   20.0 g Glucose (Sigma-Aldrich, Dublin, Ireland)
   10.0 g Yeast Extract (Oxoid Ltd., UK)
   15.0 g Agar No.1 (Oxoid Ltd., UK)

Agar plates (each type of agar) were inoculated with the 10 different *T. emersonii* strains. One batch of plates was incubated at 45°C and the second at 55°C (moisture content of the air was ∼20-30%). Cultures were checked daily, and the following results were recorded:
(a) measurement of culture diameter (using a calipers)
(b) photographic record using a 7.0 M pixel digital camera
(c) record of visible phenotypic changes/differences
(d) indication of spore formation (microscopic analysis) or otherwise

### Results:

Cultivation of the *T. emersonii* strains on the different agar media revealed clear differences between the 393751 strain and the other 9 strains with respect to the following:
**Tables 43 and 44** Summary of the rate and extent of growth at 45°C - culture diameter measurements taken at day 2 and day 5
**Table 45:** Rate of growth at 55°C -culture diameter measurements taken at day 5
**Table 46:** Summary of visible phenotypic changes and evidence for spore formation.

**Important observation 1:** many of the IMI/CBS strains yielded multi-coloured cultures. However, the purity of these cultures was verified. The colours/pigments produced and the pattern of production is typical of many species of *Penicillium,* e.g. *P. pinophilum.*

**Important observation 2:** As indicated in Table 20, clear differences exist between the 393751 strain and the other in terms of spore formation.

**Table 43: Rate and extent of growth at 45°C - culture diameter measurements taken at day 5**

| **Strain** | **SDA** | **CMA** | **PMA** | **MEA** | **YpSs** | **YG** | **Nutr Agar** | **Czapek Dox** | **OMA** |
|---|---|---|---|---|---|---|---|---|---|
| | | | | | | | | | |
| IMI 393751 (our strain) | ++++++ | +++++ | ++ | ++++++ | ++ | ++++++ | ++ | ++++++ | ++++++ |
| | | | | | | | | | |
| | | | | | | | | | |
| IMI393752 (CBS 549.92) | ++++++ | +++++ | ++ | +++ | ++ | ++++ | ++ | +++ | +++++ |
| | | | | | | | | | |
| | | | | | | | | | |
| IMI393753 (CBS 180.68) | +++ | ++++ | + | +++ | + | ++++ | ++ | +++ | +++ |
| | | | | | | | | | |
| | | | | | | | | | |
| IMI393755 (CBS 355.92) | ++++ | +++++ | ++ | ++++ | + | ++++ | + | ++ | ++++ |
| | | | | | | | | | |
| | | | | | | | | | |
| IMI393756 (CBS 393.64) | ++++ | +++++ | +++ | ++++ | ++ | ++++ | ++ | +++ | ++++ |
| | | | | | | | | | |
| | | | | | | | | | |
| IMI393757 (CBS 394.64) | +++ | +++ | + | ++++ | + | +++ | + | ++ | +++ |
| | | | | | | | | | |
| | | | | | | | | | |
| IMI393758 (CBS 395.64) | +++++ | +++++ | ++ | +++ | + | ++++ | +++ | ++ | ++++ |
| | | | | | | | | | |
| | | | | | | | | | |
| IMI393759 (CBS 397.64) | +++++ | +++++ | ++ | +++ | ++ | +++++ | ++ | +++ | ++++ |
| | | | | | | | | | |
| | | | | | | | | | |
| IMI393760 (CBS 472.92) | +++++ | +++++ | ++++ | +++++ | ++ | ++++ | ++ | +++ | ++++++ |
| | | | | | | | | | |
| | | | | | | | | | |
| IMI393761 (CBS 759.71) | +++++ | +++++ | nd | ++++ | ++ | +++ | ++ | ++ | ++++ |

| | | | | | | | | | |
|---|---|---|---|---|---|---|---|---|---|
| SDA, Sabouraud Dextrose Agar; CMA, Cornmeal Agar; PMA, Potato Maltose Agar; MEA, Malt Extract agar; YpSs, Yeast potassium soluble starch; YG, Yeast Glucose Agar; NA, Nutrient Agar; OMA, Oatmeal Agar. Culture diameters: Full plate, ++++++; 65-80 mm, +++++; 50-65 mm, ++++; 35-50 mm, +++; 20-35 mm, ++; and <20 mm, +. | | | | | | | | | |

**Table 44: Rate and extent of growth at 45°C - culture diameter measurements taken at day 2**

| **Strain** | **SDA** | **CMA** | **PMA** | **MEA** | **YpSs** | **YG** | **Nutr Agar** | **Czapek Dox** | **OMA** |
|---|---|---|---|---|---|---|---|---|---|
| | | | | | | | | | |
| IMI 393751 (our strain) | ++++ | ++ | + | ++++ | + | +++ | + | +++ | ++++ |
| | | | | | | | | | |
| | | | | | | | | | |
| IMI393752 (CBS 549.92) | +++ | +++ | + | + | + | ++ | + | + | +++ |
| | | | | | | | | | |
| | | | | | | | | | |
| IMI393753 (CBS 180.68) | ++ | ++ | - | + | + | ++ | + | + | + |
| | | | | | | | | | |
| | | | | | | | | | |
| IMI393755 (CBS 355.92) | ++ | ++ | + | ++ | - | + | - | - | + |
| | | | | | | | | | |
| | | | | | | | | | |
| IMI393756 (CBS 393.64) | ++ | + | + | + | + | ++ | - | + | + |
| | | | | | | | | | |
| | | | | | | | | | |
| IMI393757 (CBS 394.64) | + | + | - | ++ | - | + | - | + | + |
| | | | | | | | | | |
| | | | | | | | | | |
| IMI393758 (CBS 395.64) | ++ | ++ | + | + | - | + | + | + | ++ |
| | | | | | | | | | |
| | | | | | | | | | |
| IMI393759 (CBS 397.64) | ++ | ++ | + | + | + | + | + | + | + |
| | | | | | | | | | |
| | | | | | | | | | |
| IMI393760 (CBS 472.92) | ++ | ++ | ++ | ++ | + | ++ | - | + | +++ |
| | | | | | | | | | |
| | | | | | | | | | |
| IMI393761 (CBS 759.711 | ++ | ++ | nd | + | +/- | + | +/- | + | + |

| | | | | | | | | | |
|---|---|---|---|---|---|---|---|---|---|
| SDA, Sabouraud Dextrose Agar; CMA, Cornmeal Agar; PMA, Potato Maltose Agar; MEA, Malt Extract agar; YpSs, Yeast potassium soluble starch; YG, Yeast Glucose Agar; NA, Nutrient Agar; OMA, Oatmeal Agar. Culture diameters: Full plate, ++++++; 65-80 mm, +++++; 50-65 mm, ++++; 35-50 mm, +++; 20-35 mm, ++; and <20 mm, +; no evidence for growth, -. | | | | | | | | | |

**Table 45: Rate and extent of growth at 55°C - culture diameter measurements taken at day 5**

| **Strain** | **SDA** | **CMA** | **PMA** | **MEA** | **YpSs** | **YG** | **Nutr Agar** | **Czapek Dox** | **OMA** |
|---|---|---|---|---|---|---|---|---|---|
| | | | | | | | | | |
| IMI 393751 (our strain) | ++++++ | +++++ | ++ | ++++++ | +++ | ++++++ | ++ | ++++++ | ++++++ |
| | | | | | | | | | |
| | | | | | | | | | |
| IMI393752 (CBS 549.92) | ++ | ++ | + | + | + | ++ | + | + | +++ |
| | | | | | | | | | |
| | | | | | | | | | |
| IMI393753 (CBS 180.68) | + | + | - | + | - | + | - | + | + |
| | | | | | | | | | |
| | | | | | | | | | |
| IMI393755 (CBS 355.92) | + | ++ | +/- | + | - | + | - | - | ++ |
| | | | | | | | | | |
| | | | | | | | | | |
| IMI393756 (CBS 393.64) | + | ++ | + | - | - | +- | - | - | + |
| | | | | | | | | | |
| | | | | | | | | | |
| IMI393757 (CBS 394.64) | + | + | - | + | - | + | - | - | +/- |
| | | | | | | | | | |
| | | | | | | | | | |
| IMI393758 (CBS 395.64) | +++ | +++ | + | +++ | + | ++ | + | + | +++ |
| | | | | | | | | | |
| | | | | | | | | | |
| IMI393759 (CBS 397.64) | + | - | - | - | - | ++ | - | +/- | +/- |
| | | | | | | | | | |
| | | | | | | | | | |
| IMI393760 (CBS 472.92) | + | ++ | ++ | ++ | - | +/- | - | - | ++ |
| | | | | | | | | | |
| | | | | | | | | | |
| IMI393761 (CBS 759.71) | + | - | - | +/- | - | +/- | - | - | + |

| | | | | | | | | | |
|---|---|---|---|---|---|---|---|---|---|
| SDA, Sabouraud Dextrose Agar; CMA, Cornmeal Agar; PMA, Potato Maltose Agar; MEA, Malt Extract agar; YpSs, Yeast potassium solube starch; YG, Yeast Glucose Agar; NA, Nutrient Agar; Agar; OMA, Oatmeal Agar. Culture diameters: Full plate, ++++++; 65-80 mm, +++++; 50-65 mm, ++++; 35-50 mm, +++; 20-35 mm, ++; and <20 mm, +; no evidence for growth,-. | | | | | | | | | |

**Table 46: Summary of visible phenotypic changes and evidence for spore formation (5 day old cultures, 45°C).**

| **Strain** | | **SDA** | **CMA** | **PMA** | **MEA** | **YpSs** | **YG** | **Nutr Agar** | **Czapek Dox** | **OMA** |
|---|---|---|---|---|---|---|---|---|---|---|
| IMI 393751 (our strain) | **Appearance** | **Very dense, creamy white fluffy growth; orange colour at centre** | **Light, translucent growth; dense growth at inoculation point** | **Buff coloured and fluffy appearance;** | **White fluffy growth; buff colour at leading edge** | **Dark buff centre; white at leading edge dense growth** | **Dark buff centre; lighter buff at leading edge dense growth** | **Translu-cent growth; dark buff leading edge** | **Translu-cent growth; 'feathery' appearance** | **White and Buff centre** |
| | Sporulation | X | some | X | X | X | X | X | X | Yes |
| | **Appearance** | **White/cream; lime green; darker buff-green at centre** | **Pale translucent growth; green spores** | **Dense growth - Cream-buff centre and buff edges** | **Pale cream centre; Lime green** | **Pale cream-buff** | **Lime green; cream leading edge** | **Pale buff; slimy leading edge** | **Translu-cent growth; green centre** | **Dense growth time green; pinkish centre; cream leading edge** |
| IMI393752 (CBS 549.92) | | | | | | | | | | |
| | **Sporulation** | **Yes** | **Yes** | **X** | **Yes** | **Some** | **Yes** | **X** | **Yes** | **Yes** |
| | **Appearance** | **Fluffy white with green to deep buff centre** | **White cream translucent growth** | **Very dense growth; cream-buff** | **Dense growth; cream edge, yellow-green-pale orange centre** | **Cream-buff** | **Green edges; pale cream and green centre** | **Dense buff-cream; slimy leading edge** | **Translu-cent growth; light green centre** | **Pale cream leading edge; light green to dark cream centre** |
| IMI393753 (CBS 180.68) | | | | | | | | | | |
| | **Sporulation** | **Yes** | **Yes** | **Yes** | **Yes** | **Some** | **Some** | **X** | **Yes** | **Yes** |
| | **Appearance** | **Dark buff centre; cream leading edge** | **Buff translucent growth** | **Dense growth; cream-buff** | **Cream edges; lime green mid; orange centre** | **Buff-cream** | **Cream-dark buff centre** | **buff-cream; slimy leading edge** | **Very translu-cent growth;** | **Buff edges; cream light green and dark buff centre** |
| IMI393755 (CBS 355.92) | | | | | | | | | | |
| | **Sporulation** | **Yes** | **Yes** | **Yes** | **Yes** | **Some** | **Yes** | **Some** | **Some** | **Yes** |
| | **Appearance** | **Very dense growth; Cream-buff** | **Light, translucent growth** | **dark buff** | **Pale buff- cream-light green-orange & cream centre** | **Buff, dense growth** | **Buff edges, green to darker green in centre** | **Buff-white** | **Very light & translucent growth** | **Cream at edges; green-orange centre** |
| (IMI393756 (CBS 393.64) | | | | | | | | | | |
| | **Sporulation** | **Yes** | **Some** | **Yes** | **Yes** | **Yes** | **Yes** | **Yes** | **Some** | **Yes** |
| | **Appearance** | **Cream at edges; lime green-orange centre; Very dense growth;** | **translucent; light green** | **Dark buff; cream sporangia** | **Cruciform; pale green edges; orange-buff centre** | **Cream** | **Dense green-buff growth** | **Cream-buff; slimy appearance** | **Light; buff spores at edges** | **Pale green-cream edges; darker green- centre** |
| IMI393757 (CBS 394.64) | | | | | | | | | | |
| | **Sporulation** | **Yes** | **Yes** | **Yes** | **Yes** | **Inconclusive** | **Yes** | **Some** | **Some** | **Yes** |
| | **Appearance** | **Buff-white edges; darker centre** | **Poor growth; translucent; buff spores** | **Dense growth; cream-green** | **Dense, fluffy growth; buff-green** | **Buff-cream; very poor growth** | **Buff edges; green spores** | **buff-some white; slimy** | **Very light & translucent; very poor** | **Dense Growth; Cream-buff centre** |
| IMI393758 (CBS 395.64) | | | | | | | | | | |
| | **Sporulation** | **Yes** | **Yes** | **Yes** | **Yes (yellow)** | **Yes** | **Yes** | **Some** | **Inconclusive** | **Yes** |
| | **Appearance** | **White edges, green- buff at centre** | **translucent; white spores** | **Dense buff** | **Cream edge; Green - orange centre** | **Poor growth; buff, white centre** | **Green-buff edges; green-buffdense centre** | **Poor growth; Buff, slimy at edges** | **Very light & translucent; Pale buff** | **White edges yellow-green** |
| IMI393759 (CBS 397.64) | | | | | | | | | | |
| | **Sporulation** | **Yes** | **Yes** | **Inconclusive** | **Yes** | **Inconclusive** | **Yes** | **Inconclusive** | **Yes** | **Yes** |
| | **Appearance** | **Dense ochre-buff-lime green centre** | **White; light growth** | **White** | **Pale edges; fluorescent green centre** | **Pale white-cream** | **Dense centre-deep green; buff edges** | **Buff, slimy appearance** | **translucent; some white spores** | **Translucent and buff** |
| IMI393760 (CBS 472.92) | | | | | | | | | | |
| | **Sporulation** | **Yes** | **Yes** | **Yes** | **Yes** | **Some** | **Yes** | **Inconclusive** | **Some** | **Yes** |
| | **Appearance** | **Dark buff edges; pale cream-lime green- ochre** | **Poor growth-pale green tinge** | **nd** | **White edges, yellow - deep ochre- dark buff centre** | **White to pale at edges** | **Dense cream-buff growth** | **Cream-buff, slimy appearance** | **Poor growth; white spores** | **Cream at edges, pale lime green to green interior** |
| IMI393761 (CBS 759.71) | | | | | | | | | | |
| | **Sporulation** | **Yes** | **Yes** | | **Yes** | **Inconclusive** | **Inconclusive** | **Inconclusive** | **Yes** | **Yes** |

| | | | | | | | | | | |
|---|---|---|---|---|---|---|---|---|---|---|
| SDA, Sabouraud Dextrose Agar; CMA, Cornmeal Agar; PMA, Potato Maltose Agar; MEA, Malt Extract agar; YpSs, Yeast potassium soluble starch; YG, Yeast Glucose Agar; NA, Nutrient Agar; OMA, Oatmeal Agar. | | | | | | | | | | |

### Example 20: Reducing the volume and increasing the calorific value of waste streams prior to incineration

### Cellulose-rich sterilized clinical waste

Clinical waste has a calorific value of ∼14 GJ/tonne. In this form, it contains ∼50-55% cellulosic rich material, which has a high water binding capacity and lower calorific value compared with plastics present in the waste stream. Treatment of the cellulose-rich component of this waste using the thermostable enzyme cocktails of the invention, e.g. cocktails 5 and 8, can decrease the waste volume by ∼73-8%, and convert the cellulose present (79-84% conversion) and generate ∼2.25-2.61 GJ/tonne (calorific value). The residue, which is enriched in plastics could be used as a 'sorted' refuse-derived fuel with a potential calorific value of up to 34-38 GJ/tonne.

### Organic Fraction of Municipal Solid Waste

The organic fraction of municipal solid waste has a calorific value of ∼9.2-10.2 GJ/tonne. In this form, it contains ∼50-65% carbohydrate-rich material (mainly from food and paper wastes), which has a high water binding capacity and lower calorific value compared with other components, e.g. polystyrene, plastics and rubber present in the waste stream. Treatment of the cellulose-rich component of this waste with the thermostable enzyme cocktails of the invention can decrease the waste volume by ∼50-70%, and convert the carbohydrate present (71-91% conversion) and generate ∼3.8-7.8 GJ/tonne (calorific value). The residue, which is enriched in non-biodegradable components (e.g. polystyrene, plastics and rubber), could be used as a 'sorted' refuse-derived fuel with a potential calorific value of up to 26-38 GJ/tonne.

It is thus possible to treat municipal waste and other forms of waste prior to incineration. By so treating such feedstocks one could reduce, increase or alter their calorific value. So where a treatment produces a liquid sugar mix or leechate that is collected or removed it would be possible to reduce calorific value. Alteration in calorific value could facilitate incineration of such feedstocks: for example by reducing calorific value to a "set" level it would make it easier to control the incineration process; by increasing calorific value (i.e., treat to present sugars as simple sugars in the treated feedstock) the calorific value could be increased which means that the amount of energy required for incineration itself is reduced. For example, by converting the cellulose present in the cellulose-rich clinical waste stream to simple sugars and removing these, the calorific value of the residue would be quite low as it would mainly contain plastics, some metals, etc. Enzymatic conversion of complex carbohydrates in paper waste to simple sugars and recovery of the simple sugars would significantly reduce the calorific value of the waste-the residue would have still have calorific value, but this would be much lower (and would depend very much on the lignin content).

Thus in a further aspect the invention provides a method of altering the calorific value of a waste stream by treating the waste stream with a Talaromyces emersonii strain of the invention or an enzyme or enzyme composition of the invention.

### References

1. Raeder U. and Broda P. (1985), Lett. Appl. Microbiol. Vol. 1 pgs 17-20.
2. P. Chomozynski and N. Sacchi (1987),, Anal. Biochem Vol. 162, 156-159.
3. J. Sambrook, E.F. Fritsch and T. Maniatis (1989), , "Cold Spring Harbor Laboratory Press, New York.
4. G. Zaide, D. Shallom, S. Shulami, G. Zolotnitsky, G. Golan, T. Baasov, G. Shoham and Y. Shoham (2001) Eur. J. Biochem. Vol. 268; 3006-1016.
5. P.G. Murray, C.M. Collins, A. Grassick and M.G. Tuohy (2003), Biochem. Biophys. Res. Commun. Vol. 301, 280-286.
6. A.J. Harvey, M. Hrmova, R. De Gori, J.N. Varghese and G.B. Fincher, (2000) Proteins Vol. 41, 257-269.
7. A.P. Moloney, T.J. Hackett, P.J. Considine, M.P. Coughlan, (1983) Enzyme Microb. Technol. Vol. 5, 260-264.
8. G.L. Miller (1951), Anal. Chem. Vol. 31, 426-428.
9. M. Dubois, K. Gilles, P.A. Hamillton, P.A. Rebers, F. Smith, (1956) Anal. Chem. 28, 350-356.
10. U.R. Laemmli, (1970), Nature (London), Vol. 227, 680-685.
11. P.G. Murray, A-Grassick, C.D. Laffey, M.M. Cuffe, T. Higgins, A.V. Savage, A. Planas, M.G. Tuohy, (2001), Enzyme Microbial Technol. Vol. 29, 90-98.
12. O.H. Lowry, N.J. Rosebrough, A.L. Farr, R.J. Randall (1951), J. Biol. Chem. 193:265-275.
13. L. Falquet, M. Pagni, P. Bucher, N. Hulo, C.J. Sigrist, K. Hofmann, A. Bairoch, (2002). Nucleic Acids Research, Vo. 30, 235-238.
14. H. Nielsen, J. Engelbrecht, S. Brunak, G. on Heigne, (1997), Int. J. Neural Syst., Vol. 8, 581-599.
15. D. Higgins, J. Thompson, T. Gibson (1994) : Nucleic Acids Res, Vo. 22. 4673-4680.
16. A.C. Stolk, R.A. Samson (1972) Studies in Mycology No.2, pp. 1-65.
17. Tuohy M.G., Coughlan, M.P. (1992) Bioresource Technol. 39, 131-137
18. Tuohy, M.G., Walsh, D.J., Murray, P.G., Claeyssens, M., Cuffe, M.M., Savage, A.V., Coughlan, M.P. (2002). Biochim. Biophys. Acta, 1596, 336-380.
19. Reynolds, P.J. (1986) Ph.D. Thesis, National University of Ireland, Galway.
20. APHA, A., WEF. (1992) Standard methods for the examination of wastewater.
21. Walsh, D.J. (1997) Ph.D. Thesis, National University of Ireland, Galway.
22. Gilleran, C.T. (2004) Ph.D. Thesis, National University of Ireland, Galway.
23. Maloney, A.P., Callan, S.M., Murray, P.G., Tuohy, M.G. (2004) Eur. J. Biochem. 271, 3115-3126.
24. Braet, C. (2005) Ph.D. Thesis, National University of Ireland, Galway. Bensadoun, A. and Weinstein, D. (1976). Anal. Biochem. 70: 241-250.
25. Tuohy, M.G., Laffey, C.D. & Coughlan. M.P. (1994) Bioresource Technol. 50, 37-42. Tuohy, M.G., Puls, J., Claeyssens, M. Vrsanska, M.& Coughan. M.P. (1993). Biochem. J. 290, 515-523.
26. Murray, P.G., Aro, N., Collins, C., Grassick, A., Penttilä, M., Saloheimo, M. & Tuohy, M. 2004. Prot. Expr. Purif. Vol. 38, pp. 248-257.
27. McCarthy, T., Hanniffy, O., Lalor, E., Savage, A.V. & Tuohy, M.G. 2005. Process Biochem. Vol. 40, pp. 1741-1748.
28. McCarthy, T., Hanniffy, O., Savage, A.V. & Tuohy, M.G. (2003) Int. J. Biol. Macrmol. 33: 141-148.
29. Colleran, E. & Pistilli, A. (1994) Ann. Microbiol. Enzimol. 44: 1-18.
30. Coates, J.D., Coughlan, M.F. & Colleran, E. (1996) J. Microbiol. Meth. 26: 237-246.

## Claims

1. A thermophilic strain of Talaromyces emersonii, with the deposition no. IMI 393751 or a mutant thereof which has a growth temperature range of 30 to 90°C and which actively secretes enzymes at temperatures above 55°C.

2. Use of a strain or mutant as claimed in claim 1 to produce an enzyme composition.

3. Use of the microorganism as claimed in claim 1, for bioconversion of plant or plant-derived materials or waste streams including hospital waste.

4. Use of the microorganism as claimed in claim 1, in the production of monosaccharide-rich feedstocks from plant residues.

5. Use of the microorganism as claimed in claim 1, in processing and recycling of wood paper products, paper and textiles.

6. Use of the microorganism as claimed in claim 1, in the saccharification of paper wastes.

7. Use of the microorganism as claimed in claim 1, in antifungal, biocontrol and slime control.

8. Use of the microorganism as claimed in claim 1, for horticultural applications.

9. Use of the microorganism as claimed in claim 1, in animal feed production to enhance the digestibility of cereal-based feedstuffs.

10. Use of the microorganism as claimed in claim 1, in the production of low pentose-containing cereal-based feedstuffs for monogastric animals with improved digestibility and low non-cellulosic β-glucan contents.

11. Use of the microorganism as claimed in claim 1 , in the production of functional feedstuffs with bioactive potential for use in veterinary healthcare.

12. Use of the microorganism as claimed in claim 1 , in the production of specialised dairy or dietary products, e.g. foodstuffs and beverage formulations for geriatric and infant healthcare.

13. Use of the microorganism as claimed in claim 1 , in the bakery and confectionary secto-s, and in the formulation of novel healthfood bakery products.

14. Use of the microorganism as claimed in claim 1 , in the generation of flavour, aroma and sensory precursor compounds in the food industry

15. Use of the microorganism as claimed in claim 1 , for the generation of functional foods.

16. Use of the microorganism as claimed in claim 1 , for production of novel designer non-alcoholic and alcoholic beverages, fruit juices and health drinks.

17. Use of the microorganism as claimed in claim 1 , in the production of biopharmaceuticals, such as bioactive oligosaccharides (including mixed linkage 1,3(4) and 1,3(6)-glucooligosaccharides, galactooligosaccharides xyloglucooligosaccharides, pectic oligosaccharides, branched and linear xylooligosaccharides, (galacto)glucomannooligosaccharides), glycopeptides and flavonoid glycosides from terrestrial and marine plants, plant residues, fungi and wastestreams or by-products rich in simple sugars.

18. Use of the microorganisms as claimed in claim 1 , to increase the bioavailability of biomolecules with natural anti-bacterial and anti-viral activity, including flavonoid and cyanogenic glycosides, saponins, oligosaccharides and phenolics (including ferulic, and *p*-coumaric acids, epicatechin, catechin, pyrogallic acid and the like).

19. Use of the microorganism as claimed in claim 1 , to increase the bioavailability of natural antioxidant biomolecules, e.g. carotenoids, lycopenes, xanthophylis, anthocyanins, phenolics and glycosides from all plants materials, residues, wastes, including various fruits and berries.

20. Use of the microorganism as claimed in claim 1 , for the generation of feedstocks from raw plant materials, plant residues and wastes for use in microbial production of antibiotics by fungi and bacteria, including *Penicillium sp.* and *Streptomyces sp..*

21. Use of the microorganism as claimed in claim 1 , for the generation of feedstocks from raw plant materials, plant residues and wastes for use in microbial production of citric acid.

22. Use of the microorganism as claimed in claim 1 , for the production of oligosaccharides from algal polysaccharides (e.g. laminaran and fucoidan) and additives derived from plant extracts, by generally regarded as safe processes, in the formulation of cosmetics.

23. Use of the microorganism as claimed in claim 1 , for the production of oligosaccharides and glycopeptides for use as research reagents, in biosensor production and as tools in functional glycomics to probe receptor-ligand interactions and in the production of substrate libraries to profile enzyme-substrate specificity.

24. Use of the microorganism strain as claimed in claim 1 , for the production of modified cellulose and β-glucans, cellooligosaccharides, modified starches and maltooligosaccharides, lactulose and polyols (e.g. mannitol, glucitol or dulcitol, xylitol. arabitol).

25. Use of the microorganism strain as claimed in claim 1 in a method of altering the calorific value of a waste stream.

26. Use as claimed in any of claims 3 - 25 further comprising use of enzymes derived from one or both of the strains *Chaetomium thermophile* and *Thermoascus aurantiacus.*

## Patentansprüche

1. Thermophiler Stamm von *Talaromyces emersonii* mit der Hinterlegungsnummer IMI 393751 oder eine Mutante davon, die einen Wachstumstemperaturbereich von 30 bis 90°C aufweist und bei Temperaturen oberhalb 55 °C aktiv Enzyme sezerniert.

2. Verwendung eines Stamms oder einer Mutante gemäß Anspruch 1 zur Herstellung einer Enzymzusammensetzung.

3. Verwendung des Mikroorganismus gemäß Anspruch 1 zur Biokonversion von Pflanzen oder von Pflanzen stammenden Materialien oder Abfallströmen einschließlich Krankenhausmüll.

4. Verwendung des Mikroorganismus gemäß Anspruch 1 bei der Herstellung von monosaccharidreichen Ausgangsmaterialien aus Pflanzenresten.

5. Verwendung des Mikroorganismus gemäß Anspruch 1 bei der Aufbereitung und dem Recycling von Holz, Papierprodukten, Papier und Textilien.

6. Verwendung des Mikroorganismus gemäß Anspruch 1 bei der Verzuckerung von Papierabfällen.

7. Verwendung des Mikroorganismus gemäß Anspruch 1 bei der Pilzbekämpfung, biologischen Bekämpfung und Schleimbekämpfung.

8. Verwendung des Mikroorganismus gemäß Anspruch 1 für gartenbauliche Anwendungen.

9. Verwendung des Mikroorganismus gemäß Anspruch 1 bei der Herstellung von Tierfutter zur Erhöhung der Verdaulichkeit von Futter auf Getreidebasis.

10. Verwendung des Mikroorganismus gemäß Anspruch 1 bei der Herstellung von pentosearmem Futter auf Getreidebasis für nichtwiederkäuende Tiere mit verbesserter Verdaulichkeit und geringem Gehalt an nichtcellulosischem β-Glucan.

11. Verwendung des Mikroorganismus gemäß Anspruch 1 bei der Herstellung von funktionellem Futter mit bioaktivem Potential zur Verwendung in der Veterinärmedizin.

12. Verwendung des Mikroorganismus gemäß Anspruch 1 bei der Herstellung von spezialisierten Milch- oder Nahrungsprodukten, zum Beispiel Lebensmitteln und Getränkezubereitungen für die Geriatrie und Säuglingsmedizin.

13. Verwendung des Mikroorganismus gemäß Anspruch 1 im Bäckerei- und Konditoreisektor und bei der Zubereitung von neuen ernährungsphysiologisch wertvollen Backwaren.

14. Verwendung des Mikroorganismus gemäß Anspruch 1 bei der Erzeugung von Geschmacks-, Aroma- und sensorischen Vorläuferverbindungen in der Lebensmittelindustrie.

15. Verwendung des Mikroorganismus gemäß Anspruch 1 zur Erzeugung von funktionellen Lebensmitteln.

16. Verwendung des Mikroorganismus gemäß Anspruch 1 zur Herstellung von neuen alkoholfreien und alkoholischen Designer-Getränken, Fruchtsäften und Gesundheitsgetränken.

17. Verwendung des Mikroorganismus gemäß Anspruch 1 bei der Herstellung von Biopharmaka, wie bioaktiven Oligosacchariden (einschließlich gemischtbindigen 1,3(4)- und 1,3(6)-Glucooligosacchariden, Galactooligosacchariden, Xyloglucooligosacchariden, Pektin-Oligosacchariden, verzweigten und linearen Xylooligosacchariden, (Galacto)glucomannooligosacchariden), Glycopeptiden und Flavonoidglycosiden aus Land- und Meerespflanzen, Pflanzenresten, Pilzen und Abfallströmen oder Nebenprodukten, die reich an einfachen Zuckern sind.

18. Verwendung des Mikroorganismus gemäß Anspruch 1 zur Erhöhung der Bioverfügbarkeit von Biomolekülen mit natürlicher antibakterieller und antiviraler Aktivität einschließlich Flavonoid- und cyanogenen Glycosiden, Saponinen, Oligosacchariden und Phenolderivaten (einschließlich Ferulaund p-Cumarinsäure, Epicatechin, Catechin, Pyrogallol und dergleichen).

19. Verwendung des Mikroorganismus gemäß Anspruch 1 zur Erhöhung der Bioverfügbarkeit von natürlichen antioxidativen Biomolekülen, zum Beispiel Carotinoiden, Lycopenen, Xanthophyllen, Anthocyaninen, Phenolderivaten und Glycosiden aus allen Pflanzenmaterialien, -resten, - abfällen einschließlich verschiedener Früchte und Beeren.

20. Verwendung des Mikroorganismus gemäß Anspruch 1 zur Erzeugung von Ausgangsmaterialien aus pflanzlichen Rohstoffen, Pflanzenresten und -abfällen zur Verwendung in der mikrobiellen Produktion von Antibiotika durch Pilze und Bakterien einschließlich *Penicillium* sp. und *Streptomyces* sp.

21. Verwendung des Mikroorganismus gemäß Anspruch 1 zur Erzeugung von Ausgangsmaterialien aus pflanzlichen Rohstoffen, Pflanzenresten und -abfällen zur Verwendung in der mikrobiellen Produktion von Zitronensäure.

22. Verwendung des Mikroorganismus gemäß Anspruch 1 zur Erzeugung von Oligosacchariden aus Algenpolysacchariden (z.B. Laminaran und Fucoidan) und Additiven, die von Pflanzenextrakten stammen, durch als unbedenklich anerkannten Verfahren bei der Zubereitung von Kosmetik.

23. Verwendung des Mikroorganismus gemäß Anspruch 1 zur Erzeugung von Oligosacchariden und Glycopeptiden zur Verwendung als Forschungsreagentien, bei der Herstellung von Biosensoren und als Werkzeuge in der funktionellen Glykomik zum Sondieren von Rezeptor-Ligand-Wechselwirkungen und bei der Herstellung von Substratbanken zum Profilieren der Enzym-Substrat-Spezifität.

24. Verwendung des Mikroorganismenstammes gemäß Anspruch 1 zur Herstellung von modifizierter Cellulose und von β-Glucanen, Cellooligosacchariden, modifizierten Stärken und Maltooligosacchariden, Lactulose und Polyolen (z.B. Mannit, Glucit oder Dulcit, Xylit, Arabit).

25. Verwendung des Mikroorganismenstammes gemäß Anspruch 1 in einem Verfahren zur Veränderung des Brennwerts eines Abfallstroms.

26. Verwendung gemäß einem der Ansprüche 3 bis 25, weiterhin umfassend die Verwendung von Enzymen, die von Stämmen von *Chaetomium thermophile* und/oder *Thermoascus aurantiacus* abgeleitet sind.

## Revendications

1. Souche thermophile de *Talaromyces emersonii,* qui porte le numéro de dépôt IMI 393751 ou un mutant de celle-ci qui a une gamme de températures de croissance de 30 à 90°C et qui sécrète activement des enzymes à des températures supérieures à 55°C.

2. Utilisation d'une souche ou d'un mutant tel que revendiqué en revendication 1 pour produire une composition enzymatique.

3. Utilisation du micro-organisme tel que revendiqué en revendication 1, pour la bioconversion d'une plante ou de matériels provenant d'une plante ou de flux de déchets y compris des déchets hospitaliers.

4. Utilisation du micro-organisme tel que revendiqué en revendication 1, dans la production d'aliments pour animaux riches en monosaccharides à partir de résidus de plantes.

5. Utilisation du micro-organisme tel que revendiqué en revendication 1, dans le traitement et le recyclage de bois, de produits à base de papier, de papier et de textiles.

6. Utilisation du micro-organisme tel que revendiqué en revendication 1, dans la saccharification de déchets de papier.

7. Utilisation du micro-organisme tel que revendiqué en revendication 1, dans la lutte antifongique, la lutte biologique et la lutte contre les dépôts mucilagineux.

8. Utilisation du micro-organisme tel que revendiqué en revendication 1, dans des applications horticoles.

9. Utilisation du micro-organisme tel que revendiqué en revendication 1, dans la production d'aliments pour animaux afin d'améliorer la digestibilité d'aliments pour animaux à base de céréales.

10. Utilisation du micro-organisme tel que revendiqué en revendication 1, dans la production d'aliments pour animaux à base de céréales à faible teneur en pentose pour les animaux monogastriques, présentant une meilleure digestibilité et de faibles teneurs en β-glucanes non cellulosiques.

11. Utilisation du micro-organisme tel que revendiqué en revendication 1, dans la production d'aliments fonctionnels pour animaux, présentant un potentiel bioactif utilisable dans le secteur santé vétérinaire.

12. Utilisation du micro-organisme tel que revendiqué en revendication 1, dans la production de produits alimentaires et diététiques spécialisés, par exemple des formulations d'aliments et de boissons dans le secteur santé gériatrique et pédiatrique.

13. Utilisation du micro-organisme tel que revendiqué en revendication 1, dans les secteurs de la boulangerie et de la confiserie, et dans la formulation de nouveaux produits de boulangerie diététiques.

14. Utilisation du micro-organisme tel que revendiqué en revendication 1, dans la production de flaveurs, d'arômes et de composés précurseurs de goût dans l'industrie alimentaire.

15. Utilisation du micro-organisme tel que revendiqué en revendication 1, pour la production d'aliments fonctionnels.

16. Utilisation du micro-organisme tel que revendiqué en revendication 1, pour la production de nouveaux types de boissons non alcoolisées et alcoolisées, de jus de fruits et de boissons diététiques.

17. Utilisation du micro-organisme tel que revendiqué en revendication 1, pour la production de produits biopharmaceutiques, tels que des oligosaccharides bioactifs (y compris des 1,3(4) et 1,3(6)-glucooligosaccharides à liaisons mixtes, des galactooligosaccharides, des xyloglucooligosaccharides, des oligosaccharides pectiques, des xylooligosaccacharides ramifiés et linéraires, des (galacto)glucomannooligosaccharides), des glycopeptides et des glycosides flavonoïdes provenant de plantes terrestres et marines, des résidus de plantes, des champignons et des flux de déchets ou des sous-produits riches en sucres simples.

18. Utilisation du micro-organisme tel que revendiqué en revendication 1, pour améliorer la biodisponibilité de biomolécules ayant une activité antibactérienne et antivirale naturelle, y compris des glycosides flavonoïdes et cyanogènes, des saponines, des oligosaccharides et des composés phénoliques (y compris les acides férulique et p-coumarique, l'épicatéchine, la catéchine, l'acide pyrogallique et semblables).

19. Utilisation du micro-organisme tel que revendiqué en revendication 1, pour accroître la biodisponibilité de biomolécules anti-oxydantes naturelles, par exemple, des caroténoïdes, des lycopènes, des xanthophyles, des anthocyanines, des composés phénoliques et des glycosides de tous matériels, résidus, déchets végétaux, y compris divers fruits et baies.

20. Utilisation du micro-organisme tel que revendiqué en revendication 1, pour la production d'aliments pour animaux à partir de matériels végétaux bruts, de résidus et de déchets végétaux, utilisables pour la production microbienne d'antibiotiques par des champignons et des bactéries, y compris *Penicillium sp.* et *Streptomyces sp.*

21. Utilisation du micro-organisme tel que revendiqué en revendication 1, dans la production d'aliments pour animaux à partir de matériels végétaux bruts, de résidus et de déchets végétaux utilisables dans la production microbienne d'acide citrique.

22. Utilisation du micro-organisme tel que revendiqué en revendication 1, dans la production d'oligosaccharides à partir de polysaccharides d'algues (par exemple, laminarane et fucoïdane) et d'additifs dérivés d'extraits végétaux, par des procédés généralement considérés comme sûrs, dans la formulation de cosmétiques.

23. Utilisation du micro-organisme tel que revendiqué en revendication 1, pour la production d'oligosaccharides et de glycopeptides utilisables comme réactifs de recherche, dans la production de biocapteurs et comme outils en glycobiologie fonctionnelle pour tester des interactions récepteur - ligand et dans la production de banques de substrats pour déterminer le profil de spécificité enzyme - substrat.

24. Utilisation du micro-organisme tel que revendiqué en revendication 1, pour la production de cellulose modifiée et de β-lucanes, de cellooligosaccharides, d'amidons modifiés et de maltooligosaccharides, de lactulose et de polyols (par exemple, mannitol, glucitol ou dulcitol, xylitol, arabitol).

25. Utilisation de la souche de micro-organisme tel que revendiqué en revendication 1, dans une méthode de modification de la valeur calorifique d'un flux de déchets.

26. Utilisation telle que revendiquée selon l'une quelconque des revendications 3 à 25, comprenant en outre l'utilisation d'enzymes provenant d'une ou des deux souches de *Chaetomium thermophile* et *Thermoascus aurantiacus.*
